(19) Europäisches Patentamt
European Patent Office
Office européen des brevets

(11) EP 4 273 136 A1

(12) **EUROPEAN PATENT APPLICATION**
published in accordance with Art. 153(4) EPC

(43) Date of publication:
**08.11.2023 Bulletin 2023/45**

(21) Application number: **21912340.3**

(22) Date of filing: **30.12.2021**

(51) International Patent Classification (IPC):
***C07D 401/04*** *(2006.01)* ***C07D 401/02*** *(2006.01)*
***A61K 31/454*** *(2006.01)* ***A61P 37/00*** *(2006.01)*
***A61P 13/12*** *(2006.01)*

(52) Cooperative Patent Classification (CPC):
**A61K 31/454; A61P 13/12; A61P 37/00; C07D 401/02; C07D 401/04**

(86) International application number:
**PCT/CN2021/142760**

(87) International publication number:
**WO 2022/143845 (07.07.2022 Gazette 2022/27)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **30.12.2020 CN 202011609415**
**08.01.2021 CN 202110022338**
**18.06.2021 CN 202110679456**
**25.08.2021 CN 202110982108**

(71) Applicants:
- **Jiangsu Hengrui Pharmaceuticals Co., Ltd.**
  **Lianyungang, Jiangsu 222047 (CN)**
- **Shanghai Hengrui Pharmaceutical Co., Ltd.**
  **Shanghai 200245 (CN)**

(72) Inventors:
- **LI, Xin**
  **Shanghai 200245 (CN)**
- **ZHANG, Zhigao**
  **Shanghai 200245 (CN)**
- **DONG, Wenming**
  **Shanghai 200245 (CN)**
- **HE, Feng**
  **Shanghai 200245 (CN)**
- **TAO, Weikang**
  **Shanghai 200245 (CN)**

(74) Representative: **Potter Clarkson**
**Chapel Quarter**
**Mount Street**
**Nottingham NG1 6HQ (GB)**

(54) **NITROGEN-CONTAINING BRIDGED HETEROCYCLIC COMPOUND, PREPARATION METHOD THEREFOR, AND MEDICAL USE THEREOF**

(57) Disclosed are a nitrogen-containing bridged heterocyclic compound, a preparation method therefor, and a medical application thereof. Specifically, disclosed are a nitrogen-containing bridged heterocyclic compound represented by general formula (I), a preparation method therefor, a pharmaceutical composition containing the compound, and use thereof as a therapeutic agent, particularly, use as a complement factor (Factor B) inhibitor and use in preparing a drug for treatment and/or prevention of Factor B-mediated diseases or disorders.

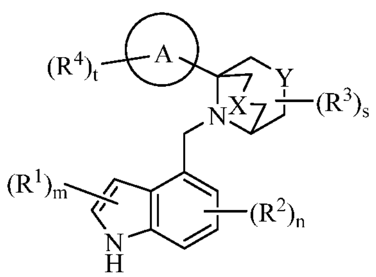

(I)

EP 4 273 136 A1

## Description

## TECHNICAL FIELD

The present disclosure belongs to the field of pharmaceutics, and relates to a nitrogen-containing bridged heterocyclic compound, a preparation method therefor, and pharmaceutical use thereof. In particular, the present disclosure relates to a nitrogen-containing bridged heterocyclic compound of general formula (I), a preparation method therefor, a pharmaceutical composition comprising same, and use thereof as a complement factor B inhibitor and in the preparation of a medicament for treating and/or preventing diseases or disorders mediated by Factor B.

## BACKGROUND

Complement is a serum protein that occurs in the serum and tissue fluid of humans and vertebrates. It is thermolabile, has enzyme activity after activation, can mediate immune and inflammatory responses, and can be activated by antigen-antibody complexes or microorganisms, causing lysis or phagocytosis of pathogenic microorganisms.

The complement system is an important regulator of inflammatory responses and tissue damage and consists of more than 20 serum proteins and cell surface proteins. The complement system includes complement innate components and a variety of regulatory proteins. The complement innate components include C1-C9, and the C3 content is the highest. The complement regulatory proteins further fall into two categories: soluble ones and membrane-bound ones. The soluble complement regulatory proteins include clusterin, S protein, complement factor H-related proteins, and the like. The membrane-bound complement regulatory proteins include membrane cofactor protein (MCP), decay accelerating factor (DAF), complement receptor 1, and the like. In addition, the complement system further includes some complement fragments and complement receptors, such as the C3a receptor and the C5a receptor.

The complement system is activated via three independent and intersecting pathways, namely the classical pathway (CP), the alternative pathway (AP), and the lectin pathway (LP, also known as the MBL (mannan-binding lectin) pathway). In the process of complement activation, a strong biological effect is produced through a series of positive feedback and is involved in the development and progression of disease. C3 convertase is an important component of the first three pathways. It causes production of a range of complement protein fragments and the membrane attack complex (MAC) via a complement activation cascade reaction. C3 convertase cleaves C3 to produce C5 convertase. Subsequently, C5 convertase cleaves C5 to produce C5a and C5b, and C5b binds to C6, C7, C8, and C9 to form C5b-9, i.e., MAC. Abnormalities in the complement pathways will cause lysis of the body's innate normal cells, thereby leading to the development of disease.

Complement factor B is a thermolabile β globulin, which can be inactivated by being simply heated at 50 °C for 30 min. It can be cleaved by complement factor D into two fragments-Ba and Bb, and Bb binds to C3b to form C3 convertase of the alternative pathway. Complement factor B, also known as C3 proactivator, is an important component of the complement alternative activation pathway. Complement factor B has a molecular weight of 93 kDa, is present in human blood at a concentration of about 3 μM, and is synthesized primarily in the liver. It is found that complement factor B is also synthesized in the retinal pigment epithelial cells of the eyes.

Paroxysmal nocturnal hemoglobinuria (PNH for short) is an acquired hematopoietic stem cell disorder. The hematopoietic stem cells are mutated through the acquired somatic cell PIG-A gene, causing abnormality in the synthesis of glycosyl phosphatidylinositol and loss of a group of membrane proteins anchored on the cell membrane by GPI and thereby non-malignant clonal diseases. The clinical manifestations mainly include loss of the hematopoietic function of bone marrow, thrombi, and acute episodes of chronic hemolytic anemia. The cause of the disease is unknown. It has been found that hemolysis in PNH is due to the sensitivity of the patient's erythrocytes to complement. PNH erythrocytes lack a complement regulatory protein known as decay accelerating factor (DAF), which functions to inhibit the formation of complement C3 convertase. The incidence of PNH is 1-9/million worldwide. The age of onset is mostly 25-45 years. Female patients seem to be a little more than male patients. Approximately 35% of PNH patients die within five years of diagnosis. The median survival time after diagnosis is about 10 to 15 years. Although the disease is caused by mutation of autosomal cells and thus is not heritable, the onset is sudden and cannot be prevented, and after the patient has it, intravascular hemolysis occurs, thereby causing severe complications such as thromboembolism and organ failure, disabling the patient, and eventually causing death. Previous treatments, including red blood cell transfusion to increase hemoglobin, use of glucocorticoids, and the like, mainly deal with symptoms. Bone marrow transplants are considered curative but require donors and involve a high risk of death. There is a lack of drugs on the market for PNH that can both control hemolysis and reduce the risk of death. In addition to hormone therapy and anticoagulant therapy, the human anti-complement C5 monoclonal antibody eculizumab and its long-acting molecule ravulizumab are the treatments of choice for PNH.

The antibody drug eculizumab is very expensive-the annual cost for treatment could reach 669,000 dollars. Moreover, the antibody drug cannot cure PNH but only control the hemolysis symptom, and cannot inhibit extravascular

hemolysis but only intravascular hemolysis. There are currently many drugs under development throughout the complement pathways, such as C3 upstream CAP inhibitors: TT30, ACH-4471 (anti-FD), and LNP023 (anti-FB); C3 inhibitors: AMY-101 and APL-2; and C3 downstream anti-C5 agents.

LNP023 is an oral small-molecule drug developed by Novartis Inc. There have been some reports on safety and effectiveness in the phase two trials of treating PNH. In a multicenter, open-label, continuous 2-cohort study involving 10 patients (25-79 years old) with PNH and active hemolysis who received eculizumab treatment, all the patients required red blood cell transfusions before LNP023 treatment. Enrolled subjects received LNP023 accompanied by eculizumab treatment twice daily for at least 13 weeks. LNP023 was found to be well tolerated and there is no treatment discontinuation and serious adverse events or thromboembolic events in the report. At 13 weeks, LNP023 showed a decrease of 34-81% in LDH in all the patients, Hb was normal in all the females, and Hb > 120 g/L in 71% of the males. The patients were exposed to LNP023, on average, for 241 days, and no patients required RBC transfusions. At the time of data cut-off, 5 patients had discontinued the eculizumab treatment and continued to use LNP023 monotherapy. All the patients using LNP023 monotherapy had constant hemoglobin levels, showed no change in any biomarker of disease activity, and did not have the breakthrough hemolysis symptom.

Glomerulopathy includes IgA nephropathy (IgAN for short), C3G glomerulopathy (C3G for short), membranous glomerulonephritis (MGN for short), and the like. IgAN and MGN are the most common of them. However, there has been some increase in the incidence of rare kidney diseases such as C3 glomerulopathy over the last decade. Glomerulopathy has been found to be closely related to the complement pathways, especially the complement alternative pathway. There are about 185 thousand new IgAN cases each year in the United States, with an age of onset of 20-40 years. The primary symptoms are hematuria, lethargy, and pain. Approximately 30% of the patients eventually develop end-stage renal disease (ESRD). There are about 80 thousand new membranous glomerulonephritis cases each year in the United States, with an age of onset of 40-60 years. Approximately 30% of the patients eventually develop ESRD. There are about 10 thousand new C3 glomerulopathy cases each year in the United States, and they are mostly teenagers. Approximately 50% of the patients eventually develop ESRD. Currently, there is a lack of clinically effective treatment regimens for primary glomerulonephritis. Medications such as hormones and immunosuppressants (e.g., cyclophosphamide, mycophenolate mofetil, tacrolimus, cyclosporine A, and the traditional Chinese medicine tripterygium glycosides) are typically used. Other medications include blood-pressure-controlling drugs, diuretics and platelet agglutination inhibitors, anticoagulants, lipid-lowering drugs, Cordyceps formulations, and other kidney-protecting and detoxifying drugs.

IgAN is the most common primary glomerular disease worldwide and pathologically manifests itself in localized mesangial hyperplasia and increases in the matrix accompanied by diffuse mesangial deposition of the IgA protein and often by IgG, C3, and C5b-9 deposition. The complement pathways are therefore thought to correlate with the development and progression of IgAN. Currently, there are two small-molecule drugs targeted at the complement pathways that are undergoing clinical trials. OMS721 is a humanized monoclonal antibody targeting the MASP-2 protein developed by Omeros Inc. The MASP-2 protein is the effector enzyme that activates the lectin pathway of the complement system. At the end of the phase 2 clinical trials of OMS721, the 4 IgAN patients enrolled in the trial all had a significantly improved proteinuria index. The drug is currently undergoing phase three clinical trials.

LNP023 is also undergoing phase two clinical trials of treating IgAN, C3G, and MGN in several countries and regions. Recently, Novartis Inc. announced that the drug achieved positive intermediate-term clinical outcomes in the phase 2 clinical trials of treating C3G patients. The effect of maintaining renal function was sustained in 7 patients who had received treatment for 6 months after being enrolled in the long-term extension study. The company will conduct phase three clinical trials this December and early next year.

Patent applications that disclose factor B inhibitors include WO2015009616A1, WO2019043609A1, WO2020016749A2, and the like.

## SUMMARY

The present disclosure aims to provide a compound of general formula (I) or a pharmaceutically acceptable salt thereof:

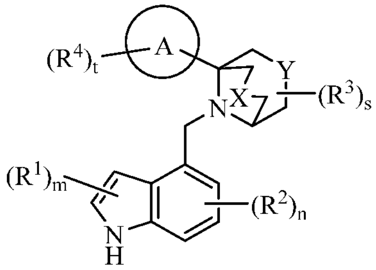

(I)

wherein:

X is selected from the group consisting of a chemical bond, $-CR^aR^b-$, -NR-, and -O-;
Y is selected from the group consisting of $-CR^{3a}R^{3b}-$, $-NR^{3c}-$, and -O-;
ring A is aryl or heteroaryl;
R, $R^a$, and $R^b$ are identical or different and are each independently selected from the group consisting of a hydrogen atom, alkyl, and haloalkyl;
each $R^1$ is identical or different and is independently selected from the group consisting of a hydrogen atom, alkyl, haloalkyl, hydroxyalkyl, alkenyl, alkynyl, alkoxy, haloalkoxy, halogen, cyano, $-NR^6R^7$, hydroxy, $-C(O)R^5$, $-CH_2C(O)R^5$, $-OCH_2C(O)R^5$, $-CH_2NHC(O)R^5$, $-C(O)NR^6R^7$, $-S(O)_pR^5$, $-S(O)_pNR^6R^7$, cycloalkyl, heterocyclyl, aryl, and heteroaryl, wherein the alkyl, alkoxy, cycloalkyl, heterocyclyl, aryl, and heteroaryl are each independently and optionally substituted with one or more substituents selected from the group consisting of halogen, alkyl, alkoxy, haloalkyl, haloalkoxy, cyano, amino, hydroxy, hydroxyalkyl, cycloalkyl, heterocyclyl, aryl, and heteroaryl;
each $R^2$ is identical or different and is independently selected from the group consisting of a hydrogen atom, alkyl, haloalkyl, hydroxyalkyl, alkenyl, alkynyl, alkoxy, haloalkoxy, halogen, cyano, $-NR^6R^7$, hydroxy, $-C(O)R^5$, $-CH_2C(O)R^5$, $-OCH_2C(O)R^5$, $-CH_2NHC(O)R^5$, $-C(O)NR^6R^7$, $-S(O)_pR^5$, $-S(O)_pNR^6R^7$, cycloalkyl, heterocyclyl, aryl, and heteroaryl, wherein the alkyl, alkoxy, cycloalkyl, heterocyclyl, aryl, and heteroaryl are each independently and optionally substituted with one or more substituents selected from the group consisting of halogen, alkyl, alkoxy, haloalkyl, haloalkoxy, cyano, amino, hydroxy, hydroxyalkyl, cycloalkyl, heterocyclyl, aryl, and heteroaryl;
each $R^3$, $R^{3a}$, $R^{3b}$, and $R^{3c}$ is identical or different and is independently selected from the group consisting of a hydrogen atom, alkyl, haloalkyl, hydroxyalkyl, alkoxy, haloalkoxy, oxo, halogen, cyano, $-NR^xR^y$, and hydroxy, wherein the alkyl is optionally substituted with one or more cyano or amino groups;
each $R^4$ is identical or different and is independently selected from the group consisting of a hydrogen atom, alkyl, haloalkyl, hydroxyalkyl, alkoxy, haloalkoxy, halogen, cyano, $-NR^8R^9$, hydroxy, $-C(O)R^{10}$, $-CH_2C(O)R^{10}$, $-C(O)NR^8R^9$, $-C(O)NHS(O)_pR^{10}$, $-S(O)_pNHC(O)R^{10}$, $-S(O)_pR^{10}$, $-S(O)_pNR^8R^9$, cycloalkyl, heterocyclyl, aryl, and heteroaryl, wherein the alkyl, cycloalkyl, heterocyclyl, aryl, and heteroaryl are each independently and optionally substituted with one or more substituents selected from the group consisting of halogen, alkyl, alkoxy, haloalkyl, haloalkoxy, cyano, amino, hydroxy, hydroxyalkyl, cycloalkyl, heterocyclyl, aryl, and heteroaryl;
$R^5$ are identical or different at each occurrence and are each independently selected from the group consisting of a hydrogen atom, hydroxy, alkyl, haloalkyl, alkoxy, cycloalkyl, heterocyclyl, aryl, and heteroaryl, wherein the alkyl, cycloalkyl, heterocyclyl, aryl, and heteroaryl are each independently and optionally substituted with one or more substituents selected from the group consisting of halogen, alkyl, alkoxy, haloalkyl, haloalkoxy, cyano, and hydroxy;
$R^{10}$ are identical or different at each occurrence and are each independently selected from the group consisting of a hydrogen atom, $-OR^{11}$, alkyl, haloalkyl, cycloalkyl, heterocyclyl, aryl, and heteroaryl, wherein the alkyl, cycloalkyl, heterocyclyl, aryl, and heteroaryl are each independently and optionally substituted with one or more substituents selected from the group consisting of halogen, alkyl, alkoxy, haloalkyl, haloalkoxy, cyano, and hydroxy;
$R^{11}$ is selected from the group consisting of a hydrogen atom, alkyl, haloalkyl, cycloalkyl, heterocyclyl, aryl, and heteroaryl, wherein the alkyl, cycloalkyl, heterocyclyl, aryl, and heteroaryl are each independently and optionally substituted with one or more substituents selected from the group consisting of halogen, alkyl, haloalkyl, cyano, $-OR^{12}$, and $-C(O)OR^{12}$,
$R^{12}$ is selected from the group consisting of a hydrogen atom, alkyl, haloalkyl, cycloalkyl, heterocyclyl, aryl, and heteroaryl, wherein the alkyl, cycloalkyl, heterocyclyl, aryl, and heteroaryl are each independently and optionally substituted with one or more substituents selected from the group consisting of halogen, alkyl, alkoxy, haloalkyl, haloalkoxy, cyano, and hydroxy;
$R^6$, $R^7$, $R^x$, $R^y$, $R^8$, and $R^9$ are identical or different at each occurrence and are each independently selected from the group consisting of a hydrogen atom, alkyl, haloalkyl, hydroxyalkyl, cycloalkyl, heterocyclyl, aryl, and heteroaryl,

wherein the alkyl, cycloalkyl, heterocyclyl, aryl, and heteroaryl are each independently and optionally substituted with one or more substituents selected from the group consisting of halogen, alkyl, alkoxy, haloalkyl, haloalkoxy, cyano, hydroxy, and hydroxyalkyl;

or $R^6$ and $R^7$, together with the nitrogen atom to which they are attached, form heterocyclyl, or $R^x$ and $R^y$, together with the nitrogen atom to which they are attached, form heterocyclyl, or $R^8$ and $R^9$, together with the nitrogen atom to which they are attached, form heterocyclyl, and the heterocyclyl is identical or different at each occurrence and is independently and optionally substituted with one or more substituents selected from the group consisting of halogen, alkyl, oxo, alkoxy, haloalkyl, haloalkoxy, cyano, amino, hydroxy, and hydroxyalkyl;

m is 0, 1, or 2;

n is 0, 1, 2, or 3;

s is 0, 1, 2, 3, or 4;

t is 0, 1, 2, 3, or 4; and

p is 0, 1, or 2.

In some embodiments of the present disclosure, the compound of general formula (I) or the pharmaceutically acceptable salt thereof is provided, wherein

X is selected from the group consisting of a chemical bond, $-CR^aR^b-$, -NR-, and -O-;

Y is selected from the group consisting of $-CR^{3a}R^{3b}-$, $-NR^{3c}-$, and -O-;

ring A is aryl or heteroaryl;

R, $R^a$, and $R^b$ are identical or different and are each independently selected from the group consisting of a hydrogen atom, alkyl, and haloalkyl;

each $R^1$ and $R^2$ is identical or different and is independently selected from the group consisting of a hydrogen atom, alkyl, haloalkyl, hydroxyalkyl, alkenyl, alkynyl, alkoxy, haloalkoxy, halogen, cyano, $-NR^6R^7$, hydroxy, $-C(O)R^5$, $-CH_2C(O)R^5$, $-OCH_2C(O)R^5$, $-CH_2NHC(O)R^5$, $-C(O)NR^6R^7$, $-S(O)_pR^5$, $-S(O)_pNR^6R^7$, cycloalkyl, heterocyclyl, aryl, and heteroaryl, wherein the alkyl, alkoxy, cycloalkyl, heterocyclyl, aryl, and heteroaryl are each independently and optionally substituted with one or more substituents selected from the group consisting of halogen, alkyl, alkoxy, haloalkyl, haloalkoxy, cyano, amino, hydroxy, hydroxyalkyl, cycloalkyl, heterocyclyl, aryl, and heteroaryl;

each $R^3$, $R^{3a}$, $R^{3b}$, and $R^{3c}$ is identical or different and is independently selected from the group consisting of a hydrogen atom, alkyl, haloalkyl, hydroxyalkyl, alkoxy, haloalkoxy, oxo, halogen, cyano, $-NR^xR^y$, and hydroxy, wherein the alkyl is optionally substituted with one or more cyano or amino groups;

each $R^4$ is identical or different and is independently selected from the group consisting of a hydrogen atom, alkyl, haloalkyl, hydroxyalkyl, alkoxy, haloalkoxy, halogen, cyano, $-NR^8R^9$, hydroxy, $-C(O)R^{10}$, $-CH_2C(O)R^{10}$, $-C(O)NR^8R^9$, $-C(O)NHS(O)_pR^{10}$, $-S(O)_pNHC(O)R^{10}$, $-S(O)_pR^{10}$, $-S(O)_pNR^8R^9$, cycloalkyl, heterocyclyl, aryl, and heteroaryl, wherein the alkyl, cycloalkyl, heterocyclyl, aryl, and heteroaryl are each independently and optionally substituted with one or more substituents selected from the group consisting of halogen, alkyl, alkoxy, haloalkyl, haloalkoxy, cyano, amino, hydroxy, hydroxyalkyl, cycloalkyl, heterocyclyl, aryl, and heteroaryl;

$R^5$ and $R^{10}$ are identical or different and are each independently selected from the group consisting of a hydrogen atom, hydroxy, alkyl, haloalkyl, alkoxy, cycloalkyl, heterocyclyl, aryl, and heteroaryl, wherein the alkyl, cycloalkyl, heterocyclyl, aryl, and heteroaryl are each independently and optionally substituted with one or more substituents selected from the group consisting of halogen, alkyl, alkoxy, haloalkyl, haloalkoxy, cyano, and hydroxy;

$R^6$, $R^7$, $R^x$, $R^y$, $R^8$, and $R^9$ are identical or different and are each independently selected from the group consisting of a hydrogen atom, alkyl, haloalkyl, hydroxyalkyl, cycloalkyl, heterocyclyl, aryl, and heteroaryl, wherein the alkyl, cycloalkyl, heterocyclyl, aryl, and heteroaryl are each independently and optionally substituted with one or more substituents selected from the group consisting of halogen, alkyl, alkoxy, haloalkyl, haloalkoxy, cyano, hydroxy, and hydroxyalkyl;

or $R^6$ and $R^7$, together with the nitrogen atom to which they are attached, form heterocyclyl, or $R^x$ and $R^y$, together with the nitrogen atom to which they are attached, form heterocyclyl, or $R^8$ and $R^9$, together with the nitrogen atom to which they are attached, form heterocyclyl, and the heterocyclyl is identical or different at each occurrence and is independently and optionally substituted with one or more substituents selected from the group consisting of halogen, alkyl, oxo, alkoxy, haloalkyl, haloalkoxy, cyano, amino, hydroxy, and hydroxyalkyl;

m is 0, 1, or 2;

n is 0, 1, 2, or 3;

s is 0, 1, 2, 3, or 4;

t is 0, 1, 2, 3, or 4; and

p is 0, 1, or 2.

In some embodiments of the present disclosure, the compound of general formula (I) or the pharmaceutically

acceptable salt thereof is provided, wherein Y is $-CR^{3a}R^{3b}-$; $R^{3a}$ and $R^{3b}$ are as defined in general formula (I).

In some embodiments of the present disclosure, the compound of general formula (I) or the pharmaceutically acceptable salt thereof is provided, wherein Y is $-CR^{3a}R^{3b}-$, and $R^{3a}$ and $R^{3b}$ are different and are each independently selected from the group consisting of a hydrogen atom, $C_{1-6}$ alkyl, $C_{1-6}$ haloalkyl, $C_{1-6}$ hydroxyalkyl, $C_{1-6}$ alkoxy, and $C_{1-6}$ haloalkoxy; preferably, Y is $-CR^{3a}R^{3b}-$, $R^{3a}$ is a hydrogen atom, and $R^{3b}$ is selected from the group consisting of a hydrogen atom, $C_{1-6}$ alkyl, $C_{1-6}$ haloalkyl, $C_{1-6}$ hydroxyalkyl, $C_{1-6}$ alkoxy, and $C_{1-6}$ haloalkoxy; further preferably, Y is $-CR^{3a}R^{3b}-$, $R^{3a}$ is a hydrogen atom, and $R^{3b}$ is selected from the group consisting of $C_{1-6}$ alkyl, $C_{1-6}$ haloalkyl, $C_{1-6}$ hydroxyalkyl, $C_{1-6}$ alkoxy, and $C_{1-6}$ haloalkoxy; more preferably, Y is $-CR^{3a}R^{3b}-$, $R^{3a}$ is a hydrogen atom, and $R^{3b}$ is $C_{1-6}$ alkoxy; most preferably, Y is $-CR^{3a}R^{3b}-$, $R^{3a}$ is a hydrogen atom, and $R^{3b}$ is ethoxy.

In some embodiments of the present disclosure, the compound of general formula (I) or the pharmaceutically acceptable salt thereof is a compound of general formula (II) or a pharmaceutically acceptable salt thereof:

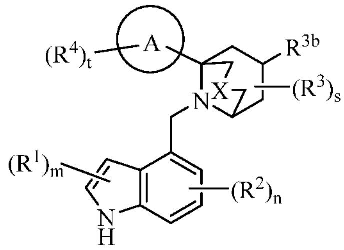

(II)

wherein:

ring A, X, $R^1$ to $R^3$, $R^{3b}$, $R^4$, m, n, s, and t are as defined in general formula (I).

In some embodiments of the present disclosure, the compound of general formula (I) or general formula (II) or the pharmaceutically acceptable salt thereof is provided, wherein s is 0 or 1; preferably, s is 0.

In some embodiments of the present disclosure, the compound of general formula (I) or general formula (II) or the pharmaceutically acceptable salt thereof is provided, wherein $R^3$ is a hydrogen atom.

In some embodiments of the present disclosure, the compound of general formula (I) or general formula (II) or the pharmaceutically acceptable salt thereof is provided, wherein m is 1.

In some embodiments of the present disclosure, the compound of general formula (I) or general formula (II) or the pharmaceutically acceptable salt thereof is provided, wherein n is 2.

In some embodiments of the present disclosure, the compound of general formula (I) or general formula (II) or the pharmaceutically acceptable salt thereof is provided, wherein m is 1, and n is 2.

In some embodiments of the present disclosure, the compound of general formula (I) or general formula (II) or the pharmaceutically acceptable salt thereof is a compound of general formula (III) or a pharmaceutically acceptable salt thereof:

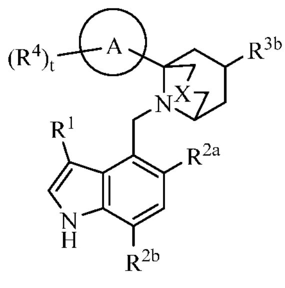

(III)

wherein:

$R^{2a}$ and $R^{2b}$ are identical or different and are each independently selected from the group consisting of a hydrogen atom, alkyl, haloalkyl, hydroxyalkyl, alkenyl, alkynyl, alkoxy, haloalkoxy, halogen, cyano, $-NR^6R^7$, hydroxy, $-C(O)R^5$, $-CH_2C(O)R^5$, $-OCH_2C(O)R^5$, $-CH_2NHC(O)R^5$, $-C(O)NR^6R^7$, $-S(O)_pR^5$, $-S(O)_pNR^6R^7$, cycloalkyl, heterocyclyl, aryl, and heteroaryl, wherein the alkyl, alkoxy, cycloalkyl, heterocyclyl, aryl, and heteroaryl are each independently and

optionally substituted with one or more substituents selected from the group consisting of halogen, alkyl, alkoxy, haloalkyl, haloalkoxy, cyano, amino, hydroxy, hydroxyalkyl, cycloalkyl, heterocyclyl, aryl, and heteroaryl;
ring A, X, $R^1$, $R^{3b}$, $R^4$ to $R^7$, p, and t are as defined in general formula (I).

In some embodiments of the present disclosure, the compound of general formula (I), general formula (II), or general formula (III) or the pharmaceutically acceptable salt thereof is provided, wherein each $R^4$ is identical or different and is independently selected from the group consisting of a hydrogen atom, $C_{1-6}$ alkyl, $C_{1-6}$ haloalkyl, $C_{1-6}$ hydroxyalkyl, $C_{1-6}$ alkoxy, $C_{1-6}$ haloalkoxy, halogen, cyano, $-NR^8R^9$, hydroxy, and $-C(O)R^{10}$, and $R^8$, $R^9$, and $R^{10}$ are as defined in general formula (I);

preferably, each $R^4$ is identical or different and is independently selected from the group consisting of a hydrogen atom, $C_{1-6}$ alkyl, $C_{1-6}$ haloalkyl, $C_{1-6}$ hydroxyalkyl, $C_{1-6}$ alkoxy, $C_{1-6}$ haloalkoxy, halogen, hydroxy, and $-C(O)R^{10}$, and $R^{10}$ is as defined in general formula (I);
more preferably, $R^4$ is $-C(O)R^{10}$, and $R^{10}$ is as defined in general formula (I).

In some embodiments of the present disclosure, the compound of general formula (I), general formula (II), or general formula (III) or the pharmaceutically acceptable salt thereof is provided, wherein $R^{10}$ is $-OR^{11}$, and $R^{11}$ is selected from the group consisting of a hydrogen atom, $C_{1-6}$ alkyl, $C_{1-6}$ haloalkyl, 3- to 8-membered cycloalkyl, and 3- to 8-membered heterocyclyl, wherein the $C_{1-6}$ alkyl, 3- to 8-membered cycloalkyl, and 3-to 8-membered heterocyclyl are each independently and optionally substituted with one or more substituents selected from the group consisting of halogen, $C_{1-6}$ alkyl, $C_{1-6}$ haloalkyl, cyano, $-OR^{12}$, and $-C(O)OR^{12}$, and $R^{12}$ is a hydrogen atom or $C_{1-6}$ alkyl; preferably, $R^{10}$ is $-OR^{11}$, and $R^{11}$ is selected from the group consisting of a hydrogen atom, $C_{1-6}$ alkyl, $C_{1-6}$ haloalkyl, 3- to 8-membered cycloalkyl, and 3- to 8-membered heterocyclyl, wherein the $C_{1-6}$ alkyl, 3- to 8-membered cycloalkyl, and 3- to 8-membered heterocyclyl are each independently and optionally substituted with one or more substituents selected from the group consisting of $-OR^{12}$ and $-C(O)OR^{12}$, and $R^{12}$ is a hydrogen atom or $C_{1-6}$ alkyl;
more preferably, $R^{10}$ is selected from the group consisting of hydroxy,

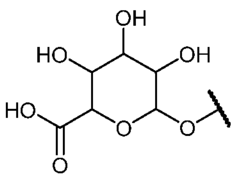 , 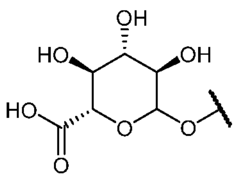 , 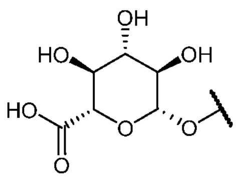 , and 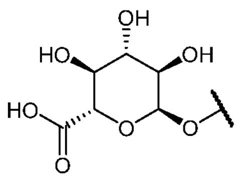 ;

most preferably, $R^{10}$ is hydroxy.

In some embodiments of the present disclosure, the compound of general formula (I), general formula (II), or general formula (III) or the pharmaceutically acceptable salt thereof is provided, wherein each $R^4$ is identical or different and is independently selected from the group consisting of a hydrogen atom, $C_{1-6}$ alkyl, $C_{1-6}$ haloalkyl, $C_{1-6}$ hydroxyalkyl, $C_{1-6}$ alkoxy, $C_{1-6}$ haloalkoxy, halogen, cyano, $-NR^8R^9$, hydroxy, and $-C(O)R^{10}$; $R^8$ and $R^9$ are identical or different and are each independently selected from the group consisting of a hydrogen atom, $C_{1-6}$ alkyl, $C_{1-6}$ haloalkyl, and $C_{1-6}$ hydroxyalkyl, wherein the $C_{1-6}$ alkyl is optionally substituted with one or more substituents selected from the group consisting of $C_{1-6}$ alkoxy, $C_{1-6}$ haloalkoxy, and cyano; $R^{10}$ is $-OR^{11}$; $R^{11}$ is selected from the group consisting of a hydrogen atom, $C_{1-6}$ alkyl, $C_{1-6}$ haloalkyl, 3- to 8-membered cycloalkyl, and 3- to 8-membered heterocyclyl, wherein the $C_{1-6}$ alkyl, 3- to 8-membered cycloalkyl, and 3- to 8-membered heterocyclyl are each independently and optionally substituted with one or more substituents selected from the group consisting of halogen, $C_{1-6}$ alkyl, $C_{1-6}$ haloalkyl, cyano, $-OR^{12}$, and $-C(O)OR^{12}$; $R^{12}$ is a hydrogen atom or $C_{1-6}$ alkyl;

preferably, each $R^4$ is identical or different and is independently selected from the group consisting of a hydrogen atom, $C_{1-6}$ alkyl, $C_{1-6}$ haloalkyl, $C_{1-6}$ hydroxyalkyl, $C_{1-6}$ alkoxy, $C_{1-6}$ haloalkoxy, halogen, hydroxy, and $-C(O)R^{10}$; $R^{10}$ is $-OR^{11}$; $R^{11}$ is selected from the group consisting of a hydrogen atom, $C_{1-6}$ alkyl, $C_{1-6}$ haloalkyl, 3- to 8-membered cycloalkyl, and 3- to 8-membered heterocyclyl, wherein the $C_{1-6}$ alkyl, 3- to 8-membered cycloalkyl, and 3- to 8-membered heterocyclyl are each independently and optionally substituted with one or more substituents selected from the group consisting of $-OR^{12}$ and $-C(O)OR^{12}$; $R^{12}$ is a hydrogen atom or $C_{1-6}$ alkyl;
more preferably, $R^4$ is $-C(O)R^{10}$, and $R^{10}$ is selected from the group consisting of hydroxy,

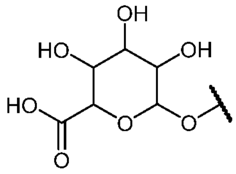, 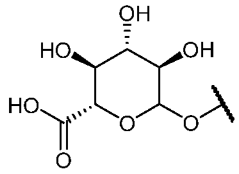, 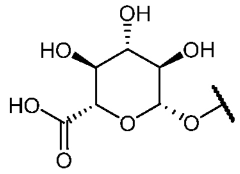,

and

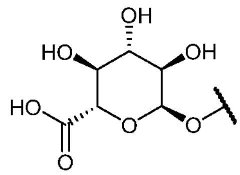;

most preferably, $R^4$ is -C(O)$R^{10}$, and $R^{10}$ is hydroxy.

In some embodiments of the present disclosure, the compound of general formula (I), general formula (II), or general formula (III) or the pharmaceutically acceptable salt thereof is provided, wherein each $R^4$ is identical or different and is independently selected from the group consisting of a hydrogen atom, $C_{1-6}$ alkyl, $C_{1-6}$ haloalkyl, $C_{1-6}$ hydroxyalkyl, $C_{1-6}$ alkoxy, $C_{1-6}$ haloalkoxy, halogen, cyano, -$NR^8R^9$, hydroxy, and -C(O)$R^{10}$, $R^8$, $R^9$, and $R^{10}$ are as defined in general formula (I); preferably, each $R^4$ is identical or different and is independently selected from the group consisting of a hydrogen atom, $C_{1-6}$ alkyl, $C_{1-6}$ haloalkyl, $C_{1-6}$ hydroxyalkyl, $C_{1-6}$ alkoxy, $C_{1-6}$ haloalkoxy, halogen, hydroxy, and -C(O)$R^{10}$, and $R^{10}$ is hydroxy; more preferably, $R^4$ is -C(O)$R^{10}$, and $R^{10}$ is hydroxy.

In some embodiments of the present disclosure, the compound of general formula (I), general formula (II), or general formula (III) or the pharmaceutically acceptable salt thereof is a compound of general formula (IV) or a pharmaceutically acceptable salt thereof:
wherein:

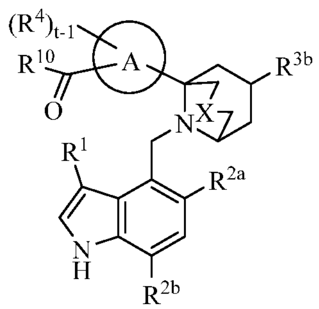

(IV)

$R^{10}$ is selected from the group consisting of hydroxy,

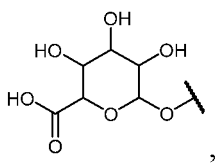, 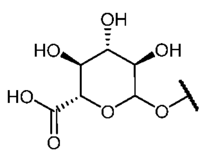,

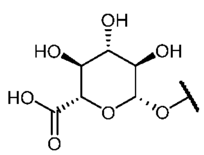, and 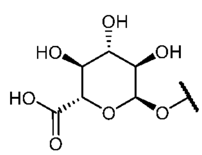;

preferably, $R^{10}$ is hydroxy;
t is 1 or 2;
$R^{2a}$ and $R^{2b}$ are identical or different and are each independently selected from the group consisting of a hydrogen atom, alkyl, haloalkyl, hydroxyalkyl, alkenyl, alkynyl, alkoxy, haloalkoxy, halogen, cyano, $-NR^6R^7$, hydroxy, $-C(O)R^5$, $-CH_2C(O)R^5$, $-OCH_2C(O)R^5$, $-CH_2NHC(O)R^5$, $-C(O)NR^6R^7$, $-S(O)_pR^5$, $-S(O)_pNR^6R^7$, cycloalkyl, heterocyclyl, aryl, and heteroaryl, wherein the alkyl, alkoxy, cycloalkyl, heterocyclyl, aryl, and heteroaryl are each independently and optionally substituted with one or more substituents selected from the group consisting of halogen, alkyl, alkoxy, haloalkyl, haloalkoxy, cyano, amino, hydroxy, hydroxyalkyl, cycloalkyl, heterocyclyl, aryl, and heteroaryl;
ring A, X, $R^1$, $R^{3b}$, $R^4$ to $R^7$, and p are as defined in general formula (I).

In some embodiments of the present disclosure, the compound of general formula (IV) or the pharmaceutically acceptable salt thereof is provided, wherein each $R^4$ is identical or different and is independently selected from the group consisting of a hydrogen atom, $C_{1-6}$ alkyl, $C_{1-6}$ haloalkyl, $C_{1-6}$ hydroxyalkyl, $C_{1-6}$ alkoxy, $C_{1-6}$ haloalkoxy, halogen, cyano, $-NR^8R^9$, and hydroxy, and $R^8$ and $R^9$ are as defined in general formula (I); preferably, each $R^4$ is selected from the group consisting of a hydrogen atom, $C_{1-6}$ alkyl, $C_{1-6}$ haloalkyl, $C_{1-6}$ hydroxyalkyl, $C_{1-6}$ alkoxy, $C_{1-6}$ haloalkoxy, halogen, and hydroxy; more preferably, $R^4$ is a hydrogen atom.

In some embodiments of the present disclosure, the compound of general formula (I), general formula (II), general formula (III), or general formula (IV) or the pharmaceutically acceptable salt thereof is provided, wherein $R^8$ and $R^9$ are identical or different and are each independently selected from the group consisting of a hydrogen atom, $C_{1-6}$ alkyl, $C_{1-6}$ haloalkyl, and $C_{1-6}$ hydroxyalkyl, wherein the $C_{1-6}$ alkyl is optionally substituted with one or more substituents selected from the group consisting of $C_{1-6}$ alkoxy, $C_{1-6}$ haloalkoxy, and cyano.

In some embodiments of the present disclosure, the compound of general formula (I), general formula (II), general formula (III), or general formula (IV) or the pharmaceutically acceptable salt thereof is provided, wherein X is a chemical bond.

In some embodiments of the present disclosure, the compound of general formula (I), general formula (II), general formula (III), or general formula (IV) or the pharmaceutically acceptable salt thereof is provided, wherein ring A is 6- to 10-membered aryl or 5- to 10-membered heteroaryl; preferably, ring A is phenyl or pyridinyl; more preferably, ring A is phenyl.

In some embodiments of the present disclosure, the compound of general formula (I) or general formula (II) or the pharmaceutically acceptable salt thereof is provided, wherein each $R^1$ is identical or different and is independently selected from the group consisting of a hydrogen atom, $C_{1-6}$ alkyl, $C_{1-6}$ haloalkyl, halogen, and cyano; preferably, $R^1$ is a hydrogen atom.

In some embodiments of the present disclosure, the compound of general formula (III) or general formula (IV) or the pharmaceutically acceptable salt thereof is provided, wherein $R^1$ is selected from the group consisting of a hydrogen atom, $C_{1-6}$ alkyl, $C_{1-6}$ haloalkyl, halogen, and cyano; preferably, $R^1$ is a hydrogen atom.

In some embodiments of the present disclosure, the compound of general formula (I) or general formula (II) or the pharmaceutically acceptable salt thereof is provided, wherein each $R^2$ is identical or different and is independently selected from the group consisting of a hydrogen atom, $C_{1-6}$ alkyl, $C_{1-6}$ haloalkyl, $C_{1-6}$ hydroxyalkyl, $C_{1-6}$ alkoxy, $C_{1-6}$ haloalkoxy, halogen, cyano, amino, hydroxy, 3- to 8-membered cycloalkyl, and 3- to 8-membered heterocyclyl; preferably, each $R^2$ is identical or different and is independently selected from the group consisting of a hydrogen atom, $C_{1-6}$ alkyl, $C_{1-6}$ alkoxy, and 3- to 6-membered cycloalkyl; more preferably, each $R^2$ is identical or different and is independently selected from the group consisting of a hydrogen atom, methyl, methoxy, and cyclopropyl.

In some embodiments of the present disclosure, the compound of general formula (I) or general formula (II) or the pharmaceutically acceptable salt thereof is provided, wherein each $R^2$ is identical or different and is independently selected from the group consisting of a hydrogen atom, $C_{1-6}$ alkyl, $C_{1-6}$ haloalkyl, $C_{1-6}$ hydroxyalkyl, $C_{1-6}$ alkoxy, $C_{1-6}$ haloalkoxy, and halogen; preferably, each $R^2$ is identical or different and is independently selected from the group consisting of a hydrogen atom, $C_{1-6}$ alkyl, and $C_{1-6}$ alkoxy; more preferably, each $R^2$ is identical or different and is independently selected from the group consisting of a hydrogen atom, methyl, and methoxy.

In some embodiments of the present disclosure, the compound of general formula (I), general formula (II), general formula (III), or general formula (IV) or the pharmaceutically acceptable salt thereof is a compound of general formula (V) or a pharmaceutically acceptable salt thereof:

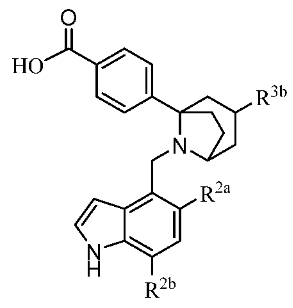

(V)

wherein:

$R^{2a}$ and $R^{2b}$ are identical or different and are each independently selected from the group consisting of a hydrogen atom, alkyl, haloalkyl, hydroxyalkyl, alkenyl, alkynyl, alkoxy, haloalkoxy, halogen, cyano, $-NR^6R^7$, hydroxy, $-C(O)R^5$, $-CH_2C(O)R^5$, $-OCH_2C(O)R^5$, $-CH_2NHC(O)R^5$, $-C(O)NR^6R^7$, $-S(O)_pR^5$, $-S(O)_pNR^6R^7$, cycloalkyl, heterocyclyl, aryl, and heteroaryl, wherein the alkyl, alkoxy, cycloalkyl, heterocyclyl, aryl, and heteroaryl are each independently and optionally substituted with one or more substituents selected from the group consisting of halogen, alkyl, alkoxy, haloalkyl, haloalkoxy, cyano, amino, hydroxy, hydroxyalkyl, cycloalkyl, heterocyclyl, aryl, and heteroaryl;
$R^{3b}$, $R^5$, $R^6$, $R^7$, and p are as defined in general formula (I).

In some embodiments of the present disclosure, the compound of general formula (V) or the pharmaceutically acceptable salt thereof is a 1:1 mixture of two enantiomers of *rel*-(1*S*,3*S*,5*R*) (i.e., cis) or *rel*-(1*R*,3*S*,5*S*) (i.e., trans), or a mixture of two diastereomers of *rel*-(1*S*,3*S*,5*R*) (i.e., cis) or *rel*-(1*R*,3*S*,5*S*) (i.e., trans), preferably a 1:1 mixture of two enantiomers of *rel*-(1*S*,3*S*,5*R*), i.e., (±)-*rel*-(1*S*,3*S*,5*R*).

In some embodiments of the present disclosure, the compound of general formula (I), general formula (II), general formula (III), general formula (IV), or general formula (V) or the pharmaceutically acceptable salt thereof is a compound of general formula (V") or a pharmaceutically acceptable salt thereof:

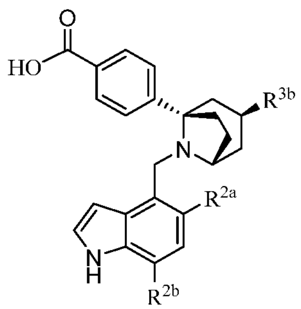

(±)-*rel*-(1*S*,3*S*,5*R*) (V")

wherein:

$R^{3b}$ is selected from the group consisting of $C_{1-6}$ alkyl, $C_{1-6}$ haloalkyl, $C_{1-6}$ hydroxyalkyl, $C_{1-6}$ alkoxy, and $C_{1-6}$ haloalkoxy;
$R^{2a}$ and $R^{2b}$ are identical or different and are each independently selected from the group consisting of a hydrogen atom, alkyl, haloalkyl, hydroxyalkyl, alkenyl, alkynyl, alkoxy, haloalkoxy, halogen, cyano, $-NR^6R^7$, hydroxy, $-C(O)R^5$, $-CH_2C(O)R^5$, $-OCH_2C(O)R^5$, $-CH_2NHC(O)R^5$, $-C(O)NR^6R^7$, $-S(O)_pR^5$, $-S(O)_pNR^6R^7$, cycloalkyl, heterocyclyl, aryl, and heteroaryl, wherein the alkyl, alkoxy, cycloalkyl, heterocyclyl, aryl, and heteroaryl are each independently and optionally substituted with one or more substituents selected from the group consisting of halogen, alkyl, alkoxy, haloalkyl, haloalkoxy, cyano, amino, hydroxy, hydroxyalkyl, cycloalkyl, heterocyclyl, aryl, and heteroaryl;
$R^5$, $R^6$, $R^7$, and p are as defined in general formula (I).

In some embodiments of the present disclosure, the compound of general formula (I), general formula (II), general formula (III), general formula (IV), general formula (V), or general formula (V") or the pharmaceutically acceptable

salt thereof is a compound of general formula (V-1) or a pharmaceutically acceptable salt thereof:

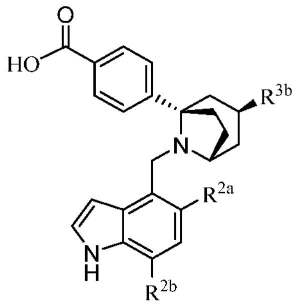

(V-1)

wherein:

$R^{3b}$ is selected from the group consisting of $C_{1-6}$ alkyl, $C_{1-6}$ haloalkyl, $C_{1-6}$ hydroxyalkyl, $C_{1-6}$ alkoxy, and $C_{1-6}$ haloalkoxy;
$R^{2a}$ and $R^{2b}$ are identical or different and are each independently selected from the group consisting of a hydrogen atom, alkyl, haloalkyl, hydroxyalkyl, alkenyl, alkynyl, alkoxy, haloalkoxy, halogen, cyano, $-NR^6R^7$, hydroxy, $-C(O)R^5$, $-CH_2C(O)R^5$, $-OCH_2C(O)R^5$, $-CH_2NHC(O)R^5$, $-C(O)NR^6R^7$, $-S(O)_pR^5$, $-S(O)_pNR^6R^7$, cycloalkyl, heterocyclyl, aryl, and heteroaryl, wherein the alkyl, alkoxy, cycloalkyl, heterocyclyl, aryl, and heteroaryl are each independently and optionally substituted with one or more substituents selected from the group consisting of halogen, alkyl, alkoxy, haloalkyl, haloalkoxy, cyano, amino, hydroxy, hydroxyalkyl, cycloalkyl, heterocyclyl, aryl, and heteroaryl;
$R^5$, $R^6$, $R^7$, and p are as defined in general formula (I).

In some embodiments of the present disclosure, the compound of general formula (I), general formula (II), general formula (III), general formula (IV), general formula (V), or general formula (V") or the pharmaceutically acceptable salt thereof is a compound of general formula (V-2) or a pharmaceutically acceptable salt thereof:

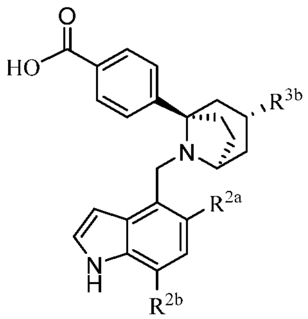

(V-2)

wherein:

$R^{3b}$ is selected from the group consisting of $C_{1-6}$ alkyl, $C_{1-6}$ haloalkyl, $C_{1-6}$ hydroxyalkyl, $C_{1-6}$ alkoxy, and $C_{1-6}$ haloalkoxy;
$R^{2a}$ and $R^{2b}$ are identical or different and are each independently selected from the group consisting of a hydrogen atom, alkyl, haloalkyl, hydroxyalkyl, alkenyl, alkynyl, alkoxy, haloalkoxy, halogen, cyano, $-NR^6R^7$, hydroxy, $-C(O)R^5$, $-CH_2C(O)R^5$, $-OCH_2C(O)R^5$, $-CH_2NHC(O)R^5$, $-C(O)NR^6R^7$, $-S(O)_pR^5$, $-S(O)_pNR^6R^7$, cycloalkyl, heterocyclyl, aryl, and heteroaryl, wherein the alkyl, alkoxy, cycloalkyl, heterocyclyl, aryl, and heteroaryl are each independently and optionally substituted with one or more substituents selected from the group consisting of halogen, alkyl, alkoxy, haloalkyl, haloalkoxy, cyano, amino, hydroxy, hydroxyalkyl, cycloalkyl, heterocyclyl, aryl, and heteroaryl;
$R^5$, $R^6$, $R^7$, and p are as defined in general formula (I).

In some embodiments of the present disclosure, the compound of general formula (I), general formula (II), general formula (III), or general formula (IV) or the pharmaceutically acceptable salt thereof is a compound of general

formula (V') or a pharmaceutically acceptable salt thereof:

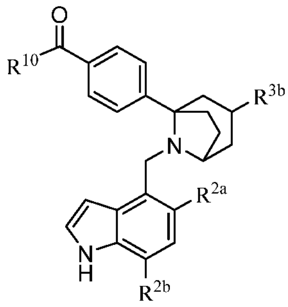

(V')

wherein:

$R^{2a}$ and $R^{2b}$ are identical or different and are each independently selected from the group consisting of a hydrogen atom, alkyl, haloalkyl, hydroxyalkyl, alkenyl, alkynyl, alkoxy, haloalkoxy, halogen, cyano, $-NR^6R^7$, hydroxy, $-C(O)R^5$, $-CH_2C(O)R^5$, $-OCH_2C(O)R^5$, $-CH_2NHC(O)R^5$, $-C(O)NR^6R^7$, $-S(O)_pR^5$, $-S(O)_pNR^6R^7$, cycloalkyl, heterocyclyl, aryl, and heteroaryl, wherein the alkyl, alkoxy, cycloalkyl, heterocyclyl, aryl, and heteroaryl are each independently and optionally substituted with one or more substituents selected from the group consisting of halogen, alkyl, alkoxy, haloalkyl, haloalkoxy, cyano, amino, hydroxy, hydroxyalkyl, cycloalkyl, heterocyclyl, aryl, and heteroaryl;

$R^{10}$ is selected from the group consisting of hydroxy,

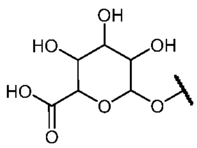, 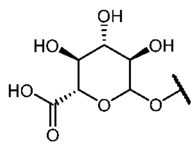,

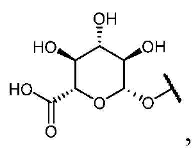, and 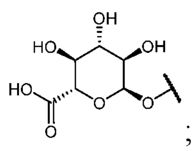;

$R^{3b}$, $R^5$, $R^6$, $R^7$, and p are as defined in general formula (I).

In some embodiments of the present disclosure, the compound of general formula (V') or the pharmaceutically acceptable salt thereof is a 1:1 mixture of two enantiomers of *rel*-(1*S*,3*S*,5*R*) (i.e., cis) or *rel*-(1*R*,3*S*,5*S*) (i.e., trans), or a mixture of two diastereomers of *rel*-(1*S*,3*S*,5*R*) (i.e., cis) or *rel*-(1*R*,3*S*,5*S*) (i.e., trans), preferably a 1:1 mixture of two enantiomers of *rel*-(1*S*,3*S*,5*R*), i.e., (±)-rel-(1*S*,3*S*,5*R*).

In some embodiments of the present disclosure, the compound of general formula (I), general formula (II), general formula (III), general formula (IV), or general formula (V') or the pharmaceutically acceptable salt thereof is a compound of general formula (V'-1) or a pharmaceutically acceptable salt thereof:

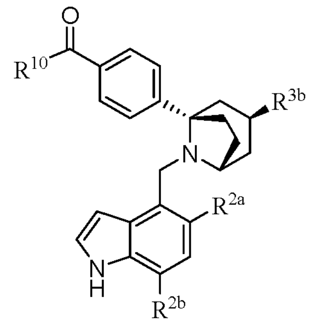

(V'-1)

wherein:

$R^{3b}$ is selected from the group consisting of $C_{1-6}$ alkyl, $C_{1-6}$ haloalkyl, $C_{1-6}$ hydroxyalkyl, $C_{1-6}$ alkoxy, and $C_{1-6}$ haloalkoxy;

$R^{2a}$ and $R^{2b}$ are identical or different and are each independently selected from the group consisting of a hydrogen atom, alkyl, haloalkyl, hydroxyalkyl, alkenyl, alkynyl, alkoxy, haloalkoxy, halogen, cyano, $-NR^6R^7$, hydroxy, $-C(O)R^5$, $-CH_2C(O)R^5$, $-OCH_2C(O)R^5$, $-CH_2NHC(O)R^5$, $-C(O)NR^6R^7$, $-S(O)_pR^5$, $-S(O)_pNR^6R^7$, cycloalkyl, heterocyclyl, aryl, and heteroaryl, wherein the alkyl, alkoxy, cycloalkyl, heterocyclyl, aryl, and heteroaryl are each independently and optionally substituted with one or more substituents selected from the group consisting of halogen, alkyl, alkoxy, haloalkyl, haloalkoxy, cyano, amino, hydroxy, hydroxyalkyl, cycloalkyl, heterocyclyl, aryl, and heteroaryl;

$R^{10}$ is selected from the group consisting of hydroxy,

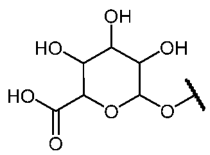
,

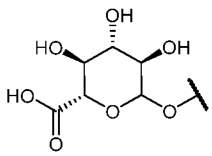
,
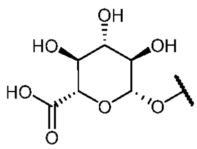
, and
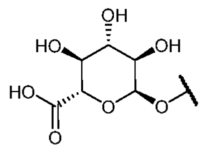
;

$R^5$, $R^6$, $R^7$, and p are as defined in general formula (I).

In some embodiments of the present disclosure, the compound of general formula (I), general formula (II), general formula (III), general formula (IV), or general formula (V') or the pharmaceutically acceptable salt thereof is a compound of general formula (V'-2) or a pharmaceutically acceptable salt thereof

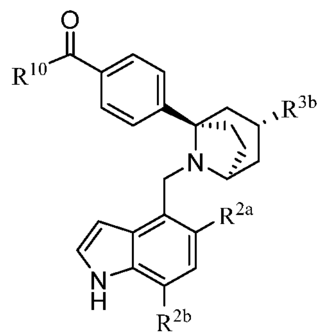

(V'-2)

wherein:

$R^{3b}$ is selected from the group consisting of $C_{1-6}$ alkyl, $C_{1-6}$ haloalkyl, $C_{1-6}$ hydroxyalkyl, $C_{1-6}$ alkoxy, and $C_{1-6}$ haloalkoxy;

$R^{2a}$ and $R^{2b}$ are identical or different and are each independently selected from the group consisting of a hydrogen atom, alkyl, haloalkyl, hydroxyalkyl, alkenyl, alkynyl, alkoxy, haloalkoxy, halogen, cyano, $-NR^6R^7$, hydroxy, $-C(O)R^5$, $-CH_2C(O)R^5$, $-OCH_2C(O)R^5$, $-CH_2NHC(O)R^5$, $-C(O)NR^6R^7$, $-S(O)_pR^5$, $-S(O)_pNR^6R^7$, cycloalkyl, heterocyclyl, aryl, and heteroaryl, wherein the alkyl, alkoxy, cycloalkyl, heterocyclyl, aryl, and heteroaryl are each independently and optionally substituted with one or more substituents selected from the group consisting of halogen, alkyl, alkoxy, haloalkyl, haloalkoxy, cyano, amino, hydroxy, hydroxyalkyl, cycloalkyl, heterocyclyl, aryl, and heteroaryl;

$R^{10}$ is selected from the group consisting of hydroxy,

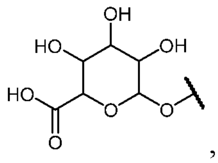, 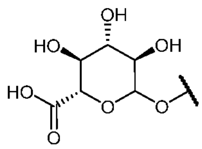,

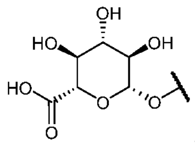, and 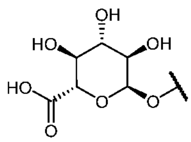;

$R^5$, $R^6$, $R^7$, and p are as defined in general formula (I).

In some embodiments of the present disclosure, the compound of general formula (III), general formula (IV), general formula (V), general formula (V"), general formula (V-1), general formula (V-2), general formula (V"), general formula (V'-1), or general formula (V'-2) or the pharmaceutically acceptable salt thereof is provided, wherein $R^{2a}$ and $R^{2b}$ are identical or different and are each independently selected from the group consisting of a hydrogen atom, $C_{1-6}$ alkyl, $C_{1-6}$ haloalkyl, $C_{1-6}$ hydroxyalkyl, $C_{1-6}$ alkoxy, $C_{1-6}$ haloalkoxy, halogen, cyano, amino, hydroxy, 3- to 8-membered cycloalkyl, and 3- to 8-membered heterocyclyl; preferably, $R^{2a}$ and $R^{2b}$ are identical or different and are each independently selected from the group consisting of $C_{1-6}$ alkyl, $C_{1-6}$ alkoxy, and 3- to 6-membered cycloalkyl; more preferably, $R^{2a}$ is selected from the group consisting of $C_{1-6}$ alkyl, $C_{1-6}$ alkoxy, and 3- to 6-membered cycloalkyl, and $R^{2b}$ is $C_{1-6}$ alkyl; most preferably, $R^{2a}$ is selected from the group consisting of methyl, methoxy, and cyclopropyl, and $R^{2b}$ is methyl.

In some embodiments of the present disclosure, the compound of general formula (III) or general formula (IV) or the pharmaceutically acceptable salt thereof is provided, wherein $R^{2a}$ and $R^{2b}$ are identical or different and are each independently selected from the group consisting of a hydrogen atom, $C_{1-6}$ alkyl, $C_{1-6}$ haloalkyl, $C_{1-6}$ hydroxyalkyl, $C_{1-6}$ alkoxy, $C_{1-6}$ haloalkoxy, and halogen; preferably, $R^{2a}$ and $R^{2b}$ are identical or different and are each independently $C_{1-6}$ alkyl or $C_{1-6}$ alkoxy; more preferably, $R^{2a}$ is methoxy, and $R^{2b}$ is methyl.

In some embodiments of the present disclosure, the compound of general formula (I), general formula (II), general formula (III), general formula (IV), general formula (V), or general formula (V') or the pharmaceutically acceptable salt thereof is provided, wherein $R^{3b}$ is selected from the group consisting of a hydrogen atom, $C_{1-6}$ alkyl, $C_{1-6}$ haloalkyl, $C_{1-6}$ hydroxyalkyl, $C_{1-6}$ alkoxy, and $C_{1-6}$ haloalkoxy; preferably, $R^{3b}$ is selected from the group consisting of $C_{1-6}$ alkyl, $C_{1-6}$ haloalkyl, $C_{1-6}$ hydroxyalkyl, $C_{1-6}$ alkoxy, and $C_{1-6}$ haloalkoxy; more preferably, $R^{3b}$ is $C_{1-6}$ alkoxy; most preferably, $R^{3b}$ is ethoxy.

In some embodiments of the present disclosure, the compound of formula (V"), general formula (V-1), general formula (V-2), general formula (V'-1), or general formula (V'-2) or the pharmaceutically acceptable salt thereof is provided, wherein $R^{3b}$ is $C_{1-6}$ alkoxy; preferably, $R^{3b}$ is ethoxy.

In some embodiments of the present disclosure, the compound of general formula (I), general formula (II), or general formula (III) or the pharmaceutically acceptable salt thereof is provided, wherein t is 1, 2, 3, or 4; preferably, t is 1 or 2; most preferably, t is 1.

In some embodiments of the present disclosure, the compound of general formula (I) or the pharmaceutically acceptable salt thereof is provided, wherein X is a chemical bond; Y is $-CR^{3a}R^{3b}-$; $R^{3a}$ and $R^{3b}$ are different and are each independently selected from the group consisting of a hydrogen atom, $C_{1-6}$ alkyl, $C_{1-6}$ haloalkyl, $C_{1-6}$ hydroxyalkyl, $C_{1-6}$ alkoxy, and $C_{1-6}$ haloalkoxy; ring A is 6- to 10-membered aryl or 5- to 10-membered heteroaryl; each $R^1$ is identical or different and is independently selected from the group consisting of a hydrogen atom, $C_{1-6}$ alkyl, $C_{1-6}$ haloalkyl,

halogen, and cyano; each $R^2$ is identical or different and is independently selected from the group consisting of a hydrogen atom, $C_{1-6}$ alkyl, $C_{1-6}$ haloalkyl, $C_{1-6}$ hydroxyalkyl, $C_{1-6}$ alkoxy, $C_{1-6}$ haloalkoxy, halogen, cyano, amino, hydroxy, 3- to 8-membered cycloalkyl, and 3- to 8-membered heterocyclyl; each $R^4$ is identical or different and is independently selected from the group consisting of a hydrogen atom, $C_{1-6}$ alkyl, $C_{1-6}$ haloalkyl, $C_{1-6}$ hydroxyalkyl, $C_{1-6}$ alkoxy, $C_{1-6}$ haloalkoxy, halogen, cyano, $-NR^8R^9$, hydroxy, and $-C(O)R^{10}$; $R^8$ and $R^9$ are identical or different and are each independently selected from the group consisting of a hydrogen atom, $C_{1-6}$ alkyl, $C_{1-6}$ haloalkyl, and $C_{1-6}$ hydroxyalkyl, wherein the $C_{1-6}$ alkyl is optionally substituted with one or more substituents selected from the group consisting of $C_{1-6}$ alkoxy, $C_{1-6}$ haloalkoxy, and cyano; $R^{10}$ is $-OR^{11}$, and $R^{11}$ is selected from the group consisting of a hydrogen atom, $C_{1-6}$ alkyl, $C_{1-6}$ haloalkyl, 3- to 8-membered cycloalkyl, and 3- to 8-membered heterocyclyl, wherein the $C_{1-6}$ alkyl, 3- to 8-membered cycloalkyl, and 3- to 8-membered heterocyclyl are each independently and optionally substituted with one or more substituents selected from the group consisting of halogen, $C_{1-6}$ alkyl, $C_{1-6}$ haloalkyl, cyano, $-OR^{12}$, and $-C(O)OR^{12}$, and $R^{12}$ is a hydrogen atom or $C_{1-6}$ alkyl; s is 0; m is 1; n is 2; t is 1, 2, 3, or 4.

In some embodiments of the present disclosure, the compound of general formula (I) or the pharmaceutically acceptable salt thereof is provided, wherein X is a chemical bond; Y is $-CR^{3a}R^{3b}-$; $R^{3a}$ and $R^{3b}$ are different and are each independently selected from the group consisting of a hydrogen atom, $C_{1-6}$ alkyl, $C_{1-6}$ haloalkyl, $C_{1-6}$ hydroxyalkyl, $C_{1-6}$ alkoxy, and $C_{1-6}$ haloalkoxy; ring A is 6- to 10-membered aryl or 5- to 10-membered heteroaryl; each $R^1$ is identical or different and is independently selected from the group consisting of a hydrogen atom, $C_{1-6}$ alkyl, $C_{1-6}$ haloalkyl, halogen, and cyano; each $R^2$ is identical or different and is independently selected from the group consisting of a hydrogen atom, $C_{1-6}$ alkyl, $C_{1-6}$ haloalkyl, $C_{1-6}$ hydroxyalkyl, $C_{1-6}$ alkoxy, $C_{1-6}$ haloalkoxy, halogen, cyano, amino, hydroxy, 3- to 8-membered cycloalkyl, and 3- to 8-membered heterocyclyl; each $R^4$ is identical or different and is independently selected from the group consisting of a hydrogen atom, $C_{1-6}$ alkyl, $C_{1-6}$ haloalkyl, $C_{1-6}$ hydroxyalkyl, $C_{1-6}$ alkoxy, $C_{1-6}$ haloalkoxy, halogen, cyano, $-NR^8R^9$, hydroxy, and $-C(O)R^{10}$; $R^8$ and $R^9$ are identical or different and are each independently selected from the group consisting of a hydrogen atom, $C_{1-6}$ alkyl, $C_{1-6}$ haloalkyl, and $C_{1-6}$ hydroxyalkyl, wherein the $C_{1-6}$ alkyl is optionally substituted with one or more substituents selected from the group consisting of $C_{1-6}$ alkoxy, $C_{1-6}$ haloalkoxy, and cyano; $R^{10}$ is selected from the group consisting of a hydrogen atom, hydroxy, $C_{1-6}$ alkyl, $C_{1-6}$ haloalkyl, and $C_{1-6}$ alkoxy; s is 0; m is 1; n is 2; t is 1, 2, 3, or 4.

In some embodiments of the present disclosure, the compound of general formula (I) or the pharmaceutically acceptable salt thereof is provided, wherein X is a chemical bond; Y is $-CR^{3a}R^{3b}-$; $R^{3a}$ and $R^{3b}$ are different and are each independently selected from the group consisting of a hydrogen atom, $C_{1-6}$ alkyl, $C_{1-6}$ haloalkyl, $C_{1-6}$ hydroxyalkyl, $C_{1-6}$ alkoxy, and $C_{1-6}$ haloalkoxy; ring A is 6- to 10-membered aryl or 5- to 10-membered heteroaryl; each $R^1$ is identical or different and is independently selected from the group consisting of a hydrogen atom, $C_{1-6}$ alkyl, $C_{1-6}$ haloalkyl, halogen, and cyano; each $R^2$ is identical or different and is independently selected from the group consisting of a hydrogen atom, $C_{1-6}$ alkyl, $C_{1-6}$ haloalkyl, $C_{1-6}$ hydroxyalkyl, $C_{1-6}$ alkoxy, $C_{1-6}$ haloalkoxy, and halogen; each $R^4$ is identical or different and is independently selected from the group consisting of a hydrogen atom, $C_{1-6}$ alkyl, $C_{1-6}$ haloalkyl, $C_{1-6}$ hydroxyalkyl, $C_{1-6}$ alkoxy, $C_{1-6}$ haloalkoxy, halogen, cyano, $-NR^8R^9$, hydroxy, and $-C(O)R^{10}$; $R^8$ and $R^9$ are identical or different and are each independently selected from the group consisting of a hydrogen atom, $C_{1-6}$ alkyl, $C_{1-6}$ haloalkyl, and $C_{1-6}$ hydroxyalkyl, wherein the $C_{1-6}$ alkyl is optionally substituted with one or more substituents selected from the group consisting of $C_{1-6}$ alkoxy, $C_{1-6}$ haloalkoxy, and cyano; $R^{10}$ is selected from the group consisting of a hydrogen atom, hydroxy, $C_{1-6}$ alkyl, $C_{1-6}$ haloalkyl, and $C_{1-6}$ alkoxy; s is 0; m is 1; n is 2; t is 1, 2, 3, or 4.

In some embodiments of the present disclosure, the compound of general formula (II) or the pharmaceutically acceptable salt thereof is provided, wherein X is a chemical bond; $R^{3b}$ is selected from the group consisting of a hydrogen atom, $C_{1-6}$ alkyl, $C_{1-6}$ haloalkyl, $C_{1-6}$ hydroxyalkyl, $C_{1-6}$ alkoxy, and $C_{1-6}$ haloalkoxy; ring A is 6- to 10-membered aryl or 5- to 10-membered heteroaryl; each $R^1$ is identical or different and is independently selected from the group consisting of a hydrogen atom, $C_{1-6}$ alkyl, $C_{1-6}$ haloalkyl, halogen, and cyano; each $R^2$ is identical or different and is independently selected from the group consisting of a hydrogen atom, $C_{1-6}$ alkyl, $C_{1-6}$ haloalkyl, $C_{1-6}$ hydroxyalkyl, $C_{1-6}$ alkoxy, $C_{1-6}$ haloalkoxy, halogen, cyano, amino, hydroxy, 3- to 8-membered cycloalkyl, and 3- to 8-membered heterocyclyl; each $R^4$ is identical or different and is independently selected from the group consisting of a hydrogen atom, $C_{1-6}$ alkyl, $C_{1-6}$ haloalkyl, $C_{1-6}$ hydroxyalkyl, $C_{1-6}$ alkoxy, $C_{1-6}$ haloalkoxy, halogen, cyano, $-NR^8R^9$, hydroxy, and $-C(O)R^{10}$; $R^8$ and $R^9$ are identical or different and are each independently selected from the group consisting of a hydrogen atom, $C_{1-6}$ alkyl, $C_{1-6}$ haloalkyl, and $C_{1-6}$ hydroxyalkyl, wherein the $C_{1-6}$ alkyl is optionally substituted with one or more substituents selected from the group consisting of $C_{1-6}$ alkoxy, $C_{1-6}$ haloalkoxy, and cyano; $R^{10}$ is $-OR^{11}$, and $R^{11}$ is selected from the group consisting of a hydrogen atom, $C_{1-6}$ alkyl, $C_{1-6}$ haloalkyl, 3- to 8-membered cycloalkyl, and 3- to 8-membered heterocyclyl, wherein the $C_{1-6}$ alkyl, 3- to 8-membered cycloalkyl, and 3- to 8-membered heterocyclyl are each independently and optionally substituted with one or more substituents selected from the group consisting of halogen, $C_{1-6}$ alkyl, $C_{1-6}$ haloalkyl, cyano, $-OR^{12}$, and $-C(O)OR^{12}$, and $R^{12}$ is a hydrogen atom or $C_{1-6}$ alkyl; s is 0; m is 1; n is 2; t is 1, 2, 3, or 4.

In some embodiments of the present disclosure, the compound of general formula (II) or the pharmaceutically acceptable salt thereof is provided, wherein X is a chemical bond; $R^{3b}$ is selected from the group consisting of a hydrogen

atom, $C_{1-6}$ alkyl, $C_{1-6}$ haloalkyl, $C_{1-6}$ hydroxyalkyl, $C_{1-6}$ alkoxy, and $C_{1-6}$ haloalkoxy; ring A is 6- to 10-membered aryl or 5- to 10-membered heteroaryl; each $R^1$ is identical or different and is independently selected from the group consisting of a hydrogen atom, $C_{1-6}$ alkyl, $C_{1-6}$ haloalkyl, halogen, and cyano; each $R^2$ is identical or different and is independently selected from the group consisting of a hydrogen atom, $C_{1-6}$ alkyl, $C_{1-6}$ haloalkyl, $C_{1-6}$ hydroxyalkyl, $C_{1-6}$ alkoxy, $C_{1-6}$ haloalkoxy, halogen, cyano, amino, hydroxy, 3- to 8-membered cycloalkyl, and 3- to 8-membered heterocyclyl; each $R^4$ is identical or different and is independently selected from the group consisting of a hydrogen atom, $C_{1-6}$ alkyl, $C_{1-6}$ haloalkyl, $C_{1-6}$ hydroxyalkyl, $C_{1-6}$ alkoxy, $C_{1-6}$ haloalkoxy, halogen, cyano, $-NR^8R^9$, hydroxy, and $-C(O)R^{10}$; $R^8$ and $R^9$ are identical or different and are each independently selected from the group consisting of a hydrogen atom, $C_{1-6}$ alkyl, $C_{1-6}$ haloalkyl, and $C_{1-6}$ hydroxyalkyl, wherein the $C_{1-6}$ alkyl is optionally substituted with one or more substituents selected from the group consisting of $C_{1-6}$ alkoxy, $C_{1-6}$ haloalkoxy, and cyano; $R^{10}$ is selected from the group consisting of a hydrogen atom, hydroxy, $C_{1-6}$ alkyl, $C_{1-6}$ haloalkyl, and $C_{1-6}$ alkoxy; s is 0; m is 1; n is 2; t is 1, 2, 3, or 4.

In some embodiments of the present disclosure, the compound of general formula (II) or the pharmaceutically acceptable salt thereof is provided, wherein X is a chemical bond; $R^{3b}$ is selected from the group consisting of a hydrogen atom, $C_{1-6}$ alkyl, $C_{1-6}$ haloalkyl, $C_{1-6}$ hydroxyalkyl, $C_{1-6}$ alkoxy, and $C_{1-6}$ haloalkoxy; ring A is 6- to 10-membered aryl or 5- to 10-membered heteroaryl; each $R^1$ is identical or different and is independently selected from the group consisting of a hydrogen atom, $C_{1-6}$ alkyl, $C_{1-6}$ haloalkyl, halogen, and cyano; each $R^2$ is identical or different and is independently selected from the group consisting of a hydrogen atom, $C_{1-6}$ alkyl, $C_{1-6}$ haloalkyl, $C_{1-6}$ hydroxyalkyl, $C_{1-6}$ alkoxy, $C_{1-6}$ haloalkoxy, and halogen; each $R^4$ is identical or different and is independently selected from the group consisting of a hydrogen atom, $C_{1-6}$ alkyl, $C_{1-6}$ haloalkyl, $C_{1-6}$ hydroxyalkyl, $C_{1-6}$ alkoxy, $C_{1-6}$ haloalkoxy, halogen, cyano, $-NR^8R^9$, hydroxy, and $-C(O)R^{10}$; $R^8$ and $R^9$ are identical or different and are each independently selected from the group consisting of a hydrogen atom, $C_{1-6}$ alkyl, $C_{1-6}$ haloalkyl, and $C_{1-6}$ hydroxyalkyl, wherein the $C_{1-6}$ alkyl is optionally substituted with one or more substituents selected from the group consisting of $C_{1-6}$ alkoxy, $C_{1-6}$ haloalkoxy, and cyano; $R^{10}$ is selected from the group consisting of a hydrogen atom, hydroxy, $C_{1-6}$ alkyl, $C_{1-6}$ haloalkyl, and $C_{1-6}$ alkoxy; s is 0; m is 1; n is 2; t is 1, 2, 3, or 4.

In some embodiments of the present disclosure, the compound of general formula (III) or the pharmaceutically acceptable salt thereof is provided, wherein X is a chemical bond; $R^{3b}$ is selected from the group consisting of a hydrogen atom, $C_{1-6}$ alkyl, $C_{1-6}$ haloalkyl, $C_{1-6}$ hydroxyalkyl, $C_{1-6}$ alkoxy, and $C_{1-6}$ haloalkoxy; ring A is phenyl or 5- to 6-membered heteroaryl; $R^1$ is selected from the group consisting of a hydrogen atom, $C_{1-6}$ alkyl, $C_{1-6}$ haloalkyl, halogen, and cyano; $R^{2a}$ and $R^{2b}$ are identical or different and are each independently selected from the group consisting of a hydrogen atom, $C_{1-6}$ alkyl, $C_{1-6}$ haloalkyl, $C_{1-6}$ hydroxyalkyl, $C_{1-6}$ alkoxy, $C_{1-6}$ haloalkoxy, halogen, cyano, amino, hydroxy, 3- to 8-membered cycloalkyl, and 3- to 8-membered heterocyclyl; each $R^4$ is identical or different and is independently selected from the group consisting of a hydrogen atom, $C_{1-6}$ alkyl, $C_{1-6}$ haloalkyl, $C_{1-6}$ hydroxyalkyl, $C_{1-6}$ alkoxy, $C_{1-6}$ haloalkoxy, halogen, cyano, $-NR^8R^9$, hydroxy, and $-C(O)R^{10}$; $R^8$ and $R^9$ are identical or different and are each independently selected from the group consisting of a hydrogen atom, $C_{1-6}$ alkyl, $C_{1-6}$ haloalkyl, and $C_{1-6}$ hydroxyalkyl, wherein the $C_{1-6}$ alkyl is optionally substituted with one or more substituents selected from the group consisting of $C_{1-6}$ alkoxy, $C_{1-6}$ haloalkoxy, and cyano; $R^{10}$ is $-OR^{11}$, and $R^{11}$ is selected from the group consisting of a hydrogen atom, $C_{1-6}$ alkyl, $C_{1-6}$ haloalkyl, 3- to 8-membered cycloalkyl, and 3- to 8-membered heterocyclyl, wherein the $C_{1-6}$ alkyl, 3- to 8-membered cycloalkyl, and 3- to 8-membered heterocyclyl are each independently and optionally substituted with one or more substituents selected from the group consisting of $-OR^{12}$ and $-C(O)OR^{12}$, and $R^{12}$ is a hydrogen atom or $C_{1-6}$ alkyl; t is 1 or 2.

In some embodiments of the present disclosure, the compound of general formula (III) or the pharmaceutically acceptable salt thereof is provided, wherein X is a chemical bond; $R^{3b}$ is selected from the group consisting of a hydrogen atom, $C_{1-6}$ alkyl, $C_{1-6}$ haloalkyl, $C_{1-6}$ hydroxyalkyl, $C_{1-6}$ alkoxy, and $C_{1-6}$ haloalkoxy; ring A is phenyl or 5- to 6-membered heteroaryl; $R^1$ is selected from the group consisting of a hydrogen atom, $C_{1-6}$ alkyl, $C_{1-6}$ haloalkyl, halogen, and cyano; $R^{2a}$ and $R^{2b}$ are identical or different and are each independently selected from the group consisting of a hydrogen atom, $C_{1-6}$ alkyl, $C_{1-6}$ haloalkyl, $C_{1-6}$ hydroxyalkyl, $C_{1-6}$ alkoxy, $C_{1-6}$ haloalkoxy, halogen, cyano, amino, hydroxy, 3- to 8-membered cycloalkyl, and 3- to 8-membered heterocyclyl; each $R^4$ is identical or different and is independently selected from the group consisting of a hydrogen atom, $C_{1-6}$ alkyl, $C_{1-6}$ haloalkyl, $C_{1-6}$ hydroxyalkyl, $C_{1-6}$ alkoxy, $C_{1-6}$ haloalkoxy, halogen, cyano, $-NR^8R^9$, hydroxy, and $-C(O)R^{10}$; $R^8$ and $R^9$ are identical or different and are each independently selected from the group consisting of a hydrogen atom, $C_{1-6}$ alkyl, $C_{1-6}$ haloalkyl, and $C_{1-6}$ hydroxyalkyl, wherein the $C_{1-6}$ alkyl is optionally substituted with one or more substituents selected from the group consisting of $C_{1-6}$ alkoxy, $C_{1-6}$ haloalkoxy, and cyano; $R^{10}$ is selected from the group consisting of a hydrogen atom, hydroxy, $C_{1-6}$ alkyl, $C_{1-6}$ haloalkyl, and $C_{1-6}$ alkoxy; t is 1 or 2.

In some embodiments of the present disclosure, the compound of general formula (III) or the pharmaceutically acceptable salt thereof is provided, wherein X is a chemical bond; $R^{3b}$ is selected from the group consisting of a hydrogen atom, $C_{1-6}$ alkyl, $C_{1-6}$ haloalkyl, $C_{1-6}$ hydroxyalkyl, $C_{1-6}$ alkoxy, and $C_{1-6}$ haloalkoxy; ring A is phenyl or 5- to 6-membered heteroaryl; $R^1$ is selected from the group consisting of a hydrogen atom, $C_{1-6}$ alkyl, $C_{1-6}$ haloalkyl, halogen, and cyano; $R^{2a}$ and $R^{2b}$ are identical or different and are each independently selected from the group consisting of a hydrogen atom, $C_{1-6}$ alkyl, $C_{1-6}$ haloalkyl, $C_{1-6}$ hydroxyalkyl, $C_{1-6}$ alkoxy, $C_{1-6}$ haloalkoxy, and halogen; each $R^4$ is identical or different

and is independently selected from the group consisting of a hydrogen atom, $C_{1-6}$ alkyl, $C_{1-6}$ haloalkyl, $C_{1-6}$ hydroxyalkyl, $C_{1-6}$ alkoxy, $C_{1-6}$ haloalkoxy, halogen, cyano, $-NR^8R^9$, hydroxy, and $-C(O)R^{10}$; $R^8$ and $R^9$ are identical or different and are each independently selected from the group consisting of a hydrogen atom, $C_{1-6}$ alkyl, $C_{1-6}$ haloalkyl, and $C_{1-6}$ hydroxyalkyl, wherein the $C_{1-6}$ alkyl is optionally substituted with one or more substituents selected from the group consisting of $C_{1-6}$ alkoxy, $C_{1-6}$ haloalkoxy, and cyano; $R^{10}$ is selected from the group consisting of a hydrogen atom, hydroxy, $C_{1-6}$ alkyl, $C_{1-6}$ haloalkyl, and $C_{1-6}$ alkoxy; t is 1 or 2.

In some embodiments of the present disclosure, the compound of general formula (IV) or the pharmaceutically acceptable salt thereof is provided, wherein X is a chemical bond; $R^{3b}$ is selected from the group consisting of a hydrogen atom, $C_{1-6}$ alkyl, $C_{1-6}$ haloalkyl, $C_{1-6}$ hydroxyalkyl, $C_{1-6}$ alkoxy, and $C_{1-6}$ haloalkoxy; ring A is phenyl or 5- to 6-membered heteroaryl; $R^1$ is selected from the group consisting of a hydrogen atom, $C_{1-6}$ alkyl, $C_{1-6}$ haloalkyl, halogen, and cyano; $R^{2a}$ and $R^{2b}$ are identical or different and are each independently selected from the group consisting of $C_{1-6}$ alkyl, $C_{1-6}$ alkoxy, and 3- to 6-membered cycloalkyl; each $R^4$ is identical or different and is independently selected from the group consisting of a hydrogen atom, $C_{1-6}$ alkyl, $C_{1-6}$ haloalkyl, $C_{1-6}$ hydroxyalkyl, $C_{1-6}$ alkoxy, $C_{1-6}$ haloalkoxy, halogen, hydroxy, and $-C(O)R^{10}$; $R^{10}$ is selected from the group consisting of hydroxy,

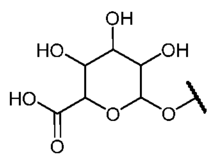, 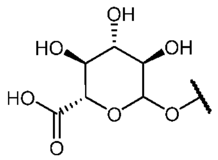,

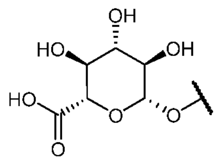, and 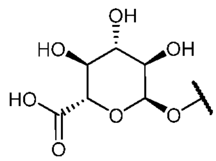;

t is 1 or 2.

In some embodiments of the present disclosure, the compound of general formula (IV) or the pharmaceutically acceptable salt thereof is provided, wherein X is a chemical bond; $R^{3b}$ is selected from the group consisting of a hydrogen atom, $C_{1-6}$ alkyl, $C_{1-6}$ haloalkyl, $C_{1-6}$ hydroxyalkyl, $C_{1-6}$ alkoxy, and $C_{1-6}$ haloalkoxy; ring A is phenyl or 5- to 6-membered heteroaryl; $R^1$ is selected from the group consisting of a hydrogen atom, $C_{1-6}$ alkyl, $C_{1-6}$ haloalkyl, halogen, and cyano; $R^{2a}$ and $R^{2b}$ are identical or different and are each independently selected from the group consisting of $C_{1-6}$ alkyl, $C_{1-6}$ alkoxy, and 3- to 6-membered cycloalkyl; each $R^4$ is identical or different and is independently selected from the group consisting of a hydrogen atom, $C_{1-6}$ alkyl, $C_{1-6}$ haloalkyl, $C_{1-6}$ hydroxyalkyl, $C_{1-6}$ alkoxy, $C_{1-6}$ haloalkoxy, halogen, hydroxy, and $-C(O)R^{10}$; $R^{10}$ is hydroxy; t is 1 or 2.

In some embodiments of the present disclosure, the compound of general formula (IV) or the pharmaceutically acceptable salt thereof is provided, wherein X is a chemical bond; $R^{3b}$ is selected from the group consisting of a hydrogen atom, $C_{1-6}$ alkyl, $C_{1-6}$ haloalkyl, $C_{1-6}$ hydroxyalkyl, $C_{1-6}$ alkoxy, and $C_{1-6}$ haloalkoxy; ring A is phenyl or 5- to 6-membered heteroaryl; $R^1$ is selected from the group consisting of a hydrogen atom, $C_{1-6}$ alkyl, $C_{1-6}$ haloalkyl, halogen, and cyano; $R^{2a}$ and $R^{2b}$ are identical or different and are each independently $C_{1-6}$ alkyl or $C_{1-6}$ alkoxy; each $R^4$ is identical or different and is independently selected from the group consisting of a hydrogen atom, $C_{1-6}$ alkyl, $C_{1-6}$ haloalkyl, $C_{1-6}$ hydroxyalkyl, $C_{1-6}$ alkoxy, $C_{1-6}$ haloalkoxy, halogen, hydroxy, and $-C(O)R^{10}$; $R^{10}$ is hydroxy; t is 1 or 2.

In some embodiments of the present disclosure, the compound of general formula (V') or the pharmaceutically acceptable salt thereof is provided, wherein $R^{2a}$ and $R^{2b}$ are identical or different and are each independently selected from the group consisting of a hydrogen atom, $C_{1-6}$ alkyl, $C_{1-6}$ haloalkyl, $C_{1-6}$ hydroxyalkyl, $C_{1-6}$ alkoxy, $C_{1-6}$ haloalkoxy, halogen, cyano, amino, hydroxy, 3- to 8-membered cycloalkyl, and 3- to 8-membered heterocyclyl; $R^{3b}$ is selected from the group consisting of a hydrogen atom, $C_{1-6}$ alkyl, $C_{1-6}$ haloalkyl, $C_{1-6}$ hydroxyalkyl, $C_{1-6}$ alkoxy, and $C_{1-6}$ haloalkoxy; $R^{10}$ is selected from the group consisting of hydroxy,

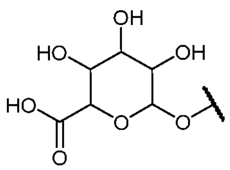, 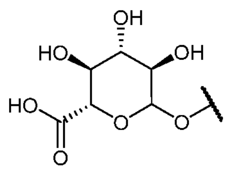,

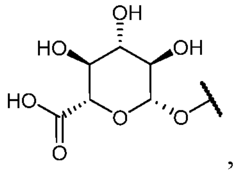, and 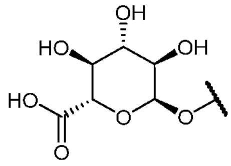.

In some embodiments of the present disclosure, the compound of general formula (V'-1) or general formula (V'-2) or the pharmaceutically acceptable salt thereof is provided, wherein $R^{2a}$ and $R^{2b}$ are identical or different and are each independently selected from the group consisting of $C_{1-6}$ alkyl, $C_{1-6}$ alkoxy, and 3- to 6-membered cycloalkyl; $R^{3b}$ is $C_{1-6}$ alkoxy; $R^{10}$ is selected from the group consisting of hydroxy,

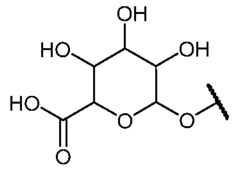,

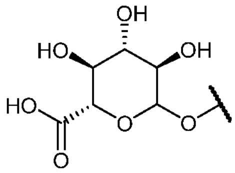, 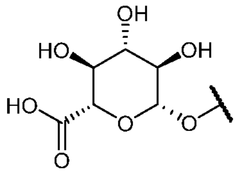, and 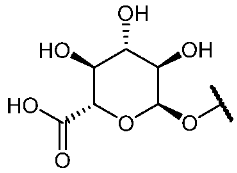.

In some embodiments of the present disclosure, the compound of general formula (V) or the pharmaceutically acceptable salt thereof is provided, wherein $R^{2a}$ and $R^{2b}$ are identical or different and are each independently selected from the group consisting of a hydrogen atom, $C_{1-6}$ alkyl, $C_{1-6}$ haloalkyl, $C_{1-6}$ hydroxyalkyl, $C_{1-6}$ alkoxy, $C_{1-6}$ haloalkoxy, halogen, cyano, amino, hydroxy, 3- to 8-membered cycloalkyl, and 3- to 8-membered heterocyclyl; $R^{3b}$ is selected from the group consisting of a hydrogen atom, $C_{1-6}$ alkyl, $C_{1-6}$ haloalkyl, $C_{1-6}$ hydroxyalkyl, $C_{1-6}$ alkoxy, and $C_{1-6}$ haloalkoxy.

In some embodiments of the present disclosure, the compound of general formula (V") or the pharmaceutically acceptable salt thereof is provided, wherein $R^{2a}$ and $R^{2b}$ are identical or different and are each independently selected from the group consisting of a hydrogen atom, $C_{1-6}$ alkyl, $C_{1-6}$ haloalkyl, $C_{1-6}$ hydroxyalkyl, $C_{1-6}$ alkoxy, $C_{1-6}$ haloalkoxy, halogen, cyano, amino, hydroxy, 3- to 8-membered cycloalkyl, and 3- to 8-membered heterocyclyl; $R^{3b}$ is $C_{1-6}$ alkoxy.

In some embodiments of the present disclosure, the compound of general formula (V-1) or general formula (V-2) or the pharmaceutically acceptable salt thereof is provided, wherein $R^{2a}$ and $R^{2b}$ are identical or different and are each independently selected from the group consisting of $C_{1-6}$ alkyl, $C_{1-6}$ alkoxy, and 3- to 6-membered cycloalkyl; $R^{3b}$ is $C_{1-6}$ alkoxy.

In some embodiments of the present disclosure, the compound of general formula (V-1) or general formula (V-2) or the pharmaceutically acceptable salt thereof is provided, wherein $R^{2a}$ is selected from the group consisting of $C_{1-6}$ alkyl, $C_{1-6}$ alkoxy, and 3- to 6-membered cycloalkyl, and $R^{2b}$ is $C_{1-6}$ alkyl; $R^{3b}$ is $C_{1-6}$ alkoxy.

Table A. Typical compounds disclosed herein include, but are not limited to:

| Example No. | Structures and names of compounds |
|---|---|
| | 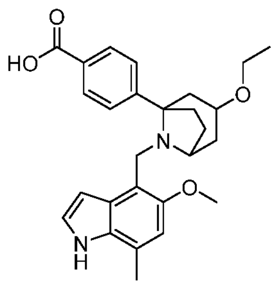 |
| | 4-(3-Ethoxy-8-((5-methoxy-7-methyl-1*H*-indol-4-yl)methyl)-8-azabi cyclo[3.2.1]octan-1-yl)benzoic acid |

(continued)

| Example No. | Structures and names of compounds |
|---|---|
| 1 | 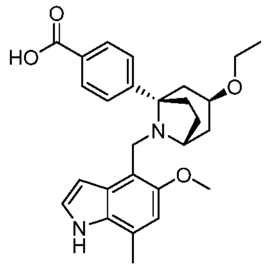 (±)-*rel*-(1*S*,3*S*,5*R*) **1** |
| | (±)-*rel*-4-((1*S*,3*S*,5*R*)-3-Ethoxy-8-((5-methoxy-7-methyl-1*H*-indol-4-yl)methyl)-8-azabicyclo[3.2.1] octan-1-yl)benzoic acid 1 |
| 2 | 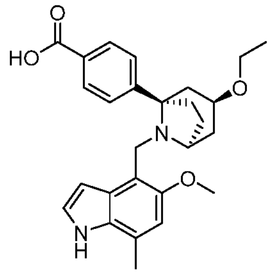 (±)-*rel*-(1*R*,3*S*,5*S*) **2** |
| | (±)-*rel*-4-((1*R*,3*S*,5*S*)-3-Ethoxy-8-((5-methoxy-7-methyl-1*H*-indol-4-yl)methyl)-8-azabicyclo[3.2.1] octan-1-yl)benzoic acid 2 |
| | 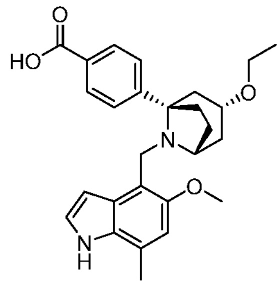 |
| | 4-((1*S*,3*R*,5*R*)-3-Ethoxy-8-((5-methoxy-7-methyl-1*H*-indol-4-yl)met hyl)-8-azabicyclo[3.2.1]octan-1-yl) benzoic acid |
| 1-1 | 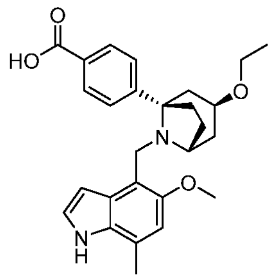 **1-1** |
| | 4-((1*S*,3*S*,5*R*)-3-Ethoxy-8-((5-methoxy-7-methyl-1*H*-indol-4-yl)meth yl)-8-azabicyclo[3.2.1]octan-1-yl) benzoic acid 1-1 |

(continued)

| Example No. | Structures and names of compounds |
|---|---|
| 1-2 | 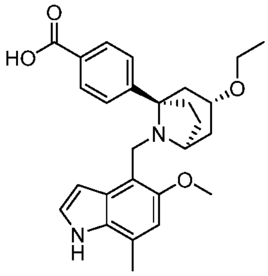<br>**1-2** 4-((1*R*,3*R*,5*S*)-3-Ethoxy-8-((5-methoxy-7-methyl-1*H*-indol-4-yl)met hyl)-8-azabicyclo[3.2.1]octan-1-yl)benzoic acid 1-2 |
| | 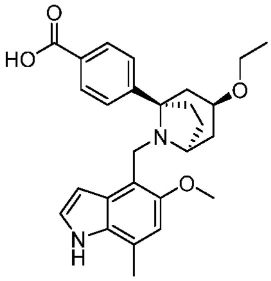<br>4-((1*R*,3*S*,5*S*)-3-Ethoxy-8-((5-methoxy-7-methyl-1*H*-indol-4-yl)meth yl)-8-azabicyclo[3.2.1]octan-1-yl) benzoic acid |
| 3 | 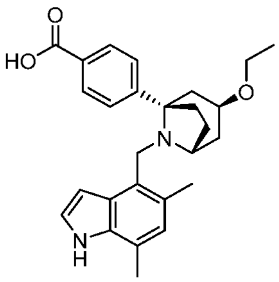<br>**3** 4-((1*S*,3*S*,5*R*)-8-((5,7-Dimethyl-1*H*-indol-4-yl)methyl)-3-ethoxy-8-a zabicyclo[3.2.1]octan-1-yl)benzoic acid 3 |
| 4 | 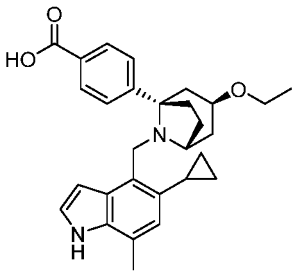<br>**4**<br>4-((1*S*,3*S*,5*R*)-8-((5-Cyclopropyl-7-methyl-1*H*-indol-4-yl)methyl)-3-ethoxy-8-azabicyclo[3.2.1]octan-1-yl)benzoic acid 4 |

(continued)

| Example No. | Structures and names of compounds |
|---|---|
| | 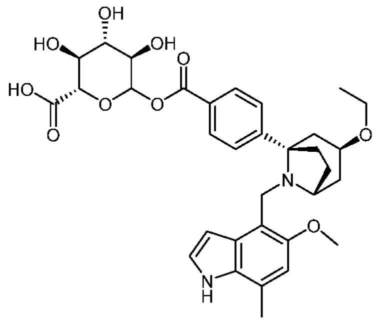 |
| | (2*S*,3*S*,4*S*,5*R*)-6-((4-((1*S*,3*S*,5*R*)-3-Ethoxy-8-((5-methoxy-7-methyl-1*H*-indol-4-yl)methyl)-8-azabicyclo[3.2.1]octan-1-yl)benzoyl)oxy)-3, 4,5-trihydroxytetrahydro-2H-pyran-2-carboxylic acid |
| 5 | 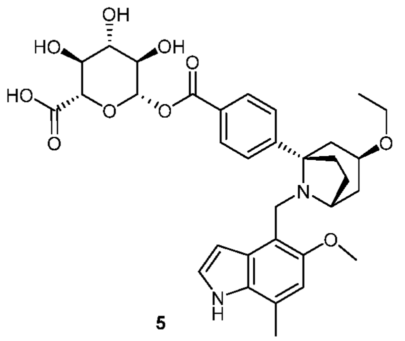 5 |
| | (2*S*,3*S*,4*S*,5*R*,6*S*)-6-((4-((1*S*,3*S*,5*R*)-3-Ethoxy-8-((5-methoxy-7-meth yl-1*H*-indol-4-yl)methyl)-8-azabicyclo[3.2.1]octan-1-yl)benzoyl)oxy )-3,4,5-trihydroxytetrahydro-2*H*-pyran-2-carboxylic acid 5 |
| | 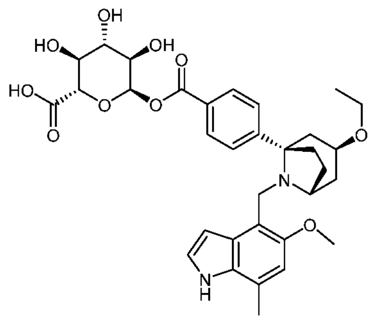 |
| | (2*S*,3*S*,4*S*,5*R*,6*R*)-6-((4-((1*S*,3*S*,5*R*)-3-Ethoxy-8-((5-methoxy-7-meth yl-1H-indol-4-yl)methyl)-8-azabicyclo[3.2.1]octan-1-yl)benzoyl)oxy )-3,4,5-trihydroxytetrahydro-2*H*-pyran-2-carboxylic acid |

Another aspect of the present disclosure relates to a compound of general formula (IVA) or a pharmaceutically acceptable salt thereof:

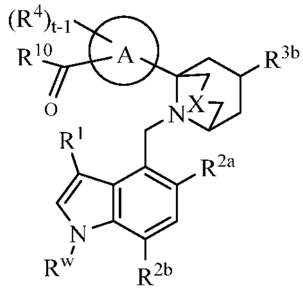

(IVA)

wherein:

$R^w$ is an amino protecting group; preferably, $R^w$ is tert-butoxycarbonyl or p-toluenesulfonyl; more preferably, $R^w$ is tert-butoxycarbonyl;
$R^{10}$ is alkoxy; preferably, $R^{10}$ is $C_{1-6}$ alkoxy; more preferably, $R^{10}$ is methoxy;
t is 1 or 2;
ring A, X, $R^1$, $R^{2a}$, $R^{2b}$, $R^{3b}$, and $R^4$ are as defined in general formula (IV).

Another aspect of the present disclosure relates to a compound of general formula (IVB) or a pharmaceutically acceptable salt thereof:

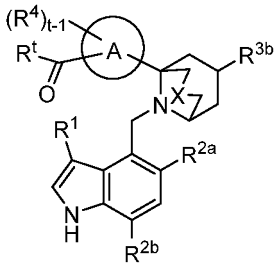

(IVB)

wherein:

$R^t$ is

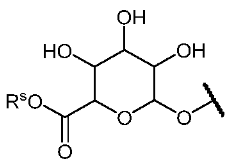 , 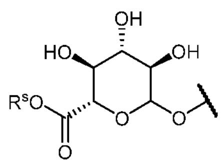 , 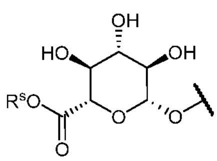 , and 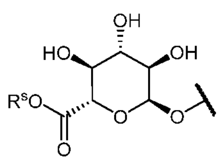 ;

$R^s$ is a hydroxy protecting group; preferably, $R^s$ is allyl;
t is 1 or 2;
ring A, X, $R^1$, $R^{2a}$, $R^{2b}$, $R^{3b}$, and $R^4$ are as defined in general formula (IV).

Another aspect of the present disclosure relates to a compound of general formula (VA) or a pharmaceutically acceptable salt thereof:

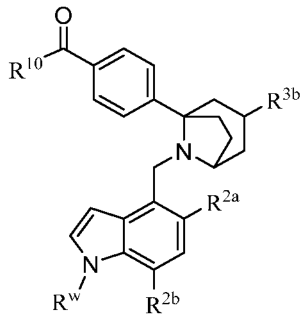

(VA)

wherein:

$R^w$ is an amino protecting group; preferably, $R^w$ is tert-butoxycarbonyl or p-toluenesulfonyl; more preferably, $R^w$ is tert-butoxycarbonyl;
$R^{10}$ is alkoxy; preferably, $R^{10}$ is $C_{1-6}$ alkoxy; more preferably, $R^{10}$ is methoxy;
$R^{2a}$, $R^{2b}$, and $R^{3b}$ are as defined in general formula (V).

Another aspect of the present disclosure relates to a compound of general formula (V-1A) or a pharmaceutically acceptable salt thereof:

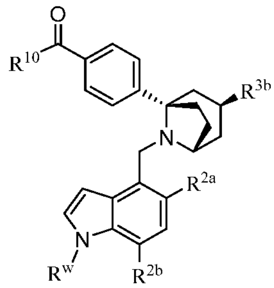

(V-1A)

wherein:

$R^w$ is an amino protecting group; preferably, $R^w$ is tert-butoxycarbonyl or p-toluenesulfonyl; more preferably, $R^w$ is tert-butoxycarbonyl;
$R^{10}$ is alkoxy; preferably, $R^{10}$ is $C_{1-6}$ alkoxy; more preferably, $R^{10}$ is methoxy;
$R^{2a}$, $R^{2b}$, and $R^{3b}$ are as defined in general formula (V-1).

Another aspect of the present disclosure relates to a compound of general formula (V-2A) or a pharmaceutically acceptable salt thereof:

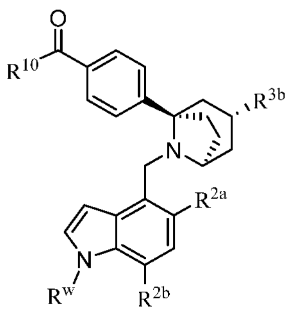

(V-2A)

wherein:

$R^w$ is an amino protecting group; preferably, $R^w$ is tert-butoxycarbonyl or p-toluenesulfonyl; more preferably, $R^w$ is tert-butoxycarbonyl;

$R^{10}$ is alkoxy; preferably, $R^{10}$ is $C_{1-6}$ alkoxy; more preferably, $R^{10}$ is methoxy;

$R^{2a}$, $R^{2b}$, and $R^{3b}$ are as defined in general formula (V-2).

Another aspect of the present disclosure relates to a compound of general formula (V'A) or a pharmaceutically acceptable salt thereof:

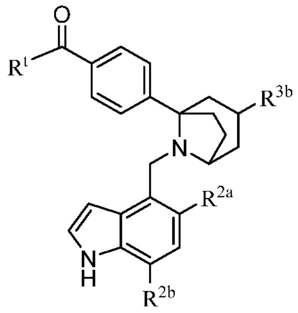

(V'A)

wherein:

$R^t$ is

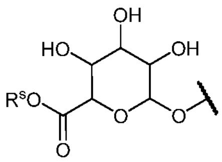
,
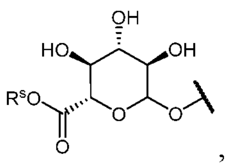
,
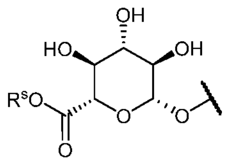
, and
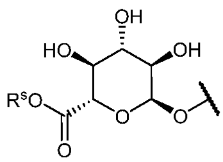
;

$R^s$ is a hydroxy protecting group; preferably, $R^s$ is allyl;

$R^{2a}$, $R^{2b}$, and $R^{3b}$ are as defined in general formula (V').

Another aspect of the present disclosure relates to a compound of general formula (V'-1A) or a pharmaceutically acceptable salt thereof:

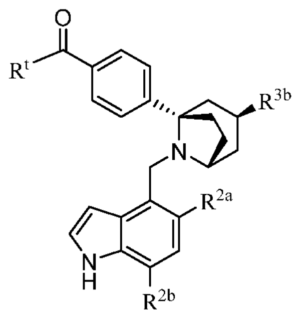

(V'-1A)

wherein:

$R^t$ is

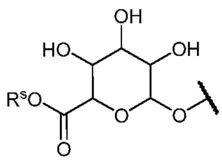, 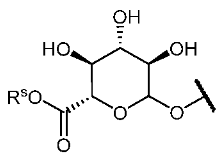, 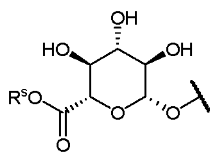, and 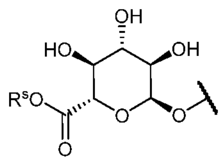;

$R^s$ is a hydroxy protecting group; preferably, $R^s$ is allyl;
$R^{2a}$, $R^{2b}$, and $R^{3b}$ are as defined in general formula (V'-1).

Another aspect of the present disclosure relates to a compound of general formula (V'-2A) or a pharmaceutically acceptable salt thereof:

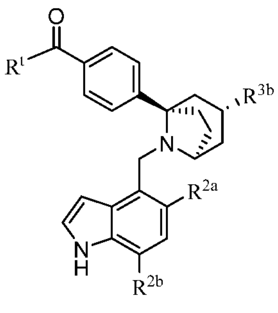

(V'-2A)

wherein:

$R^t$ is

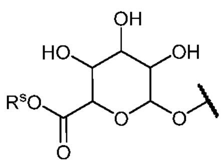, 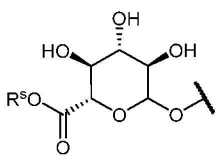, 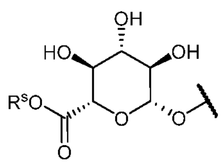, and 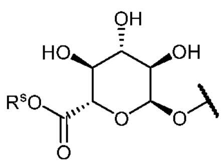;

$R^s$ is a hydroxy protecting group; preferably, $R^s$ is allyl;
$R^{2a}$, $R^{2b}$, and $R^{3b}$ are as defined in general formula (V'-2).

Table B. Typical compounds disclosed herein include, but are not limited to:

| Example No. | Structures and names of compounds |
|---|---|
| 1k | 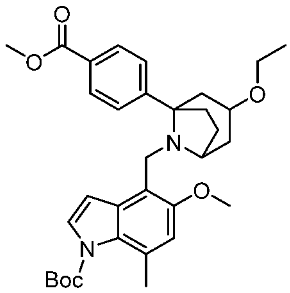 **1k** |
| | tert-Butyl 4-((3-ethoxy-1-(4-(methoxycarbonyl)phenyl)-8-azabicyclo[3.2.1]oc tan-8-yl)methyl)-5-methoxy-7-methyl-1*H*-indole-1-carboxylate **1k** |
| 3j | 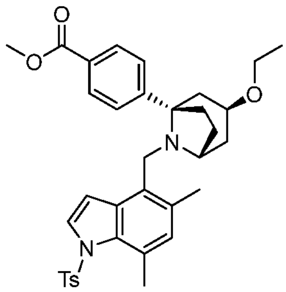 **3j** |
| | Methyl 4-((1*S*,3*S*,5*R*)-8-((5,7-dimethyl-1-tosyl-1*H*-indol-4-yl)methyl)-3-eth oxy-8-azabicyclo[3.2.1] octan-1-yl)benzoate **3j** |
| 4i | 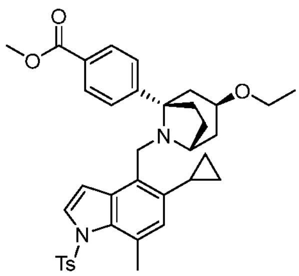 **4i** |
| | Methyl 4-((1*S*,3*S*,5*R*)-8-((5-cyclopropyl-7-methyl-1-tosyl-1*H*-indol-4-yl)m ethyl)-3-ethoxy-8-azabicyclo [3.2.1]octan-1-yl)benzoate 4i |

(continued)

| Example No. | Structures and names of compounds |
|---|---|
| 5c | 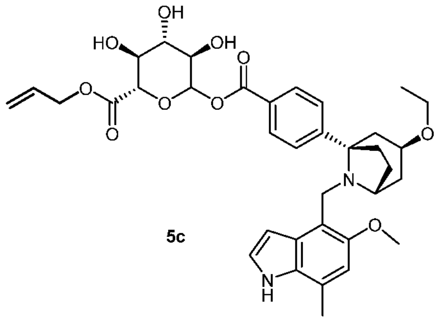 5c |
| | Allyl (2*S*,3*S*,4*S*,5*R*)-6-((4-((1*S*,3*S*,5*R*)-3-ethoxy-8-((5-methoxy-7-methyl-1H-indol-4-yl)methyl)-8-azabicyclo[3.2.1]octan-1-yl)benzoyl)oxy)-3,4,5-trihydroxytetrahydro-2H-pyran-2-carboxylate 5c |
| | 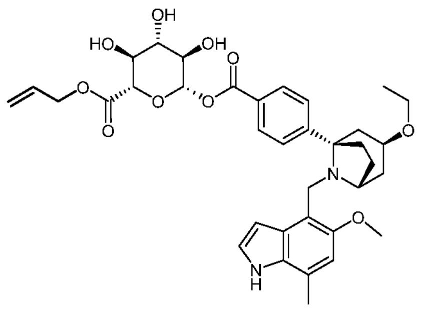 |
| | Allyl (2*S*,3*S*,4*S*,5*R*,6*S*)-6-((4-((1*S*,3*S*,5*R*)-3-ethoxy-8-((5-methoxy-7-meth yl-1H-indol-4-yl)methyl)-8-azabicyclo[3.2.1]octan-1-yl)benzoyl)ox y)-3,4,5-trihydroxytetrahydro-2H-pyran-2-carboxylate |
| | 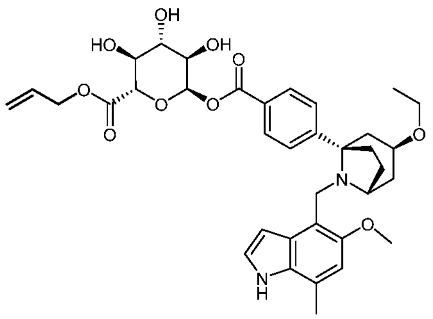 |
| | Allyl |
| | (2*S*,3*S*,4*S*,5*R*,6*R*)-6-((4-((1*S*,3*S*,5*R*)-3-ethoxy-8-((5-methoxy-7-met hyl-1H-indol-4-yl)methyl)-8-azabicyclo[3.2.1]octan-1-yl)benzoyl)o xy)-3,4,5-trihydroxytetrahydro-2H-pyran-2-carboxylate |

Another aspect of the present disclosure relates to a method for preparing a compound of general formula (IV) or a pharmaceutically acceptable salt thereof, which comprises:

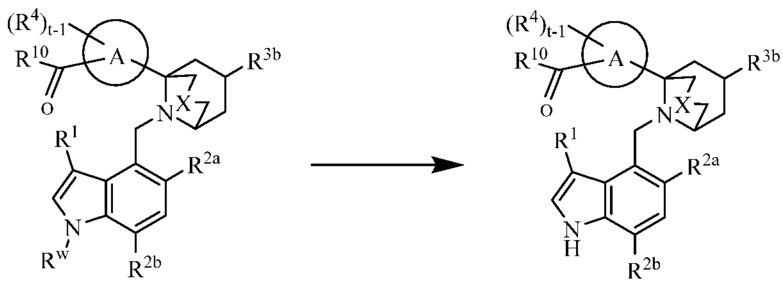

(IVA) (IV)

removing protecting group $R^w$ from a compound of general formula (IVA) in which $R^{10}$ is alkoxy (preferably $C_{1-6}$ alkoxy, and more preferably methoxy) or a pharmaceutically acceptable salt thereof and conducting a hydrolysis reaction to give a compound of general formula (IV) in which $R^{10}$ is hydroxy or a pharmaceutically acceptable salt thereof, wherein:

$R^w$ is an amino protecting group; preferably, $R^w$ is tert-butoxycarbonyl or p-toluenesulfonyl; more preferably, $R^w$ is tert-butoxycarbonyl;
t is 1 or 2;
ring A, X, $R^1$, $R^{2a}$, $R^{2b}$, $R^{3b}$, and $R^4$ are as defined in general formula (IV).

Another aspect of the present disclosure relates to a method for preparing a compound of general formula (IV) or a pharmaceutically acceptable salt thereof, which comprises:

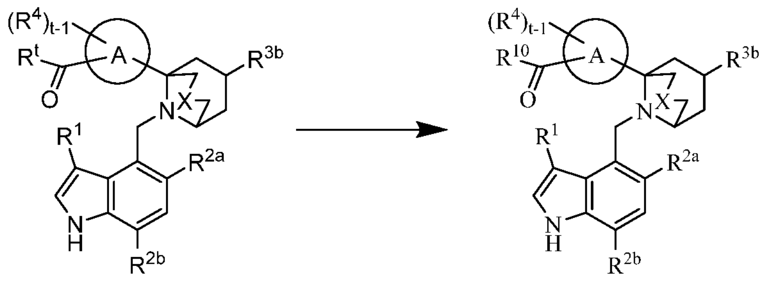

(IVB) (IV)

removing protecting group $R^s$ from a compound of general formula (IVB) or a pharmaceutically acceptable salt thereof to give the compound of general formula (IV) or the pharmaceutically acceptable salt thereof, and

optionally performing resolution to give the compound of general formula (IV) or the pharmaceutically acceptable salt thereof in a single configuration,
wherein:

$R^t$ is

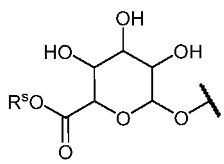 , 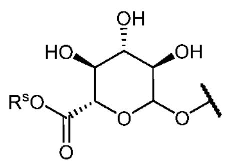 , 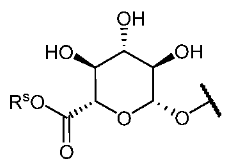 , and 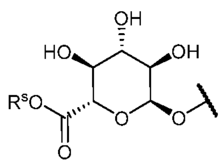 ;

$R^s$ is a hydroxy protecting group; preferably, $R^s$ is allyl;
$R^{10}$ is selected from the group consisting of

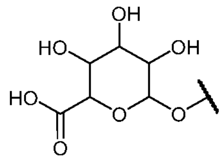, 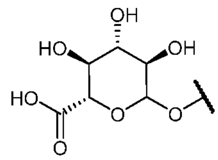,

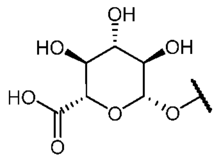, and 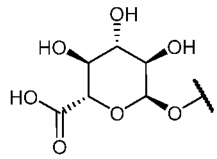;

t is 1 or 2;
ring A, X, $R^1$, $R^{2a}$, $R^{2b}$, $R^{3b}$, and $R^4$ are as defined in general formula (IV).

Another aspect of the present disclosure relates to a method for preparing a compound of general formula (V) or a pharmaceutically acceptable salt thereof, which comprises:

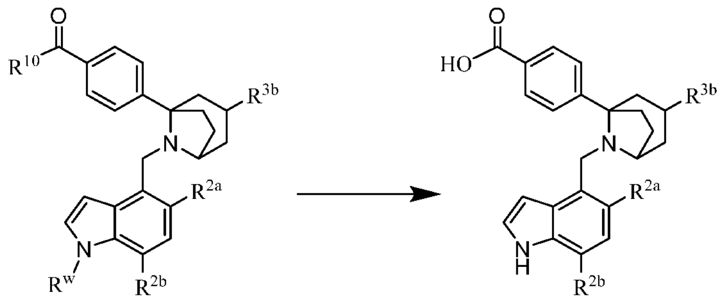

(VA) (V)

removing protecting group $R^w$ from a compound of general formula (VA) in which $R^{10}$ is alkoxy (preferably $C_{1-6}$ alkoxy, and more preferably methoxy) or a pharmaceutically acceptable salt thereof and conducting a hydrolysis reaction to give the compound of general formula (V) or the pharmaceutically acceptable salt thereof, wherein:

$R^w$ is an amino protecting group; preferably, $R^w$ is tert-butoxycarbonyl or p-toluenesulfonyl; more preferably, $R^w$ is tert-butoxycarbonyl;
$R^{2a}$, $R^{2b}$, and $R^{3b}$ are as defined in general formula (V).

Another aspect of the present disclosure relates to a method for preparing a compound of general formula (V-1) or a pharmaceutically acceptable salt thereof, which comprises:

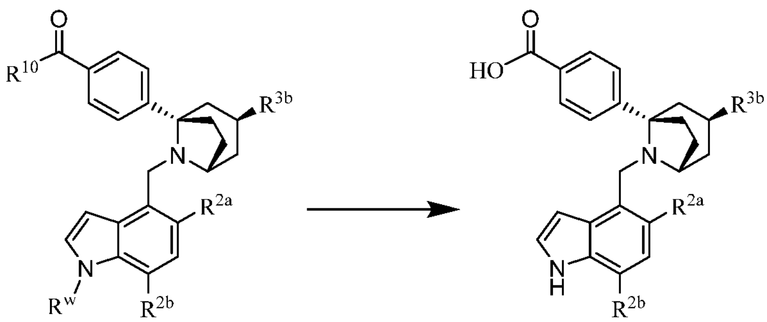

(V-1A) (V-1)

removing protecting group $R^w$ from a compound of general formula (V-1A) in which $R^{10}$ is alkoxy (preferably $C_{1-6}$ alkoxy,

and more preferably methoxy) or a pharmaceutically acceptable salt thereof and conducting a hydrolysis reaction to give the compound of general formula (V-1) or the pharmaceutically acceptable salt thereof, wherein:

$R^w$ is an amino protecting group; preferably, $R^w$ is tert-butoxycarbonyl or p-toluenesulfonyl; more preferably, $R^w$ is tert-butoxycarbonyl;
$R^{2a}$, $R^{2b}$, and $R^{3b}$ are as defined in general formula (V-1).

Another aspect of the present disclosure relates to a method for preparing a compound of general formula (V-2) or a pharmaceutically acceptable salt thereof, which comprises:

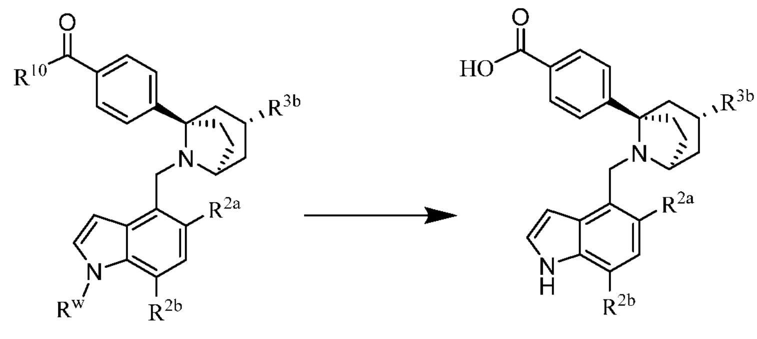

(V-2A) (V-2)

removing protecting group $R^w$ from a compound of general formula (V-2A) in which $R^{10}$ is alkoxy (preferably $C_{1-6}$ alkoxy, and more preferably methoxy) or a pharmaceutically acceptable salt thereof and conducting a hydrolysis reaction to give the compound of general formula (V-2) or the pharmaceutically acceptable salt thereof, wherein:

$R^w$ is an amino protecting group; preferably, $R^w$ is tert-butoxycarbonyl or p-toluenesulfonyl; more preferably, $R^w$ is tert-butoxycarbonyl;
$R^{2a}$, $R^{2b}$, and $R^{3b}$ are as defined in general formula (V-2).

Another aspect of the present disclosure relates to a method for preparing a compound of general formula (V') or a pharmaceutically acceptable salt thereof, which comprises:

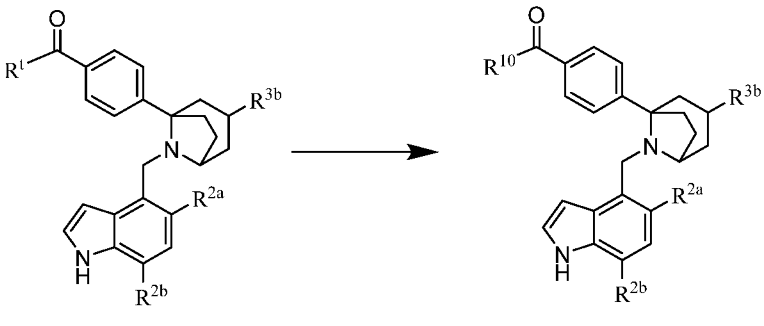

(V'A) (V')

removing protecting group $R^s$ from a compound of general formula (V'A) or a pharmaceutically acceptable salt thereof to give the compound of general formula (V') or the pharmaceutically acceptable salt thereof, and

optionally performing resolution to give the compound of general formula (V') or the pharmaceutically acceptable salt thereof in a single configuration,
wherein:

$R^t$ is

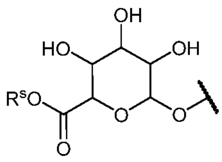, 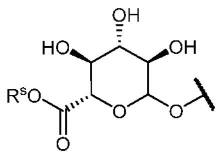, 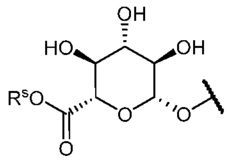, and 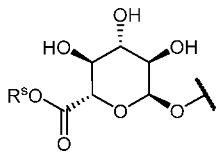;

$R^s$ is a hydroxy protecting group; preferably, $R^s$ is allyl;

$R^{10}$ is selected from the group consisting of

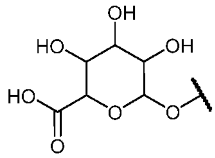, 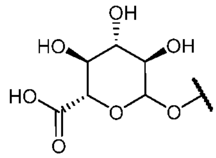,

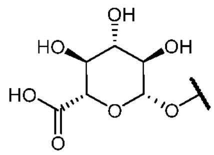, and 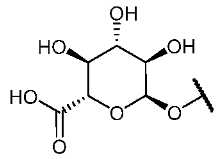;

$R^{2a}$, $R^{2b}$, and $R^{3b}$ are as defined in general formula (V').

Another aspect of the present disclosure relates to a method for preparing a compound of general formula (V'-1) or a pharmaceutically acceptable salt thereof, which comprises:

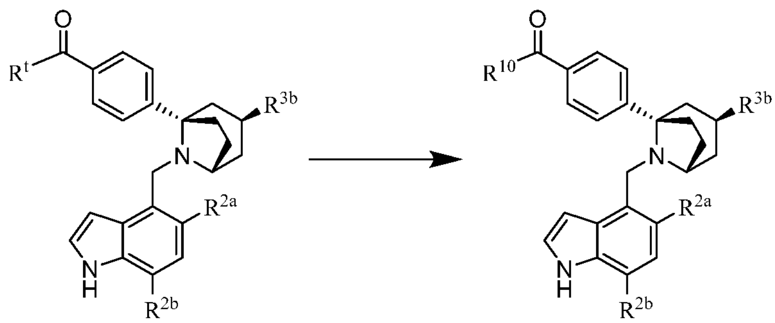

(V'-1A) (V'-1)

removing protecting group $R^s$ from a compound of general formula (V'-1A) or a pharmaceutically acceptable salt thereof to give the compound of general formula (V'-1) or the pharmaceutically acceptable salt thereof, and

optionally performing resolution to give the compound of general formula (V'-1) or the pharmaceutically acceptable salt thereof in a single configuration,
wherein:

$R^t$ is

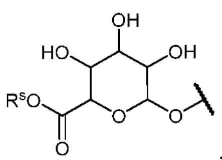, 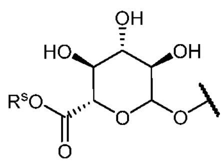, 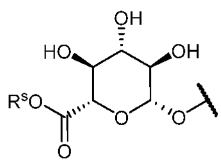, and 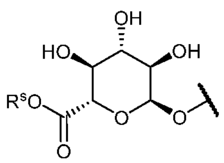;

$R^s$ is a hydroxy protecting group; preferably, $R^s$ is allyl;

$R^{10}$ is selected from the group consisting of

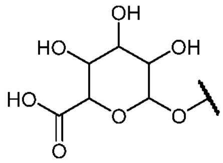
,
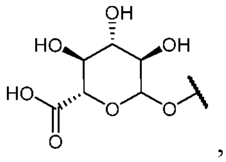
,

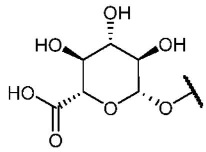
, and
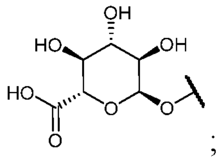
;

$R^{2a}$, $R^{2b}$, and $R^{3b}$ are as defined in general formula (V'-1).

Another aspect of the present disclosure relates to a method for preparing a compound of general formula (V'-2) or a pharmaceutically acceptable salt thereof, which comprises:

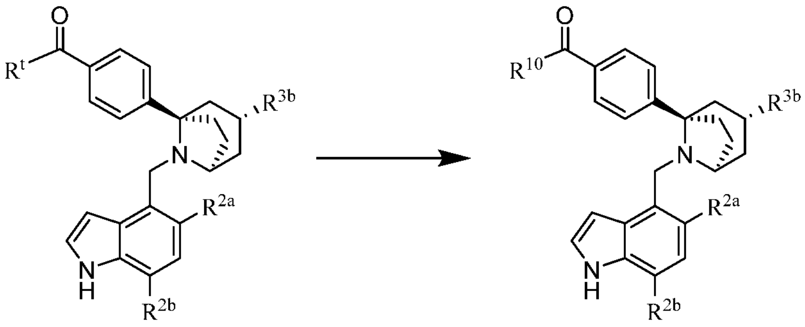

(V'-2A) (V'-2)

removing protecting group $R^s$ from a compound of general formula (V'-2A) or a pharmaceutically acceptable salt thereof to give the compound of general formula (V'-2) or the pharmaceutically acceptable salt thereof, and

optionally performing resolution to give the compound of general formula (V'-2) or the pharmaceutically acceptable salt thereof in a single configuration,
wherein:

$R^t$ is

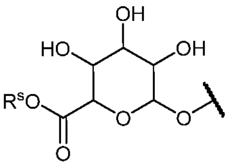
,
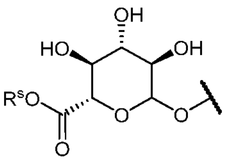
,
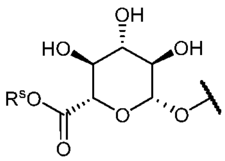
, and
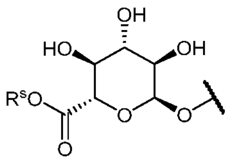
;

$R^s$ is a hydroxy protecting group; preferably, $R^s$ is allyl;
$R^{10}$ is selected from the group consisting of

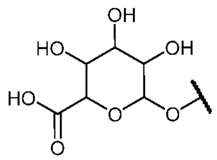, 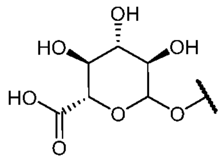,

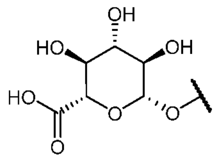, and 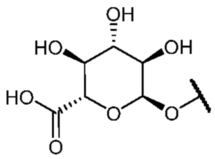;

$R^{2a}$, $R^{2b}$, and $R^{3b}$ are as defined in general formula (V-2).

Another aspect of the present disclosure relates to a pharmaceutical composition comprising the compound of general formula (I), general formula (II), general formula (III), general formula (IV), general formula (V), general formula (V''), general formula (V-1), general formula (V-2), general formula (V'), general formula (V'-1), or general formula (V'-2) of the present disclosure and a compound shown in Table A, or pharmaceutically acceptable salts thereof, and one or more pharmaceutically acceptable carriers, diluents, or excipients.

The present disclosure further relates to use of the compound of general formula (I), general formula (II), general formula (III), general formula (IV), general formula (V), general formula (V''), general formula (V-1), general formula (V-2), general formula (V'), general formula (V'-1), or general formula (V'-2) and a compound shown in Table A, or pharmaceutically acceptable salts thereof, or the pharmaceutical composition comprising same in the preparation of a medicament for inhibiting activation of the complement alternative pathway, preferably, in the preparation of a medicament for inhibiting factor B.

The present disclosure further relates to use of the compound of general formula (I), general formula (II), general formula (III), general formula (IV), general formula (V), general formula (V''), general formula (V-1), general formula (V-2), general formula (V'), general formula (V'-1), or general formula (V'-2) and a compound shown in Table A, or pharmaceutically acceptable salts thereof, or the pharmaceutical composition comprising same in the preparation of a medicament for treating and/or preventing a disease or disorder mediated by activation of the complement alternative pathway; preferably in the preparation of a medicament for treating and/or preventing a disease or disorder mediated by factor B, wherein the disease or disorder is selected from the group consisting of glomerulopathy (such as glomerulonephritis), hemolytic uremic syndrome (such as E. coli-induced hemolytic uremic syndrome), atypical haemolytic uraemic syndrome (aHUS), paroxysmal nocturnal hemoglobinuria, age-related macular degeneration, geographic atrophy, diabetic retinopathy, uveitis (such as anterior uveitis, posterior uveitis, and intermediate uveitis), retinitis pigmentosa, macular edema, Behcet's uveitis, multifocal choroiditis, Vogt-Koyanagi-Harada syndrome, birdshot retino-chorioditis, sympathetic ophthalmia, ocular dicatricial pemphigoid, ocular pemphigus, nonartertic ischemic optic neuropathy, post-operative inflammation, retinal vein occlusion, neurological disorders, multiple sclerosis, stroke, Guillain-Barré syndrome, traumatic brain injury, Parkinson's disease, disorders of inappropriate or undesirable complement activation, hemodialysis complications, hyperacute allograft rejection, xenograft rejection, interleukin-2 induced toxicity during IL-2 therapy, Crohn's disease, adult respiratory distress syndrome, myocarditis, post-ischemic reperfusion conditions, myocardial infarction, balloon angioplasty, post-pump syndrome in cardiopulmonary bypass or renal bypass, atherosclerosis, hemodialysis, renal ischemia, mesenteric artery reperfusion after aortic reconstruction, infectious disease or sepsis, immune complex disorders, autoimmune diseases (such as rheumatoid arthritis, systemic lupus erythematosus (SLE), immune thrombocytopenia, and cold agglutinin syndrome), systemic lupus erythematosus nephritis, proliferative nephritis, liver fibrosis, hemolytic anemia, myasthenia gravis, tissue regeneration, neural regeneration, dyspnea, hemoptysis, acute respiratory distress syndrome (ARDS), asthma, chronic obstructive pulmonary disease (COPD), emphysema, pulmonary embolisms and infarcts, pneumonia (such as hypersensitivity pneumonitis), fibrogenic dust diseases, pulmonary fibrosis, asthma, allergy, bronchoconstriction, parasitic diseases, Goodpasture's syndrome, pulmonary vasculitis, pauci-immune vasculitis, immune complex-associated inflammation, antiphospholipid syndrome, and obesity, wherein the glomerulopathy is preferably selected from the group consisting of C3 glomerulopathy (such as C3 glomerulonephritis), IgA nephropathy (such as primary IgA nephropathy), and membranous glomerulonephritis; and more preferably in the preparation of a medicament for treating and/or preventing C3 glomerulopathy (such as C3 glomerulonephritis), IgA nephropathy, membranous glomerulonephritis, atypical haemolytic uraemic syndrome, and paroxysmal nocturnal hemoglobinuria.

The present disclosure further relates to use of the compound of general formula (I), general formula (II), general formula (III), general formula (IV), general formula (V), general formula (V''), general formula (V-1), general formula (V-2), general formula (V-

2), general formula (V'), general formula (V'-1), or general formula (V'-2) and a compound shown in Table A, or pharmaceutically acceptable salts thereof, or the pharmaceutical composition comprising same in the preparation of a medicament for treating and/or preventing a disease or disorder mediated by factor B, wherein the disease or disorder is an inflammatory disorder.

The present disclosure further relates to a method for inhibiting activation of the complement alternative pathway, preferably a method for inhibiting factor B, which comprises administering to a patient in need thereof a therapeutically effective amount of the compound of general formula (I), general formula (II), general formula (III), general formula (IV), general formula (V), general formula (V"), general formula (V-1), general formula (V-2), general formula (V'), general formula (V'-1), or general formula (V'-2) and a compound shown in Table A, or pharmaceutically acceptable salts thereof, or the pharmaceutical composition comprising same.

The present disclosure further relates to a method for treating and/or preventing a disease or disorder mediated by activation of the complement alternative pathway, preferably a method for treating and/or preventing a disease or disorder mediated by factor B, which comprises administering to a patient in need thereof a therapeutically effective amount of the compound of general formula (I), general formula (II), general formula (III), general formula (IV), general formula (V), general formula (V"), general formula (V-1), general formula (V-2), general formula (V'), general formula (V'-1), or general formula (V'-2) and a compound shown in Table A, or pharmaceutically acceptable salts thereof, or the pharmaceutical composition comprising same.

The present disclosure further relates to a compound of general formula (I), general formula (II), general formula (III), general formula (IV), general formula (V), general formula (V"), general formula (V-1), general formula (V-2), general formula (V'), general formula (V'-1), or general formula (V'-2) and a compound shown in Table A, or pharmaceutically acceptable salts thereof, or a pharmaceutical composition comprising same for use as a medicament.

The present disclosure further relates to a compound of general formula (I), general formula (II), general formula (III), general formula (IV), general formula (V), general formula (V"), general formula (V-1), general formula (V-2), general formula (V'), general formula (V'-1), or general formula (V'-2) and a compound shown in Table A, or pharmaceutically acceptable salts thereof, or a pharmaceutical composition comprising same for use in inhibiting activation of the complement alternative pathway, preferably for use in inhibiting factor B.

The present disclosure further relates to a compound of general formula (I), general formula (II), general formula (III), general formula (IV), general formula (V), general formula (V"), general formula (V-1), general formula (V-2), general formula (V'), general formula (V'-1), or general formula (V'-2) and a compound shown in Table A, or pharmaceutically acceptable salts thereof, or a pharmaceutical composition comprising same for use in treating and/or preventing a disease or disorder mediated by activation of the complement alternative pathway, preferably for use in treating and/or preventing a disease or disorder mediated by factor B.

The disease or disorder mediated by activation of the complement alternative pathway (preferably a disease or disorder mediated by factor B) described in the present disclosure is selected from the group consisting of glomerulopathy (such as glomerulonephritis), hemolytic uremic syndrome (such as E. coli-induced hemolytic uremic syndrome), atypical haemolytic uraemic syndrome, paroxysmal nocturnal hemoglobinuria, age-related macular degeneration, geographic atrophy, diabetic retinopathy, uveitis (such as anterior uveitis, posterior uveitis, and intermediate uveitis), retinitis pigmentosa, macular edema, Behcet's uveitis, multifocal choroiditis, Vogt-Koyanagi-Harada syndrome, birdshot retinochorioditis, sympathetic ophthalmia, ocular dicatricial pemphigoid, ocular pemphigus, nonartertic ischemic optic neuropathy, post-operative inflammation, retinal vein occlusion, neurological disorders, multiple sclerosis, stroke, Guillain-Barré syndrome, traumatic brain injury, Parkinson's disease, disorders of inappropriate or undesirable complement activation, hemodialysis complications, hyperacute allograft rejection, xenograft rejection, interleukin-2 induced toxicity during IL-2 therapy, Crohn's disease, adult respiratory distress syndrome, myocarditis, post-ischemic reperfusion conditions, myocardial infarction, balloon angioplasty, post-pump syndrome in cardiopulmonary bypass or renal bypass, atherosclerosis, hemodialysis, renal ischemia, mesenteric artery reperfusion after aortic reconstruction, infectious disease or sepsis, immune complex disorders, autoimmune diseases (such as rheumatoid arthritis, systemic lupus erythematosus (SLE), immune thrombocytopenia, and cold agglutinin syndrome), systemic lupus erythematosus nephritis, proliferative nephritis, liver fibrosis, hemolytic anemia, myasthenia gravis, tissue regeneration, neural regeneration, dyspnea, hemoptysis, acute respiratory distress syndrome (ARDS), asthma, chronic obstructive pulmonary disease (COPD), emphysema, pulmonary embolisms and infarcts, pneumonia, fibrogenic dust diseases, pulmonary fibrosis, asthma, allergy, bronchoconstriction, hypersensitivity pneumonitis, parasitic diseases, Goodpasture's syndrome, pulmonary vasculitis, pauci-immune vasculitis, immune complex-associated inflammation, antiphospholipid syndrome, and obesity, wherein the glomerulopathy is preferably selected from the group consisting of C3 glomerulopathy (such as C3 glomerulonephritis), IgA nephropathy (such as primary IgA nephropathy), and membranous glomerulonephritis; preferably, the disease or disorder is selected from the group consisting of C3 glomerulopathy (such as C3 glomerulonephritis), IgA nephropathy, membranous glomerulonephritis, atypical haemolytic uraemic syndrome, and paroxysmal nocturnal hemoglobinuria.

The disease or disorder mediated by factor B described in the present disclosure is an inflammatory disorder.

The active compound may be formulated into a form suitable for administration by any suitable route, and one or more pharmaceutically acceptable carriers are used to formulate the composition of the present disclosure by conventional methods. Thus, the active compound of the present disclosure may be formulated into a variety of dosage forms for oral administration, administration by injection (e.g., intravenous, intramuscular, or subcutaneous), or administration by inhalation or insufflation. The compound of the present disclosure may also be formulated into a sustained-release dosage form, such as tablets, hard or soft capsules, aqueous or oily suspensions, emulsions, injections, dispersible powders or granules, suppositories, lozenges or syrups.

As a general guide, the active compound is preferably in a form of a unit dose, or in a form of a single dose that can be self-administered by a patient. The unit dose of the compound or composition of the present disclosure may be in a tablet, capsule, cachet, vial, powder, granule, lozenge, suppository, regenerating powder, or liquid formulation. A suitable unit dose may be 0.1-1000 mg.

The pharmaceutical composition of the present disclosure may comprise, in addition to the active compound, one or more auxiliary materials selected from the group consisting of a filler (diluent), a binder, a wetting agent, a disintegrant, an excipient, and the like. Depending on the method of administration, the composition may comprise 0.1 wt.% to 99 wt.% of the active compound.

The tablet comprises the active ingredient and a non-toxic pharmaceutically acceptable excipient that is used for mixing and is suitable for the preparation of the tablet. Such an excipient may be an inert excipient, a granulating agent, a disintegrant, a binder, and a lubricant. Such a tablet may be uncoated or may be coated by known techniques for masking the taste of the drug or delaying the disintegration and absorption of the drug in the gastrointestinal tract and thus enabling sustained release of the drug over a longer period.

An oral formulation in a soft gelatin capsule where the active ingredient is mixed with an inert solid diluent or with a water-soluble carrier or oil vehicle may also be provided. An aqueous suspension comprises the active substance and an excipient that is used for mixing and is suitable for the preparation of the aqueous suspension. Such an excipient is a suspending agent, a dispersant, or a wetting agent. The aqueous suspension may also comprise one or more preservatives, one or more colorants, one or more flavoring agents, and one or more sweeteners.

An oil suspension may be formulated by suspending the active ingredient in a vegetable oil, or in a mineral oil. The oil suspension may comprise a thickening agent. Sweeteners and flavoring agents may be added to provide a palatable formulation. Antioxidants may also be added to preserve the compositions.

The pharmaceutical composition of the present disclosure may also be in the form of an oil-in-water emulsion. The oil phase may be a vegetable oil or a mineral oil, or a mixture thereof. Suitable emulsifiers may be naturally occurring phospholipids, and the emulsion may also comprise a sweetener, a flavoring agent, a preservative, and an antioxidant. Such a formulation may also comprise a palliative, a preservative, a colorant, and an antioxidant.

The pharmaceutical composition of the present disclosure may be in the form of a sterile injectable aqueous solution. Acceptable vehicles or solvents that can be used include water, Ringer's solution, and isotonic sodium chloride solution. A sterile injectable formulation may be a sterile injectable oil-in-water microemulsion in which an active ingredient is dissolved in an oil phase. The injection or microemulsion can be locally injected into the bloodstream of a patient in large quantities. Alternatively, it may be desirable to administer the solution and microemulsion in such a way as to maintain a constant circulating concentration of the compound of the present disclosure. To maintain such a constant concentration, a continuous intravenous delivery device may be used. An example of such a device is a Deltec CADD-PLUS. TM. 5400 intravenous injection pump.

The pharmaceutical composition of the present disclosure may be in the form of a sterile injectable aqueous or oil suspension for intramuscular and subcutaneous administration. The suspension can be prepared according to the prior art using suitable dispersants or wetting agents and suspending agents. The sterile injectable formulation may also be a sterile injection or suspension prepared in a parenterally acceptable non-toxic diluent or solvent. In addition, a sterile fixed oil may be conventionally used as a solvent or a suspending medium. For this purpose, any blend fixed oil may be used. In addition, fatty acids may also be used to prepare injections.

The compound of the present disclosure may be administered in the form of a suppository for rectal administration. Such a pharmaceutical composition can be prepared by mixing a drug with a suitable non-irritating excipient which is a solid at ambient temperature but a liquid in the rectum and therefore will melt in the rectum to release the drug.

The compound of the present disclosure can be administered in the form of dispersible powders and granules that are formulated into aqueous suspensions by adding water. Such a pharmaceutical composition can be prepared by mixing the active ingredient with a dispersant or a wetting agent, a suspending agent, or one or more preservatives. As is well known to those skilled in the art, the dose of the drug administered depends on a variety of factors, including but not limited to, the activity of the particular compound used, the age of the patient, the body weight of the patient, the health condition of the patient, the behavior of the patient, the diet of the patient, the time of administration, the route of administration, the rate of excretion, the combination of drugs, the severity of the disease, and the like. In addition, the optimal treatment regimen, such as the mode of administration, the daily dose of the compound, or the type of pharmaceutically acceptable salts, can be verified according to conventional treatment regimens.

Description of the terms

Unless otherwise stated, the terms used in the specification and claims have the following meanings.

The term "alkyl" refers to a saturated straight-chain or branched-chain aliphatic hydrocarbon group having 1 to 20 (e.g., 1, 2, 3, 4, 5, 6, 7, 8, 9, 10, 11, 12, 13, 14, 15, 16, 17, 18, 19, or 20) carbon atoms (i.e., $C_{1-20}$ alkyl). The alkyl is preferably an alkyl group having 1 to 12 carbon atoms (i.e., $C_{1-12}$ alkyl), more preferably an alkyl group having 1 to 6 carbon atoms (i.e., $C_{1-6}$ alkyl). Non-limiting examples include: methyl, ethyl, n-propyl, isopropyl, n-butyl, isobutyl, tert-butyl, sec-butyl, n-pentyl, 1,1-dimethylpropyl, 1,2-dimethylpropyl, 2,2-dimethylpropyl, 1-ethylpropyl, 2-methylbutyl, 3-methylbutyl, n-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, n-heptyl, 2-methylhexyl, 3-methylhexyl, 4-methylhexyl, 5-methylhexyl, 2,3-dimethylpentyl, 2,4-dimethylpentyl, 2,2-dimethylpentyl, 3,3-dimethylpentyl, 2-ethylpentyl, 3-ethylpentyl, n-octyl, 2,3-dimethylhexyl, 2,4-dimethylhexyl, 2,5-dimethylhexyl, 2,2-dimethylhexyl, 3,3-dimethylhexyl, 4,4-dimethylhexyl, 2-ethylhexyl, 3-ethylhexyl, 4-ethylhexyl, 2-methyl-2-ethylpentyl, 2-methyl-3-ethylpentyl, n-nonyl, 2-methyl-2-ethylhexyl, 2-methyl-3-ethylhexyl, 2,2-diethylpentyl, n-decyl, 3,3-diethylhexyl, 2,2-diethylhexyl, and various side-chain isomers thereof, and the like. Most preferred is a lower alkyl group having 1 to 6 carbon atoms; non-limiting examples include: methyl, ethyl, n-propyl, isopropyl, n-butyl, isobutyl, tert-butyl, sec-butyl, n-pentyl, 1,1-dimethylpropyl, 1,2-dimethylpropyl, 2,2-dimethylpropyl, 1-ethylpropyl, 2-methylbutyl, 3-methylbutyl, n-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, and the like. Alkyl may be substituted or unsubstituted. When substituted, it may be substituted at any accessible connection site, and the substituent is preferably selected from one or more of a D atom, halogen, alkyl, alkoxy, haloalkyl, haloalkoxy, cycloalkyloxy, heterocyclyloxy, hydroxy, hydroxyalkyl, cyano, amino, nitro, cycloalkyl, heterocyclyl, aryl, and heteroaryl.

The term "alkylene" refers to a divalent alkyl group, wherein the alkyl is as defined above; it has 1 to 20 (e.g., 1, 2, 3, 4, 5, 6, 7, 8, 9, 10, 11, 12, 13, 14, 15, 16, 17, 18, 19, or 20) carbon atoms (i.e., $C_{1-20}$ alkylene). The alkylene is preferably an alkylene group having 1 to 12 carbon atoms (i.e., $C_{1-12}$ alkylene), and more preferably an alkylene group containing 1 to 6 carbon atoms (i.e., $C_{1-6}$ alkylene). Non-limiting examples of alkylene include, but are not limited to, methylene ($-CH_2-$), 1,1-ethylene ($-CH(CH_3)-$), 1,2-ethylene ($-CH_2CH_2-$), 1,1-propylene ($-CH(CH_2CH_3)-$), 1,2-propylene ($-CH_2CH(CH_3)-$), 1,3-propylene ($-CH_2CH_2CH_2-$), 1,4-butylene ($-CH_2CH_2CH_2CH_2-$), and the like. Alkylene may be substituted or unsubstituted. When substituted, it may be substituted at any accessible connection site, and the substituent is preferably selected from one or more of alkyl, alkenyl, alkynyl, alkoxy, haloalkoxy, cycloalkyloxy, heterocyclyloxy, alkylthio, alkylamino, halogen, sulfhydryl, hydroxy, nitro, cyano, cycloalkyl, heterocyclyl, aryl, heteroaryl, cycloalkoxy, heterocycloalkoxy, cycloalkylthio, heterocycloalkylthio, and oxo.

The term "alkenyl" refers to an alkyl group containing at least one carbon-carbon double bond in the molecule, wherein the alkyl is as defined above, and it has alkenyl of 2 to 12 (e.g., 2, 3, 4, 5, 6, 7, 8, 9, 10, 11, and 12) carbon atoms (i.e., $C_{2-12}$ alkenyl). The alkenyl is preferably an alkenyl group having 2 to 6 carbon atoms (i.e., $C_{2-6}$ alkenyl). Non-limiting examples include: ethenyl, propenyl, isopropenyl, butenyl, and the like. Alkenyl may be substituted or unsubstituted. When it is substituted, the substituent is preferably selected from one or more of alkyl, alkoxy, halogen, haloalkyl, haloalkoxy, cycloalkyloxy, heterocyclyloxy, hydroxy, hydroxyalkyl, cyano, amino, nitro, cycloalkyl, heterocyclyl, aryl, and heteroaryl.

The term "alkynyl" refers to an alkyl group containing at least one carbon-carbon triple bond in the molecule, wherein the alkyl is as defined above, and it has 2 to 12 (e.g., 2, 3, 4, 5, 6, 7, 8, 9, 10, 11, and 12) carbon atoms (i.e., $C_{2-12}$ alkynyl). The alkynyl is preferably an alkynyl group having 2 to 6 carbon atoms (i.e., $C_{2-6}$ alkynyl). Non-limiting examples include: ethynyl, propynyl, butynyl, pentynyl, hexynyl, and the like. Alkynyl may be substituted or unsubstituted. When it is substituted, the substituent is preferably selected from one or more of alkyl, alkoxy, halogen, haloalkyl, haloalkoxy, cycloalkyloxy, heterocyclyloxy, hydroxy, hydroxyalkyl, cyano, amino, nitro, cycloalkyl, heterocyclyl, aryl, and heteroaryl.

The term "alkoxy" refers to -O-(alkyl), wherein the alkyl is as defined above. Non-limiting examples of alkoxy include: methoxy, ethoxy, propoxy, and butoxy. Alkoxy may be optionally substituted or unsubstituted. When substituted, the substituent is preferably selected from the group consisting of a D atom, halogen, alkyl, alkoxy, haloalkyl, haloalkoxy, cycloalkyloxy, heterocyclyloxy, hydroxy, hydroxyalkyl, cyano, amino, nitro, cycloalkyl, heterocyclyl, aryl, and heteroaryl.

The term "cycloalkyl" refers to a saturated or partially unsaturated monocyclic or polycyclic hydrocarbon substituent, and the cycloalkyl ring has 3 to 20 (e.g., 3, 4, 5, 6, 7, 8, 9, 10, 11, 12, 13, 14, 15, 16, 17, 18, 19, or 20) carbon atoms (i.e., 3- to 20-membered cycloalkyl), preferably 3 to 12 carbon atoms (i.e., 3- to 12-membered cycloalkyl), more preferably 3 to 8 carbon atoms (i.e., 3- to 8-membered cycloalkyl), and most preferably 3 to 6 carbon atoms (i.e., 3- to 6-membered cycloalkyl). Non-limiting examples of monocyclic cycloalkyl include: cyclopropyl, cyclobutyl, cyclopentyl, cyclopentenyl, cyclohexyl, cyclohexenyl, cyclohexadienyl, cycloheptyl, cycloheptatrienyl, cyclooctyl, and the like. Polycyclic cycloalkyl includes spiro cycloalkyl, fused cycloalkyl, and bridged cycloalkyl.

The term "spiro cycloalkyl" refers to a 5- to 20-membered polycyclic group in which monocyclic rings share one

carbon atom (referred to as the spiro atom), and it may contain one or more double bonds. It is preferably 6- to 14-membered, and more preferably 7- to 10-membered (e.g., 7-membered, 8-membered, 9-membered, or 10-membered). According to the number of the spiro atoms shared among the rings, the spiro cycloalkyl may be monospiro cycloalkyl or polyspiro cycloalkyl (e.g., bispiro cycloalkyl), preferably monospiro cycloalkyl and bispiro cycloalkyl, and more preferably 3-membered/5-membered, 3-membered/6-membered, 4-membered/4-membered, 4-membered/5-membered, 4-membered/6-membered, 5-membered/5-membered, 5-membered/6-membered, 6-membered/4-membered, 6-membered/5-membered, or 6-membered/6-membered monospiro cycloalkyl. Non-limiting examples of spiro cycloalkyl include:

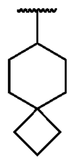 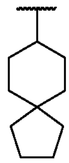 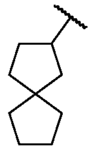 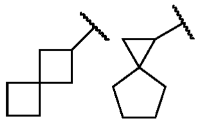.

The term "fused cycloalkyl" refers to a 5- to 20-membered all-carbon polycyclic group in which rings share a pair of adjacent carbon atoms, wherein one or more of the rings may contain one or more double bonds. It is preferably 6- to 14-membered, and more preferably 7- to 10-membered (e.g., 7-membered, 8-membered, 9-membered, or 10-membered). According to the number of constituent rings, the fused cycloalkyl may be bicyclic, tricyclic, tetracyclic, etc., preferably bicyclic or tricyclic, and more preferably 3-membered/4-membered, 3-membered/5-membered, 3-membered/6-membered, 4-membered/4-membered, 4-membered/5-membered, 4-membered/6-membered, 5-membered/3-membered, 5-membered/4-membered, 5-membered/5-membered, 5-membered/6-membered, 5-membered/7-membered, 6-membered/3 -membered, 6-membered/4-membered, 6-membered/5-membered, 6-membered/6-membered, 6-membered/7-membered, 7-membered/5-membered, or 7-membered/6-membered bicyclic alkyl. Non-limiting examples of fused cycloalkyl include:

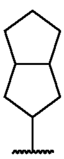 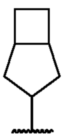 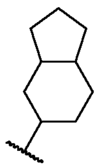 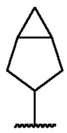 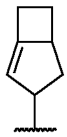 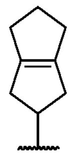 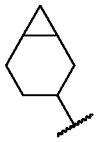 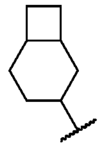 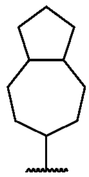.

The term "bridged cycloalkyl" refers to a 5- to 20-membered all-carbon polycyclic group in which any two rings share two carbon atoms that are not directly connected, and it may contain one or more double bonds. It is preferably 6- to 14-membered, and more preferably 7- to 10-membered (e.g., 7-membered, 8-membered, 9-membered, or 10-membered). According to the number of constituent rings, the bridged cycloalkyl may be bicyclic, tricyclic, tetracyclic, etc., preferably bicyclic, tricyclic, or tetracyclic, and more preferably bicyclic or tricyclic. Non-limiting examples of bridged cycloalkyl include:

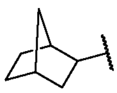 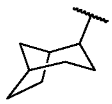 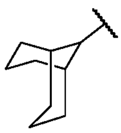 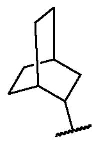 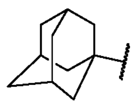.

The cycloalkyl ring includes those in which the cycloalkyl described above (including monocyclic, spiro, fused, and bridged ones) fuses with an aryl, heteroaryl, or heterocycloalkyl ring, wherein the ring connected to the parent structure is cycloalkyl; non-limiting examples include

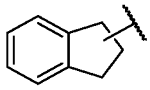, 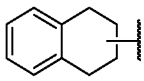, 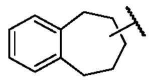,

etc., preferably

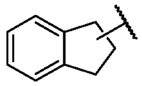 and 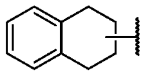.

Cycloalkyl may be substituted or unsubstituted. When substituted, it may be substituted at any accessible connection site, and the substituent is preferably selected from one or more of halogen, alkyl, alkoxy, haloalkyl, haloalkoxy, cycloalkyloxy, heterocyclyloxy, hydroxy, hydroxyalkyl, cyano, amino, nitro, cycloalkyl, heterocyclyl, aryl, and heteroaryl.

The term "heterocyclyl" refers to a saturated or partially unsaturated monocyclic or polycyclic substituent having 3 to 20 ring atoms, of which one or more are heteroatoms selected from the group consisting of nitrogen, oxygen, and sulfur, and the sulfur may optionally be substituted with oxo (i.e., to form sulfoxide or sulfone), but excluding a cyclic portion of -O-O-, -O-S-, or -S-S-; the other ring atoms are carbon. Preferably, it has 3 to 12 (e.g. 3, 4, 5, 6, 7, 8, 9, 10, 11, and 12) ring atoms, of which 1 to 4 (e.g. 1, 2, 3, and 4) are heteroatoms (i.e. 3- to 12-membered heterocyclyl); further preferably, it has 3 to 8 ring atoms (e.g., 3, 4, 5, 6, 7, and 8), of which 1 to 3 are heteroatoms (e.g., 1, 2, and 3) (i.e., 3- to 8-membered heterocyclyl); more preferably, it has 3 to 6 ring atoms, of which 1 to 3 are heteroatoms (i.e. 3- to 6-membered heterocyclyl); most preferably, it has 5 or 6 ring atoms, of which 1 to 3 are heteroatoms (i.e. 5- or 6-membered heterocyclyl). Non-limiting examples of monocyclic heterocyclyl include: pyrrolidinyl, tetrahydropyranyl, 1,2,3,6-tetrahydropyridinyl, piperidinyl, piperazinyl, morpholinyl, thiomorpholinyl, homopiperazinyl, and the like. Polycyclic heterocyclyl includes spiro heterocyclyl, fused heterocyclyl, and bridged heterocyclyl.

The term "spiro heterocyclyl" refers to a 5- to 20-membered polycyclic heterocyclyl group in which monocyclic rings share one atom (referred to as the spiro atom), wherein one or more ring atoms are heteroatoms selected from the group consisting of nitrogen, oxygen, and sulfur, and the sulfur may optionally be substituted with oxo (i.e., to form sulfoxide or sulfone); the other ring atoms are carbon. It may contain one or more double bonds. It is preferably 6- to 14-membered, and more preferably 7- to 10-membered (e.g., 7-membered, 8-membered, 9-membered, or 10-membered). According to the number of spiro atoms shared among the rings, the spiro heterocyclyl may be monospiro heterocyclyl or polyspiro heterocyclyl (e.g., bispiro heterocyclyl), preferably monospiro heterocyclyl and bispiro heterocyclyl, and more preferably 3-membered/5-membered, 3-membered/6-membered, 4-membered/4-membered, 4-membered/5-membered, 4-membered/6-membered, 5-membered/5-membered, 5-membered/6-membered, or 6-membered/6-membered monospiro heterocyclyl. Non-limiting examples of spiro heterocyclyl include:

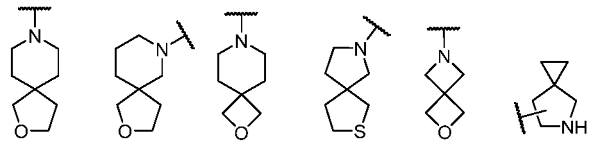.

The term "fused heterocyclyl" refers to a 5- to 20-membered polycyclic heterocyclyl group in which rings share a pair of adjacent atoms, and one or more of the rings may contain one or more double bonds, wherein one or more ring atoms are heteroatoms selected from the group consisting of nitrogen, oxygen, and sulfur, and the sulfur may optionally be substituted with oxo (i.e., to form sulfoxide or sulfone); the other ring atoms are carbon. It is preferably 6- to 14-membered, and more preferably 7- to 10-membered (e.g., 7-membered, 8-membered, 9-membered, or 10-membered). According to the number of constituent rings, the fused heterocyclyl may be bicyclic, tricyclic, tetracyclic, etc., preferably bicyclic or tricyclic, and more preferably 3-membered/4-membered, 3-membered/5-membered, 3-membered/6-membered, 4-membered/4-membered, 4-membered/5-membered, 4-membered/6-membered, 5-membered/3-membered, 5-membered/4-membered, 5-membered/5-membered, 5-membered/6-membered, 5-membered/7-membered, 6-membered/3-membered, 6-membered/4-membered, 6-membered/5-membered, 6-membered/6-membered, 6-membered/7-membered, 7-membered/5-membered, or 7-membered/6-membered bicyclic fused heterocyclyl. Non-limiting examples of fused heterocyclyl include:

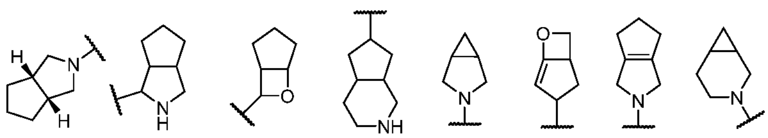

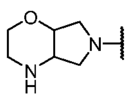 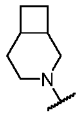 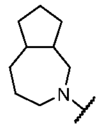 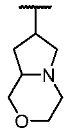 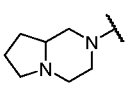 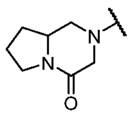 .

The term "bridged heterocyclyl" refers to a 5- to 14-membered polycyclic heterocyclyl group in which any two rings share two atoms that are not directly connected, and it may contain one or more double bonds, wherein one or more ring atoms are heteroatoms selected from the group consisting of nitrogen, oxygen, and sulfur, and the sulfur may optionally be substituted with oxo (i.e., to form sulfoxide or sulfone); the other ring atoms are carbon. It is preferably 6- to 14-membered, and more preferably 7-to 10-membered (e.g., 7-membered, 8-membered, 9-membered, or 10-membered). According to the number of constituent rings, the bridged heterocyclyl may be bicyclic, tricyclic, tetracyclic, etc., preferably bicyclic, tricyclic, or tetracyclic, and more preferably bicyclic or tricyclic. Non-limiting examples of bridged heterocyclyl include:

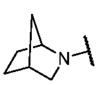 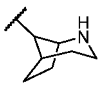 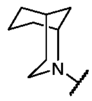 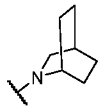 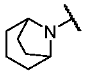 .

The heterocyclyl ring includes those in which the heterocyclyl described above (including monocyclic, spiro, fused, and bridged ones) fuses with an aryl, heteroaryl, or cycloalkyl ring, wherein the ring connected to the parent structure is heterocyclyl; its non-limiting examples include:

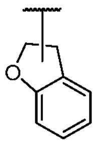 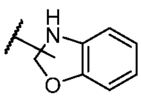 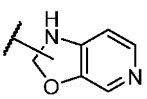 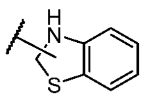 ,

and the like.

Heterocyclyl may be substituted or unsubstituted. When substituted, it may be substituted at any accessible connection site, and the substituent is preferably selected from one or more of halogen, alkyl, alkoxy, haloalkyl, haloalkoxy, cycloalkyloxy, heterocyclyloxy, hydroxy, hydroxyalkyl, cyano, amino, nitro, cycloalkyl, heterocyclyl, aryl, and heteroaryl.

The term "aryl" refers to a 6- to 14-membered, preferably 6- to 10-membered, all-carbon monocyclic or fused polycyclic (in which the rings share a pair of adjacent carbon atoms) group having a conjugated $\pi$-electron system, e.g., phenyl and naphthyl.

The aryl ring includes those in which the aryl ring described above fuses with a heteroaryl, heterocyclyl, or cycloalkyl ring, wherein the ring connected to the parent structure is the aryl ring; its non-limiting examples include:

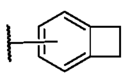 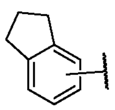 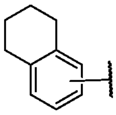 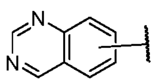 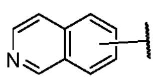 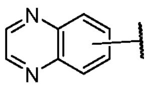 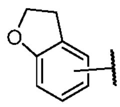

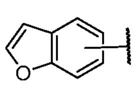 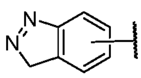 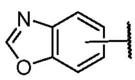 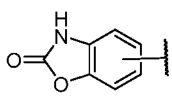 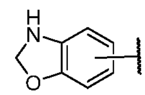 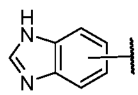

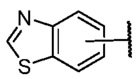 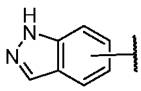 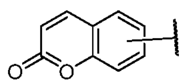 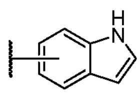

Aryl may be substituted or unsubstituted. When substituted, it may be substituted at any accessible connection site, and the substituent is preferably selected from one or more of halogen, alkyl, alkoxy, haloalkyl, haloalkoxy, cycloalkyloxy, heterocyclyloxy, hydroxy, hydroxyalkyl, cyano, amino, nitro, cycloalkyl, heterocyclyl, aryl, and heteroaryl.

The term "heteroaryl" refers to a heteroaromatic system containing 1 to 4 (e.g., 1, 2, 3, and 4) heteroatoms and 5 to 14 ring atoms, wherein the heteroatoms are selected from the group consisting of oxygen, sulfur, and nitrogen. The heteroaryl is preferably 5- to 10-membered (e.g., 5-membered, 6-membered, 7-membered, 8-membered, 9-membered, or 10-membered), and more preferably 5-membered or 6-membered, e.g., furyl, thienyl, pyridinyl, pyrrolyl, N-alkylpyrrolyl, pyrimidinyl, pyrazinyl, pyridazinyl, imidazolyl, pyrazolyl, triazolyl, and tetrazolyl. The heteroaryl ring includes those in which the heteroaryl ring described above fuses with an aryl, heterocyclyl, or cycloalkyl ring, wherein the ring connected to the parent structure is the heteroaryl ring; its non-limiting examples include:

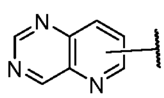 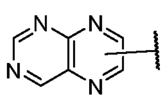 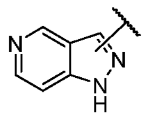 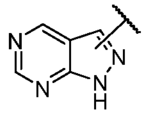 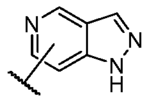 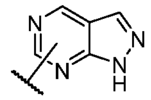

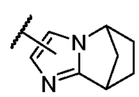 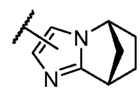 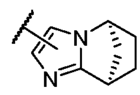 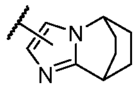 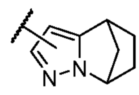 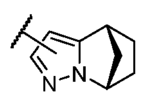

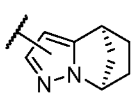 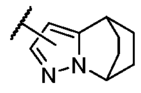 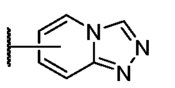 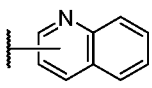 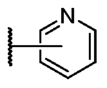 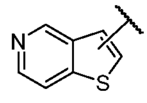

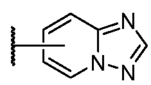 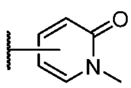 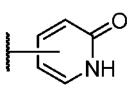 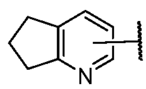 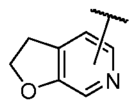 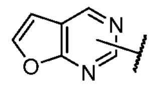

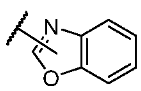 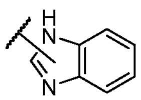 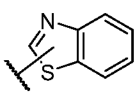 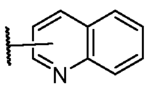 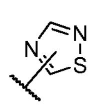 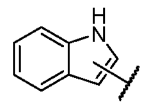

Heteroaryl may be substituted or unsubstituted. When substituted, it may be substituted at any accessible connection site, and the substituent is preferably selected from one or more of halogen, alkyl, alkoxy, haloalkyl, haloalkoxy, cycloalkyloxy, heterocyclyloxy, hydroxy, hydroxyalkyl, cyano, amino, nitro, cycloalkyl, heterocyclyl, aryl, and heteroaryl.

The cycloalkyl, heterocyclyl, aryl, and heteroaryl described above include residues derived by removal of one hydrogen atom from a ring atom of the parent structure, or residues derived by removal of two hydrogen atoms from the same ring atom or two different ring atoms of the parent structure, i.e., "cycloalkylene", "heterocyclylene", "arylene", and "heteroarylene".

The term "amino protecting group" refers to a group that is introduced onto an amino group in order for the amino group to remain unchanged when other parts of the molecule are involved in reactions, and the group can be easily removed. Non-limiting examples include: (trimethylsilyl)ethoxymethyl, tetrahydropyranyl, tert-butoxycarbonyl (Boc), acetyl, tosyl (Ts), benzyl, allyl, p-methoxybenzyl, and the like. These groups may be optionally substituted with 1 to 3 substituents selected from the group consisting of halogen, alkoxy, and nitro.

The term "hydroxy protecting group" refers to a group that is generally introduced onto a hydroxy group in order to block or protect the hydroxy group when other functional groups of the compound are involved in reactions, and the group can be easily removed. Non-limiting examples include: triethylsilyl, triisopropylsilyl, tert-butyldimethylsilyl (TBS), tert-butyldiphenylsilyl, $C_{1-6}$ alkoxy-substituted $C_{1-6}$ alkyl or phenyl-substituted $C_{1-6}$ alkyl (such as methoxymethyl (MOM) and ethoxyethyl), ($C_{1-10}$ alkyl or aryl)acyl (such as formyl, acetyl, benzoyl, and p-nitrobenzoyl), ($C_{1-6}$ alkyl or 6- to 10-membered aryl)sulfonyl, ($C_{1-6}$ alkoxy or 6- to 10-membered aryloxy)carbonyl, allyl, 2-tetrahydropyranyl (THP), and the like.

The term "cycloalkyloxy" refers to cycloalkyl-O-, wherein the cycloalkyl is as defined above.

The term "heterocyclyloxy" refers to heterocyclyl-O-, wherein the heterocyclyl is as defined above.

The term "aryloxy" refers to aryl-O-, wherein the aryl is as defined above.

The term "heteroaryloxy" refers to heteroaryl-O-, wherein the heteroaryl is as defined above.

The term "alkylthio" refers to alkyl-S-, wherein the alkyl is as defined above.

The term "haloalkyl" refers to alkyl substituted with one or more halogens, wherein the alkyl is as defined above.

The term "haloalkoxy" refers to alkoxy substituted with one or more halogens, wherein the alkoxy is as defined above.

The term "deuterated alkyl" refers to alkyl substituted with one or more deuterium atoms, wherein the alkyl is as defined above.

The term "hydroxyalkyl" refers to alkyl substituted with one or more hydroxy groups, wherein the alkyl is as defined above.

The term "halogen" refers to fluorine, chlorine, bromine, or iodine.

The term "hydroxy" refers to -OH.

The term "sulfhydryl" refers to -SH.

The term "amino" refers to $-NH_2$.

The term "cyano" refers to -CN.

The term "nitro" refers to $-NO_2$.

The term "oxo" refers to "=O".

The term "carbonyl" refers to C=O.

The term "carboxyl" refers to -C(O)OH.

The term "carboxylate group" refers to -C(O)O(alkyl), -C(O)O(cycloalkyl), (alkyl)C(O)O- or (cycloalkyl)C(O)O-, wherein the alkyl and cycloalkyl are as defined above.

The compounds of the present disclosure may exist in specific stereoisomeric forms. The term "stereoisomer" refers to isomers that are structurally identical but differ in the arrangement of the atoms in space. It includes cis and trans (or *Z* and *E*) isomers, (-)- and (+)-isomers, (*R*)- and (*S*)-enantiomers, diastereomers, (*D*)- and (*L*)-isomers, tautomers, atropisomers, conformers, and mixtures thereof (e.g., mixtures of racemates and diastereomers). Additional asymmetric atoms may be present in the substituents in the compounds of the present disclosure. All such stereoisomers and mixtures thereof are included within the scope of the present disclosure. Optically active (-)- and (+)-isomers, (*R*)- and (*S*)-enantiomers, and (*D*)- and (*L*)-isomers can be prepared by chiral synthesis, chiral reagents, or other conventional techniques. One isomer of a certain compound of the present disclosure may be prepared by asymmetric synthesis or with a chiral auxiliary, or, when the molecule contains a basic functional group (e.g., amino) or an acidic functional group (e.g., carboxyl), a diastereomeric salt is formed with an appropriate optically active acid or base, followed by diastereomeric resolution by conventional methods known in the art to give the pure isomer. Furthermore, separation of enantiomers and diastereomers is generally accomplished by chromatography.

In the chemical structure of the compound of the present disclosure, a bond "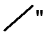" represents an unspecified configuration-that is, if chiral isomers exist in the chemical structure, the bond "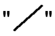" may be "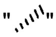" or "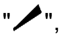", or contains both the configurations of "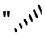" and "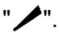".

The compounds of the present disclosure may also exist in different tautomeric forms, and all such forms are included within the scope of the present disclosure. The term "tautomer" or "tautomeric form" refers to a structural isomer that exists in equilibrium and is readily converted from one isomeric form into another. It includes all possible tautomers-that is, it is present in the form of a single isomer or in the form of a mixture of the tautomers in any ratio. Non-limiting examples include: keto-enol, imine-enamine, lactam-lactim, and the like. An example of lactam-lactim in equilibrium is shown below:

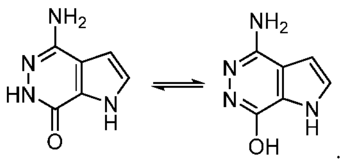

For example, reference to pyrazolyl is understood to include any one of the following two structures or a mixture of the two tautomers:

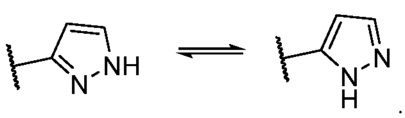

All tautomeric forms fall within the scope of the present disclosure, and the nomenclature of the compounds does not exclude any tautomers.

The compounds of the present disclosure include all suitable isotopic derivatives of the compounds thereof. The term "isotopic derivative" refers to a compound in which at least one atom is replaced with an atom having the same atomic number but a different atomic mass. Examples of isotopes that can be incorporated into the compounds of the present disclosure include stable and radioactive isotopes of hydrogen, carbon, nitrogen, oxygen, phosphorus, sulfur, fluorine, chlorine, bromine, iodine, etc., such as $^{2}H$ (deuterium, D), $^{3}H$ (deuterium, T), $^{11}C$, $^{13}C$, $^{14}C$, $^{15}N$, $^{17}O$, $^{18}O$, $^{32}p$, $^{33}p$, $^{33}S$, $^{34}S$, $^{35}S$, $^{36}S$, $^{18}F$, $^{36}Cl$, $^{82}Br$, $^{123}I$, $^{124}I$, $^{125}I$, $^{129}I$, and $^{131}I$, respectively; deuterium is preferred. Compared with non-deuterated drugs, deuterated drugs have the advantages of reduced toxic and side effects, increased drug stability, enhanced curative effect, prolonged biological half-lives, and the like. All isotopic variations of the compounds of the present disclosure, whether radioactive or not, are intended to be included within the scope of the present disclosure. Each available hydrogen atom linked to a carbon atom may be independently replaced with a deuterium atom, wherein replacement of deuterium may be partial or complete, and replacement of partial deuterium refers to replacement of at least one hydrogen with at least one deuterium.

In the compounds of the present disclosure, when a position is specifically assigned "deuterium" or "D", the position should be construed as the abundance of deuterium being at least 1000 times greater than the natural abundance of deuterium (which is 0.015%) (i.e., at least 15% deuterium incorporation). In some embodiments, the abundance of deuterium of each of the assigned deuterium atoms is at least 1000 times greater than the natural abundance of deuterium (i.e., at least 15% deuterium incorporation). In some embodiments, the abundance of deuterium of each of the assigned deuterium atoms is at least 2000 times greater than the natural abundance of deuterium (i.e., at least 30% deuterium incorporation). In some embodiments, the abundance of deuterium of each of the assigned deuterium atoms is at least 3000 times greater than the natural abundance of deuterium (i.e., at least 45% deuterium incorporation). In some embodiments, the abundance of deuterium of each of the assigned deuterium atoms is at least 3340 times greater than the natural abundance of deuterium (i.e., at least 50.1% deuterium incorporation). In some embodiments, the abundance of deuterium of each of the assigned deuterium atoms is at least 3500 times greater than the natural abundance of deuterium (i.e., at least 52.5% deuterium incorporation). In some embodiments, the abundance of deuterium of each of the assigned deuterium atoms is at least 4000 times greater than the natural abundance of deuterium (i.e., at least 60% deuterium incorporation). In some embodiments, the abundance of deuterium of each of the assigned deuterium atoms is at least 4500 times greater than the natural abundance of deuterium (i.e., at least 67.5% deuterium incorporation). In some embodiments, the abundance of deuterium of each of the assigned deuterium atoms is at least 5000 times greater than the natural abundance of deuterium (i.e., at least 75% deuterium incorporation). In some embodiments, the abundance of deuterium of each of the assigned deuterium atoms is at least 5500 times greater than the natural abundance of deuterium (i.e., at least 82.5% deuterium incorporation). In some embodiments, the abundance of deuterium of each of the assigned deuterium atoms is at least 6000 times greater than the natural abundance of deuterium (i.e., at least 90% deuterium incorporation). In some embodiments, the abundance of deuterium of each of the assigned deuterium atoms is at least 6333.3 times greater than the natural abundance of deuterium (i.e., at least 95% deuterium incorporation). In some embodiments, the abundance of deuterium of each of the assigned deuterium atoms is at least 6466.7 times greater than the natural abundance of deuterium (i.e., at least 97% deuterium incorporation). In some embodiments, the abundance of deuterium of each of the assigned deuterium atoms is at least 6600 times greater than the natural abundance of deuterium (i.e., at least 99% deuterium incorporation). In some embodiments, the abundance of deuterium of each of the assigned deuterium atoms is at least 6633.3 times greater than the natural abundance of deuterium (i.e., at least 99.5% deuterium incorporation).

"Optionally" or "optional" means that the event or circumstance subsequently described may, but does not necessarily, occur and that the description includes instances where the event or circumstance occurs or does not occur. For example, "$C_{1-6}$ alkyl optionally substituted with halogen or cyano" means that halogen or cyano may, but does not necessarily, exist, and the description includes the instance where alkyl is substituted with halogen or cyano and the instance where alkyl is not substituted with halogen and cyano.

"Substitution" or "substituted" means that one or more, preferably 1 to 6, and more preferably 1 to 3 hydrogen atoms in the group are independently substituted with a corresponding number of substituents. Those skilled in the art can determine (experimentally or theoretically) possible or impossible substitution without undue effort. For example, it may be unstable when amino or hydroxy having free hydrogen is bound to a carbon atom having an unsaturated (e.g., olefinic) bond.

"Pharmaceutical composition" refers to a mixture containing one or more of the compounds described herein or a pharmaceutically acceptable salt thereof, and other chemical components, and other components, for example, pharmaceutically acceptable carriers and excipients. The pharmaceutical composition is intended to promote the administration to an organism, so as to facilitate the absorption of the active ingredient, thereby exerting biological activity.

"Pharmaceutically acceptable salt" refers to a salt of the compound disclosed herein, which may be selected from the group consisting of inorganic and organic salts. The salts are safe and effective for use in the body of a mammal and possess the requisite biological activity. The salts may be prepared separately during the final separation and purification of the compound, or by reacting an appropriate group with an appropriate base or acid. Bases commonly

used to form pharmaceutically acceptable salts include inorganic bases such as sodium hydroxide and potassium hydroxide, and organic bases such as ammonia. Acids commonly used to form pharmaceutically acceptable salts include inorganic acids and organic acids.

For drugs or pharmacologically active agents, the term "therapeutically effective amount" refers to an amount of the drug or agent sufficient to achieve, or at least partially achieve, the desired effect. The determination of the therapeutically effective amount varies from person to person. It depends on the age and general condition of a subject, as well as the specific active substance used. The appropriate therapeutically effective amount in a case may be determined by those skilled in the art in the light of routine tests.

The term "pharmaceutically acceptable" used herein means that those compounds, materials, compositions, and/or dosage forms that are, within the scope of reasonable medical judgment, suitable for use in contact with the tissues of patients without excessive toxicity, irritation, allergic reaction, or other problems or complications, and are commensurate with a reasonable benefit/risk ratio and effective for the intended use. As used herein, the singular forms "a", "an" and "the" include plural references and vice versa, unless otherwise clearly defined in the context.

When the term "about" is applied to parameters such as pH, concentration, and temperature, it means that the parameter may vary by ±10%, and sometimes more preferably within ±5%. As will be appreciated by those skilled in the art, when the parameters are not critical, the numbers are generally given for illustrative purposes only and are not intended to be limiting.

Synthesis of the compounds of the present disclosure

To achieve the purpose of the present disclosure, the following technical schemes are adopted in the present disclosure:

Scheme 1

A method for preparing the compound of general formula (IV) or the pharmaceutically acceptable salt thereof disclosed herein is provided, and the method comprises the following step:

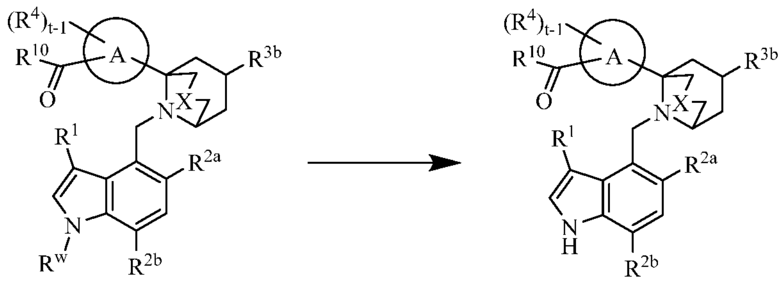

(IVA) (IV)

removing protecting group $R^w$ from a compound of general formula (IVA) in which $R^{10}$ is alkoxy (preferably $C_{1-6}$ alkoxy, and more preferably methoxy) or a pharmaceutically acceptable salt thereof under an alkaline condition and conducting a hydrolysis reaction to give a compound of general formula (IV) in which $R^{10}$ is hydroxy or a pharmaceutically acceptable salt thereof,

wherein:

$R^w$ is an amino protecting group; preferably, $R^w$ is tert-butoxycarbonyl or p-toluenesulfonyl; more preferably, $R^w$ is tert-butoxycarbonyl;
t is 1 or 2;
ring A, X, $R^1$, $R^{2a}$, $R^{2b}$, $R^{3b}$, and $R^4$ are as defined in general formula (IV).

Scheme 2

A method for preparing the compound of general formula (IV) or the pharmaceutically acceptable salt thereof disclosed herein is provided, and the method comprises the following steps:

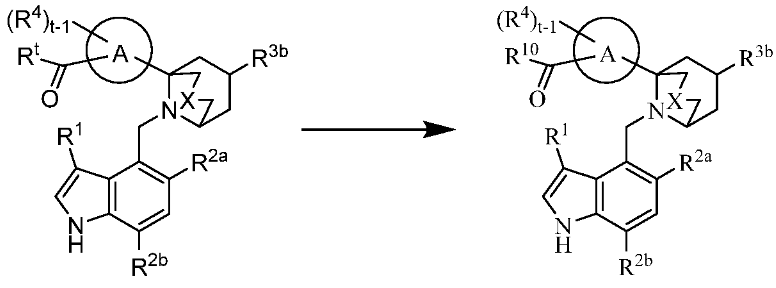

(IVB) (IV)

removing protecting group $R^s$ from a compound of general formula (IVB) or a pharmaceutically acceptable salt thereof in the presence of a catalyst (preferably tetrakis(triphenylphosphine)palladium(0)) to give the compound of general formula (IV) or the pharmaceutically acceptable salt thereof, and

optionally performing preparative chiral resolution to give the compound of general formula (IV) or the pharmaceutically acceptable salt thereof in a single configuration,

wherein:

$R^t$ is

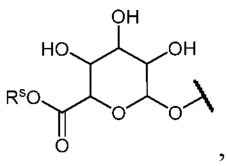 , 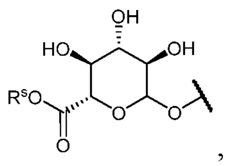 , 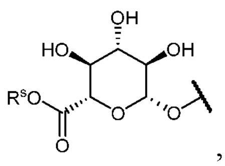 , and 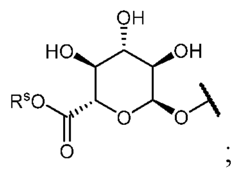 ;

$R^s$ is a hydroxy protecting group; preferably, $R^s$ is allyl;

$R^{10}$ is selected from the group consisting of

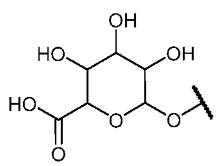 , 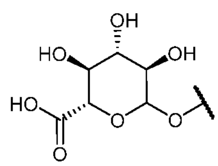 ,

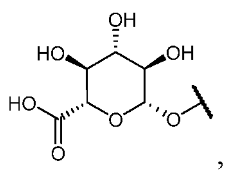 , and 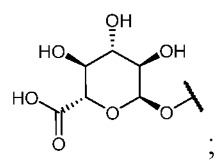 ;

t is 1 or 2;

ring A, X, $R^1$, $R^{2a}$, $R^{2b}$, $R^{3b}$, and $R^4$ are as defined in general formula (IV).

Scheme 3

A method for preparing the compound of general formula (V) or the pharmaceutically acceptable salt thereof disclosed herein is provided, and the method comprises the following step:

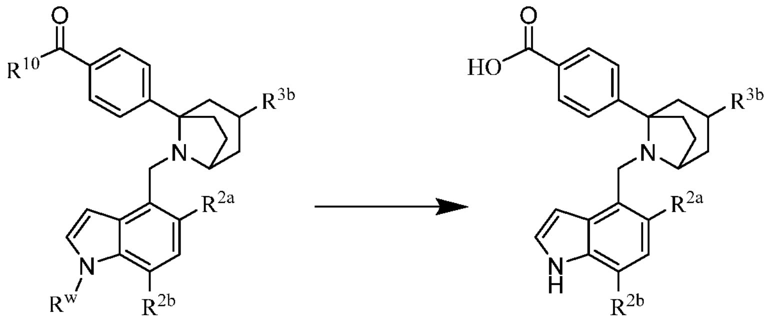

(VA) (V)

removing protecting group $R^w$ from a compound of general formula (VA) in which $R^{10}$ is alkoxy (preferably $C_{1-6}$ alkoxy, and more preferably methoxy) or a pharmaceutically acceptable salt thereof under an alkaline condition and conducting a hydrolysis reaction to give the compound of general formula (V) or the pharmaceutically acceptable salt thereof,

wherein:

$R^w$ is an amino protecting group; preferably, $R^w$ is tert-butoxycarbonyl or p-toluenesulfonyl; more preferably, $R^w$ is tert-butoxycarbonyl;

$R^{2a}$, $R^{2b}$, and $R^{3b}$ are as defined in general formula (V).

Scheme 4

A method for preparing the compound of general formula (V-1) or the pharmaceutically acceptable salt thereof disclosed herein is provided, and the method comprises the following step:

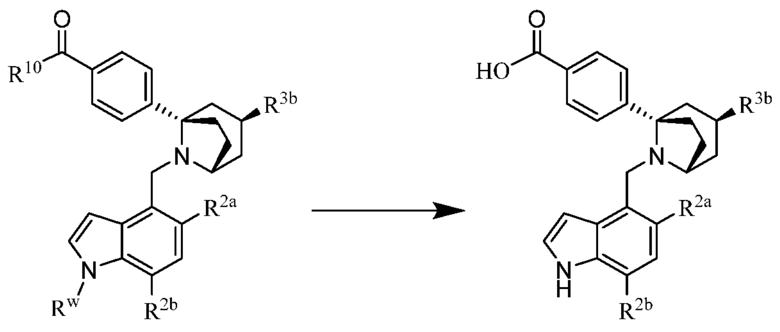

(V-1A) (V-1)

removing protecting group $R^w$ from a compound of general formula (V-1A) in which $R^{10}$ is alkoxy (preferably $C_{1-6}$ alkoxy, and more preferably methoxy) or a pharmaceutically acceptable salt thereof under an alkaline condition and conducting a hydrolysis reaction to give the compound of general formula (V-1) or the pharmaceutically acceptable salt thereof,

wherein:

$R^w$ is an amino protecting group; preferably, $R^w$ is tert-butoxycarbonyl or p-toluenesulfonyl; more preferably,

$R^w$ is tert-butoxycarbonyl;
$R^{2a}$, $R^{2b}$, and $R^{3b}$ are as defined in general formula (V-1).

Scheme 5

A method for preparing the compound of general formula (V-2) or the pharmaceutically acceptable salt thereof disclosed herein is provided, and the method comprises the following step:

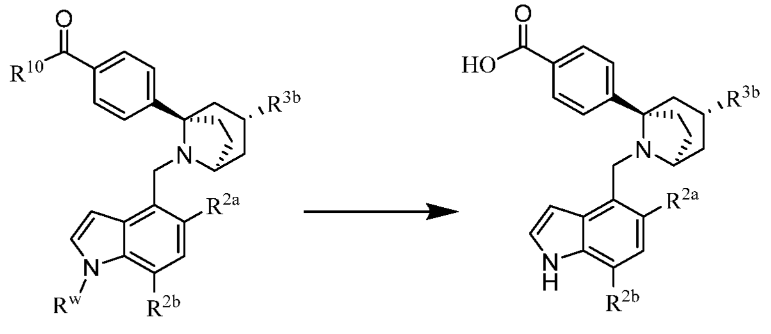

(V-2A) (V-2)

removing protecting group $R^w$ from a compound of general formula (V-2A) in which $R^{10}$ is alkoxy (preferably $C_{1-6}$ alkoxy, and more preferably methoxy) or a pharmaceutically acceptable salt thereof under an alkaline condition and conducting a hydrolysis reaction to give the compound of general formula (V-2) or the pharmaceutically acceptable salt thereof,

wherein:

$R^w$ is an amino protecting group; preferably, $R^w$ is tert-butoxycarbonyl or p-toluenesulfonyl; more preferably, $R^w$ is tert-butoxycarbonyl;
$R^{2a}$, $R^{2b}$, and $R^{3b}$ are as defined in general formula (V-2).

Scheme 6

A method for preparing the compounds of general formula (V-1) and general formula (V-2) or the pharmaceutically acceptable salts thereof disclosed herein is provided, and the method comprises the following step:

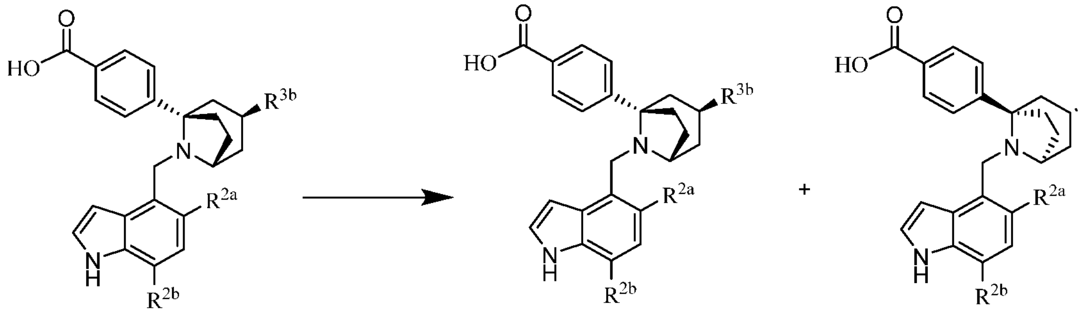

(±)-*rel*-(1*S*,3*S*,5*R*) (V'') (V-1) (V-2)

performing preparative chiral resolution of a compound of general formula (V'') or a pharmaceutically acceptable salt thereof to give the compounds of general formula (V-1) and general formula (V-2) or the pharmaceutically acceptable salts thereof;
$R^{2a}$, $R^{2b}$, and $R^{3b}$ are as defined in general formula (V-1) and general formula (V-2).

Scheme 7

A method for preparing the compound of general formula (V') or the pharmaceutically acceptable salt thereof disclosed herein is provided, and the method comprises the following steps:

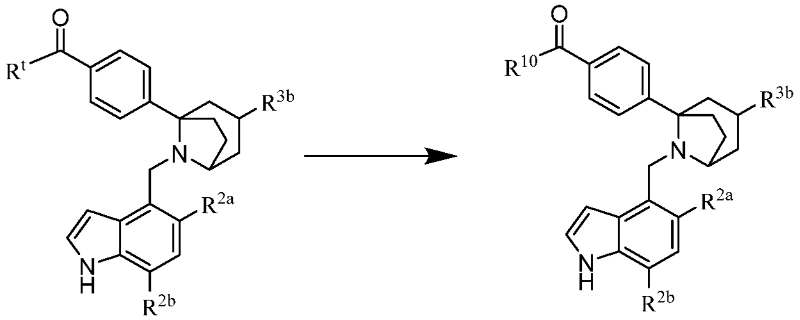

(V'A) (V')

removing protecting group $R^s$ from a compound of general formula (V'A) or a pharmaceutically acceptable salt thereof in the presence of a catalyst (preferably tetrakis(triphenylphosphine)palladium(0)) to give the compound of general formula (V') or the pharmaceutically acceptable salt thereof, and

optionally performing preparative chiral resolution to give the compound of general formula (V') or the pharmaceutically acceptable salt thereof in a single configuration,

wherein:

$R^t$ is

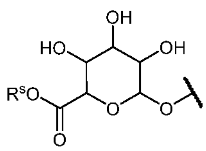 , 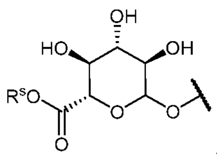 , 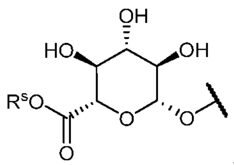 , and 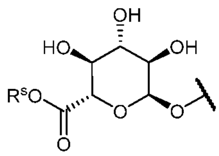 ;

$R^s$ is a hydroxy protecting group; preferably, $R^s$ is allyl;

$R^{10}$ is selected from the group consisting of

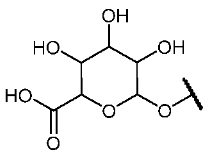 , 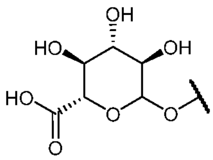 ,

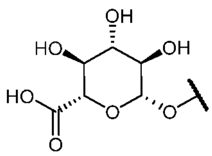 , and 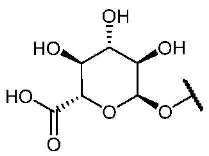 ;

$R^{2a}$, $R^{2b}$, and $R^{3b}$ are as defined in general formula (V').

Scheme 8

A method for preparing the compound of general formula (V'-1) or the pharmaceutically acceptable salt thereof disclosed herein is provided, and the method comprises the following steps:

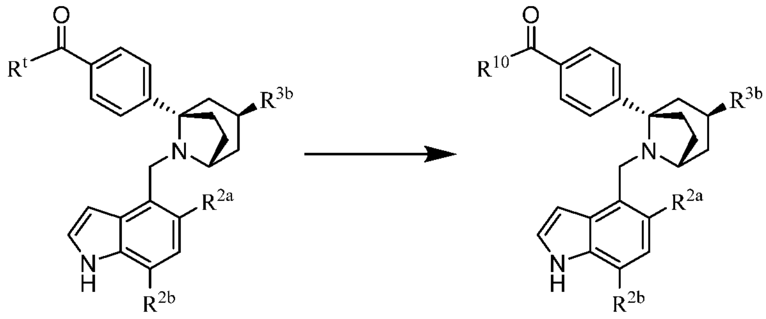

(V'-1A) (V'-1)

removing protecting group $R^s$ from a compound of general formula (V'-1A) or a pharmaceutically acceptable salt thereof in the presence of a catalyst (preferably tetrakis(triphenylphosphine)palladium(0)) to give the compound of general formula (V'-1) or the pharmaceutically acceptable salt thereof, and
optionally performing preparative chiral resolution to give the compound of general formula (V'-1) or the pharmaceutically acceptable salt thereof in a single configuration,

wherein:

$R^t$ is

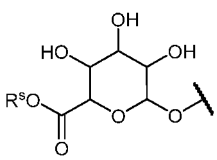 , 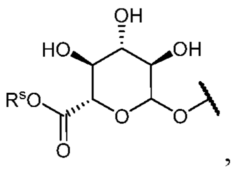 , 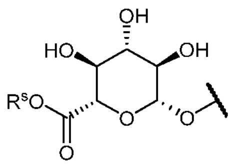 , and 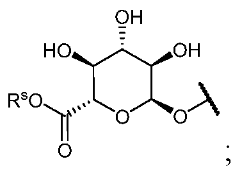 ;

$R^s$ is a hydroxy protecting group; preferably, $R^s$ is allyl;
$R^{10}$ is selected from the group consisting of

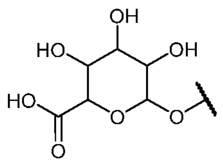 , 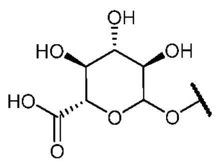 ,

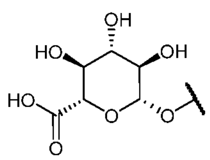 , and 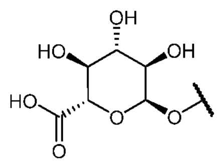 ;

$R^{2a}$, $R^{2b}$, and $R^{3b}$ are as defined in general formula (V'-1).

Scheme 9

A method for preparing the compound of general formula (V'-2) or the pharmaceutically acceptable salt thereof disclosed herein is provided, and the method comprises the following steps:

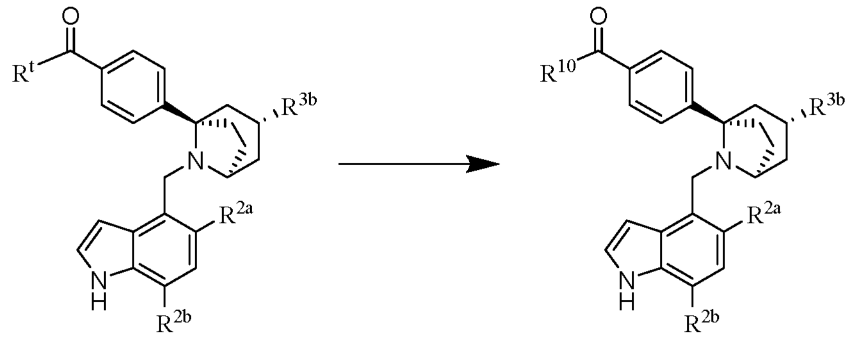

(V'-2A) (V'-2)

removing protecting group $R^s$ from a compound of general formula (V’-2A) or a pharmaceutically acceptable salt thereof in the presence of a catalyst (preferably tetrakis(triphenylphosphine)palladium(0)) to give the compound of general formula (V’-2) or the pharmaceutically acceptable salt thereof, and

optionally performing preparative chiral resolution to give the compound of general formula (V’-2) or the pharmaceutically acceptable salt thereof in a single configuration,

wherein:

$R^t$ is

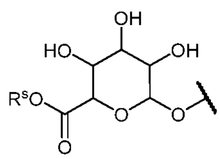 , 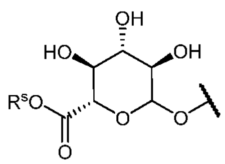 , 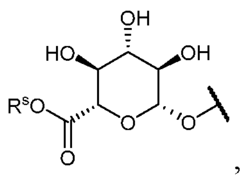 , and 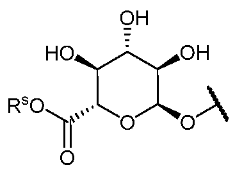 ;

$R^s$ is a hydroxy protecting group; preferably, $R^s$ is allyl;

$R^{10}$ is selected from the group consisting of

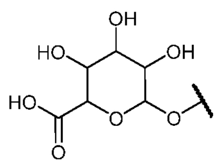 , 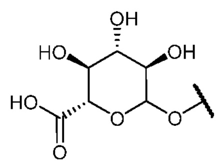 ,

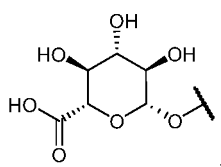 , and 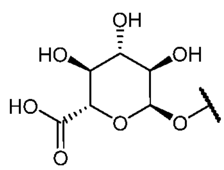 ;

$R^{2a}$, $R^{2b}$, and $R^{3b}$ are as defined in general formula (V’-2).

In the above reactions, the bases include organic bases and inorganic bases; the organic bases include, but

are not limited to, triethylamine, N,N-diisopropylethylamine, n-butyllithium, lithium diisopropylamide, sodium acetate, potassium acetate, sodium ethoxide, sodium tert-butoxide, and potassium tert-butoxide; the inorganic bases include, but are not limited to, sodium hydride, potassium phosphate, sodium carbonate, potassium carbonate, cesium carbonate, sodium hydroxide, lithium hydroxide monohydrate, lithium hydroxide, and potassium hydroxide, and are preferably selected from the group consisting of lithium hydroxide monohydrate, lithium hydroxide, and potassium hydroxide.

The reactions of the above steps are preferably conducted in solvents, including but not limited to: ethylene glycol dimethyl ether, acetic acid, methanol, ethanol, acetonitrile, n-butanol, toluene, tetrahydrofuran, dichloromethane, petroleum ether, ethyl acetate, n-hexane, dimethyl sulfoxide, 1,4-dioxane, water, N,N-dimethylformamide, N,N-dimethylacetamide, 1,2-dibromoethane, and mixtures thereof.

## BRIEF DESCRIPTION OF THE DRAWINGS

FIG. 1: the effects of the compound of Example 1-1 and the positive drug LNP023 on serum iC3b+C3d in passive heymann nephritis rats.
FIG. 2 and FIG. 3: the effects of the compound of Example 1-1 and the positive drug LNP023 on protein/creatinine in urine in passive heymann nephritis rats.

## DETAILED DESCRIPTION

The present disclosure is further described below with reference to examples, but these examples are not intended to limit the scope of the present disclosure.

### Examples

The structures of the compounds were determined by nuclear magnetic resonance (NMR) spectroscopy and/or mass spectrometry (MS). NMR shifts (δ) were given in $10^{-6}$ (ppm). NMR analysis was performed on a Bruker AVANCE NEO 500M, with deuterated dimethyl sulfoxide (DMSO-$d_6$), deuterated chloroform ($CDCl_3$), and deuterated methanol ($CD_3OD$) as determination solvents and tetramethylsilane (TMS) as an internal standard.

MS analysis was performed on an Agilent 1200/1290 DAD-6110/6120 Quadrupole MS liquid chromatography-mass spectrometry system (manufacturer: Agilent; MS model: 6110/6120 Quadrupole MS), Waters ACQuity UPLC-QD/SQD (manufacturer: Waters, MS model: Waters ACQuity Qda Detector/Waters SQ Detector) and THERMO Ultimate 3000-Q Exactive (manufacturer: THERMO, MS model: THERMO Q Exactive).

High performance liquid chromatography (HPLC) analysis was performed on Agilent HPLC 1200DAD, Agilent HPLC 1200VWD, and Waters HPLC e2695-2489 high performance liquid chromatographs.

Chiral HPLC analysis was performed on an Agilent 1260 DAD high performance liquid chromatograph.

Preparative high-performance liquid chromatography was performed on Waters 2545-2767, Waters 2767-SQ Detecor2, Shimadzu LC-20AP, and Gilson GX-281 preparative chromatographs.

Preparative chiral chromatography was performed on a Shimadzu LC-20AP preparative chromatograph.

The CombiFlash preparative flash chromatograph used was CombiFlash Rf200 (TELEDYNE ISCO).

Yantai Huanghai HSGF254 or Qingdao GF254 silica gel plates, 0.15-0.2 mm layer thickness, were adopted for thin-layer chromatography (TLC) analysis and 0.4-0.5 mm layer thickness for TLC separation and purification.

Silica gel column chromatography generally used 200- to 300-mesh silica gel (Huanghai, Yantai) as the carrier.

The mean inhibition of kinase and the $IC_{50}$ value were measured on a NovoStar microplate reader (BMG, Germany).

Known starting materials in the present disclosure may be synthesized using or according to methods known in the art, or may be purchased from ABCR GmbH & Co. KG, Acros Organics, Aldrich Chemical Company, Accela ChemBio Inc., Chembee Chemicals, and other companies.

In the examples, the reactions could all be performed in an argon atmosphere or a nitrogen atmosphere unless otherwise specified.

The argon atmosphere or nitrogen atmosphere means that the reaction flask is connected to a balloon containing about 1 L of argon or nitrogen.

The hydrogen atmosphere means that the reaction flask is connected to a balloon containing about 1 L of hydrogen.

Pressurized hydrogenation reactions were performed using a Parr 3916EKX hydrogenator and a Qinglan QL-500 hydrogenator, or a HC2-SS hydrogenator. Hydrogenation reactions generally involve 3 cycles of vacuumization and hydrogen purging.

Microwave reactions were performed on a CEM Discover-S 908860 microwave reactor. In the examples, a solution refers to an aqueous solution unless otherwise specified.

In the examples, the reaction temperature is room temperature, i.e., 20 °C to 30 °C, unless otherwise specified.

The monitoring of the reaction progress in the examples was conducted by thin-layer chromatography (TLC). The developing solvent for reactions, the eluent system for column chromatography purification, and the developing solvent system for thin-layer chromatography included: A: dichloromethane/methanol system, B: n-hexane/ethyl acetate system, C: n-hexane/dichloromethane system, D: petroleum ether/ethyl acetate system, and E: toluene/acetone system. The volume ratio of the solvents was adjusted according to the polarity of the compound, or by adding a small amount of basic or acidic reagents such as triethylamine and acetic acid.

When the compounds of the examples contained two or more chiral centers, the relative stereochemistry of these compounds was identified by NMR studies and/or X-ray diffraction. In these cases, the compounds were identified using the prefix "rel" followed by the R/S nomenclature, where R/S provides only relative stereochemical information and does not indicate absolute stereochemistry. For example, compound 1 of the present disclosure (chemical name: (±)-*rel*-4-((1*S*,3*S*,5*R*)-3-ethoxy-8-((5-methoxy-7-methyl-1*H*-indol-4-yl)methyl)-8-azabi

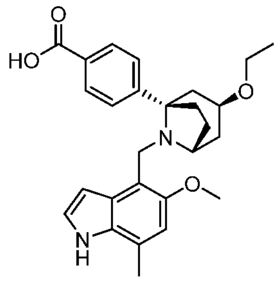

cyclo[3.2.1]octan-1-yl)benzoic acid; structural formula: (±)-*rel*-(1*S*,3*S*,5*R*)1 ) represents that the relative stereochemistry of

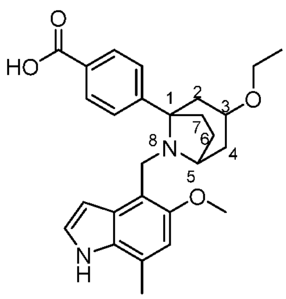

at locants 1, 3, and 5 is *1S,3S,5R* or *1R,3R,5S* (i.e., cis; specifically, the five-membered nitrogen-containing heterocyclyl where the locants 1 and 5-8 are present and the ethoxy at locant 3 are both on the same side of the plane of the six-membered nitrogen-containing heterocyclic ring where the locants 1-5 and 8 are present: both point inward or outward). Since compound 1 is a racemate, those skilled in the art can unambiguously identify compound 1 as a 1:1 mixture of the two enantiomers

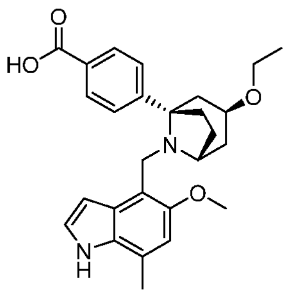

**1-1**

and

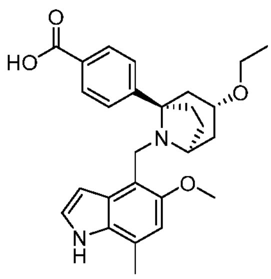

**1-2**

.

Furthermore, those skilled in the art can unambiguously determine that once the relative stereochemistry of

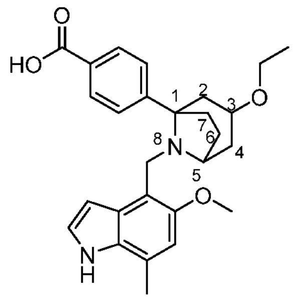

at locants 1 and 3 is determined, the relative stereochemistry at locant 5 is uniquely determined, as the 4-carboxylphenyl at locant 1 can only be present on a different side of the plane of the six-membered nitrogen-containing heterocyclic ring where the locants 1-5 and 8 are present, from the five-membered nitrogen-containing heterocyclyl where the locants 1 and 5-8 are

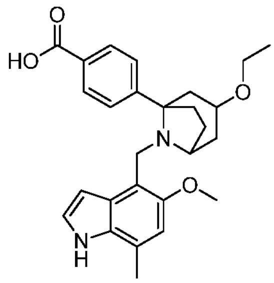

*rel*-(1S,3S)

1

present, due to steric hindrance. Therefore, and

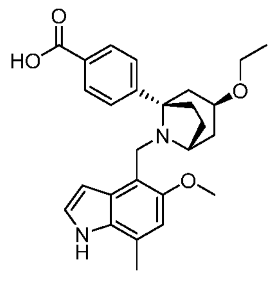

(±)-*rel*-(1*S*,3*S*,5*R*) **1**

are actually the same compound, i.e., a racemate, and

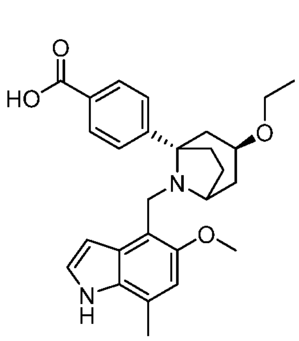

and

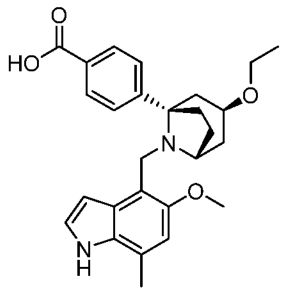

1-1

are actually the same compound.

Example 1, Example 2

(±)-*rel*-4-((1*S*,3*S*,5*R*)-3-Ethoxy-8-((5-methoxy-7-methyl-1*H*-indol-4-yl)methyl)-8-azabi cyclo[3.2.1]octan-1-yl)benzoic acid 1

(±)-*rel*-4-((1*R*,3*S*,5*S*)-3-Ethoxy-8-((5-methoxy-7-methyl-1*H*-indol-4-yl)methyl)-8-azabi cyclo[3.2.1]octan-1-yl)benzoic acid 2

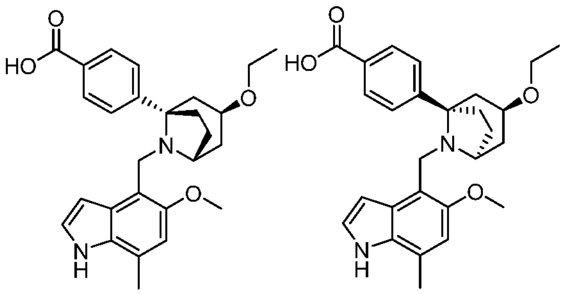

(±)-*rel*-(1*S*,3*S*,5*R*) **1** (±)-*rel*-(1*R*,3*S*,5*S*) **2**

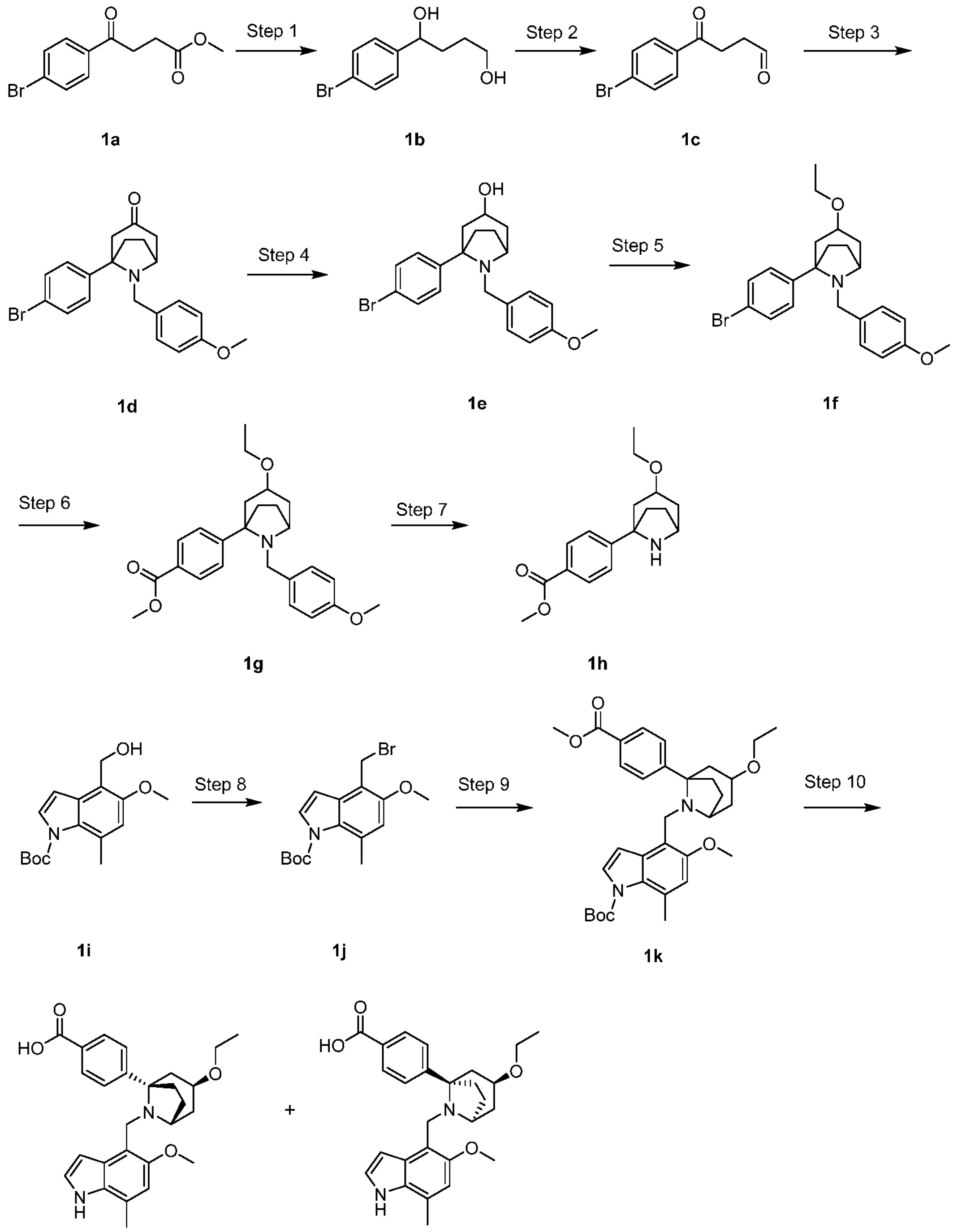


1a 1b 1c

1d 1e 1f

1g 1h

1i 1j 1k

(±)-*rel*-(1*S*,3*S*,5*R*) **1** + (±)-*rel*-(1*R*,3*S*,5*S*) **2**

Step 1

1-(4-Bromophenyl)butane-1,4-diol **1b**

Methyl 4-(4-bromophenyl)-4-oxobutanoate **1a** (5 g, 17.54 mmol, Bide Pharmatech Ltd.) was dissolved in tetrahydrofuran (50 mL), and a solution of lithium borohydride in tetrahydrofuran (17 mL, 2 mmol/mL) was added at 0 °C. The mixture was naturally warmed to room temperature and stirred overnight. The reaction mixture was quenched with saturated sodium thiosulfate solution and extracted with ethyl acetate. The organic phase was dried and concentrated to give the crude title product **1b** (4.29 g). The product was directly used in the next step without purification.
MS m/z (ESI): 242.9 [M-H].

Step 2

4-(4-Bromophenyl)-4-oxobutanal **1c**

Dimethyl sulfoxide (8.2 g, 104.95 mmol) was dissolved in dichloromethane (50 mL), and oxalyl chloride (8.8 g, 69.33 mmol) was added at -78 °C. The mixture was stirred for another 10 min, and compound **1b** (4.29 g, 17.50 mmol) was added. After 10 min, triethylamine (17.7 g, 174.92 mmol) was added. The reaction mixture was stirred for another hour, naturally warmed to room temperature, and diluted with dichloromethane. The organic phase was washed with saturated aqueous sodium bicarbonate solution, dried, concentrated under reduced pressure, and purified by silica gel column chromatography with eluent C to give the title compound **1c** (2.7g, yield: 64%).
MS m/z (ESI): 240.8 [M+1].

Step 3

1-(4-Bromophenyl)-8-[(4-methoxybenzyl)-8-azabicyclo[3.2.1]octan-3-one **1d**

4-Methoxybenzylamine (1.61 g, 11.74 mmol, Accela ChemBio Inc.) and sodium acetate (6.43 g, 78.38 mmol) were dissolved in water (7.5 mL), and 2 M hydrochloric acid (16 mL) and 1,3-acetonedicarboxylic acid (1.96 g, 13.42 mmol) were added at 0 °C. The mixture was stirred for another 30 min, and compound 1c (2.7 g, 11.20 mmol) was added. After 30 min, the mixture was stirred at 40 °C for 3 h. The reaction mixture was adjusted to pH 8-9 with saturated sodium bicarbonate solution and extracted with ethyl acetate. The organic phase was dried, concentrated under reduced pressure, and purified by silica gel column chromatography with eluent C to give the title compound **1d** (580 mg, yield: 12.9%).
MS m/z (ESI): 399.9 [M+1].

Step 4

1-(4-Bromophenyl)-8-(4-methoxybenzyl)-8-azabicyclo[3.2.1]octan-3-ol **1e**

Compound **1d** (530 mg, 1.32 mmol) was dissolved in methanol (5 mL), and sodium borohydride (200 mg, 5.29 mmol) was added. The mixture was stirred at room temperature for 2 h. The reaction mixture was quenched with saturated aqueous ammonium chloride solution and extracted with ethyl acetate. The organic phase was dried and concentrated under reduced pressure to give the crude title compound **1e** (420 mg, yield: 78.8%).
MS m/z (ESI): 401.8 [M+1].

Step 5

1-(4-Bromophenyl)-3-ethoxy-8-(4-methoxybenzyl)-8-azabicyclo[3.2.1]octane **1f**

Compound **1e** was dissolved in dimethylformamide (5 mL), and sodium hydride (83 mg, 2.08 mmol) was added at 0 °C. The reaction mixture was stirred for another hour, and iodoethane (325 mg, 2.09 mmol) was added. The reaction mixture was warmed to room temperature and stirred overnight. The reaction mixture was quenched with saturated aqueous ammonium chloride solution and extracted with ethyl acetate. The organic phase was dried, concentrated under reduced pressure, and purified by silica gel column chromatography with eluent C to give the title compound **1f** (350 mg, yield: 77.9%).
MS m/z (ESI): 429.9 [M+1].

Step 6

Methyl 4-(3-ethoxy-8-(4-methoxybenzyl)-8-azabicyclo[3.2.1]octan-1-yl)benzoate **1g**

Compound **1f** was dissolved in methanol (4 mL) and dimethylformamide (4 mL), and palladium acetate (54 mg, 240.52 μmol), diphenyl phosphoryl azide (100 mg, 242.46 μmol), and triethylamine (822 mg, 8.12 mmol) were added. The system was purged three times with carbon monoxide gas, and the mixture was stirred at 80 °C overnight. The reaction mixture was poured into water and extracted with ethyl acetate. The organic phase was dried, concentrated under reduced pressure, and purified by silica gel column chromatography with eluent C to give the title compound **1g** (225 mg, yield: 67.5%).
MS m/z (ESI): 411.0 [M+1].

Step 7

Methyl 4-(3-ethoxy-8-azabicyclo[3.2.1]octan-1-yl)benzoate **1h**

Compound **1g** (225 mg, 549.43 μmol) was dissolved in ethanol (5 mL), and the hydrogenation catalyst palladium on carbon (40 mg, 375.87 μmol) was added. The system was purged three times with hydrogen, and the mixture was stirred at room temperature for 48 h in a hydrogen atmosphere. The reaction mixture was filtered. The organic phase was concentrated under reduced pressure to give the crude title compound **1h** (130 mg). The product was directly used in the next step without purification.

MS m/z (ESI): 290.0 [M+1].

Step 8

tert-Butyl 4-(bromomethyl)-5-methoxy-7-methylindole-1-carboxylate **1j**

The compound tert-butyl 4-(hydroxymethyl)-5-methoxy-7-methyl-1*H*-indole-1-carboxylate **1i** (150 mg, 514.86 μmol, synthesized by reference to the method of preparing intermediate 1-10 in WO2015009616A1) was dissolved in dichloromethane (2 mL), and carbon tetrabromide (170 mg, 512.62 μmol) and triphenylphosphine (135 mg, 514.71 μmol) were added under nitrogen atmosphere. The reaction mixture was stirred at room temperature for 2 h and directly concentrated to give the crude compound **1j** (183 mg). The product was directly used in the next step without purification.

Step 9

tert-Butyl 4-((3-ethoxy-1-(4-(methoxycarbonyl)phenyl)-8-azabicyclo[3.2.1]octan-8-yl)methyl)-5-methoxy-7-methyl-1*H*-indole-1-carboxylate **1k**

Compound **1h** (100 mg, 345.5801 μmol) was dissolved in dimethylformamide (2 mL), and sodium hydride (27 mg, 675.07 μmol) was added at 0 °C. The reaction mixture was stirred for another hour, and then a solution of compound **1j** (183 mg, 516.60 μmol) in dimethylformamide was added. The reaction mixture was stirred for another hour and quenched with saturated aqueous ammonium chloride solution. The organic phase was dried, concentrated under reduced pressure, and purified by silica gel column chromatography with eluent C to give the title compound **1k** (130 mg, yield: 66.8%). MS m/z (ESI): 563.0 [M+1].

Step 10

(±)-*rel*-4-((1*S*,3*S*,5*R*)-3-Ethoxy-8-((5-methoxy-7-methyl-1*H*-indol-4-yl)methyl)-8-azabi cyclo[3.2.1]octan-1-yl)benzoic acid **1**

(±)-*rel*-4-((1*R*,3*S*,5*S*)-3-Ethoxy-8-((5-methoxy-7-methyl-1*H*-indol-4-yl)methyl)-8-azabi cyclo[3.2.1]octan-1-yl)benzoic acid **2**

Compound **1k** (130 mg, 231.03 μmol) was dissolved in 6 mL of a mixed solution of tetrahydrofuran, methanol, and water (V:V:V = 1:1:1). Lithium hydroxide monohydrate (58 mg, 1.38 mmol) was added. The reaction mixture was stirred at 70 °C for 3 h. The reaction mixture was concentrated, diluted with a small amount of methanol, and purified by preparative high performance liquid chromatography (Waters 2545, column: Sharpsil-T C18, 250 × 50 mm, 8 μm; mobile phase A: water (containing 10 mmol/L ammonium bicarbonate); mobile phase B: acetonitrile; 18 min gradient: 20%-38%, flow rate: 80 mL/min) to give the title compounds 1 (4 mg, yield: 3.86%) and **2** (5 mg, yield: 4.82%).

Compound **1:**

Preparative high performance liquid chromatography: retention time 17.28 min.

MS m/z (ESI): 449.1 [M+1].

$^1$H NMR (500 MHz, $CD_3OD$): δ 8.16-8.14 (m, 2H), 7.69 (br, 2H), 7.35-7.34 (m, 1H), 6.84 (s, 1H), 6.34 (br, 1H), 4.20-4.03 (m, 3H), 3.93 (s, 3H), 3.71-3.58 (m, 1H), 3.51-3.34 (m, 2H), 3.32-2.96 (m, 2H), 2.73-2.68 (m,3H), 2.54 (s, 3H), 2.25-2.04 (m, 3H), 1.25-1.22 m, 3H).

Compound **2:**

Preparative high performance liquid chromatography: retention time 14.83 min.

MS m/z (ESI): 449.1 [M+1].

$^1$H NMR (500 MHz, $CD_3OD$): δ 8.16-8.14 (m, 2H), 7.70(br, 2H), 7.39-7.26 (m, 1H), 6.83 (s, 1H), 6.31 (br, 1H), 4.19-3.92 (m, 3H), 3.87-3.79 (m, 4H), 3.66-3.45 (m, 2H), 3.07-2.58 (m, 4H), 2.50 (s, 3H), 2.38-2.16 (m, 4H), 1.24-1.06 (m, 3H).

Example **1-1,** Example **1-2**

4-((1*S*,3*S*,5*R*)-3-Ethoxy-8-((5-methoxy-7-methyl-1*H*-indol-4-yl)methyl)-8-azabicyclo[3 .2.1]octan-1-yl)benzoic acid **1-1**

4-((1*R*,3*R*,5*S*)-3-Ethoxy-8-((5-methoxy-7-methyl-1*H*-indol-4-yl)methyl)-8-azabicyclo[3 .2.1]octan-1-yl)benzoic acid **1-2**

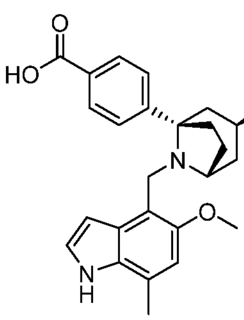

**1-1**

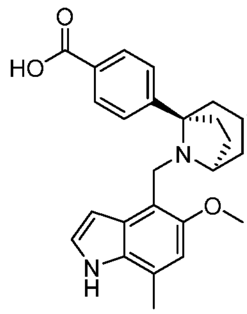

**1-2**

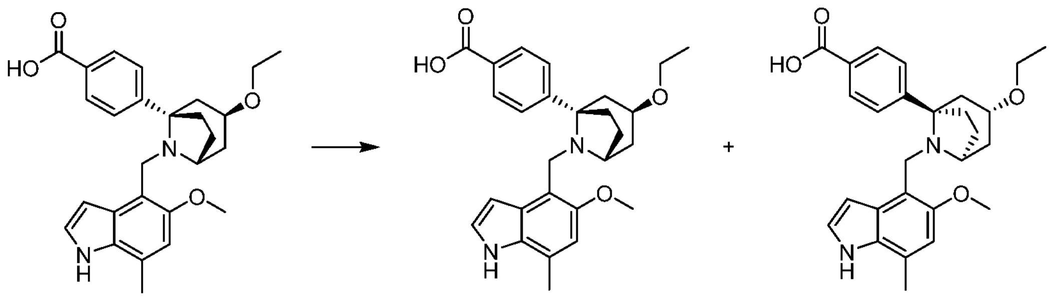

(±)-*rel*-(1*S*,3*S*,5*R*) **1** **1-1** **1-2**

Compound **1** (100 mg, 222.93 μmol) was purified by preparative chiral chromatography (resolution condition: preparative chiral column CHIRALPAK IG, 5 μm, 20 mm × 250 mm (Phenomenex); mobile phase 1: n-hexane (80%); mobile phase 2: containing 0.1% diethylamine, 0.1% trifluoroacetic acid, ethanol (20%), flow rate: 20 mL/min), and the corresponding fractions were collected and concentrated under reduced pressure to give the title compounds **1-1** (35 mg, yield: 35%) and **1-2** (33 mg, yield: 33%).

Compound **1-1:**

MS m/z (ESI): 449.1 [M+1].

Chiral HPLC analysis: retention time 7.946 min, chiral purity: 100% (column: CHIRALPAK IG, 5 μm, 20 mm × 250 mm (Phenomenex); mobile phase 1: n-hexane (80%); mobile phase 2: containing 0.1% diethylamine, 0.1% trifluoroacetic acid, ethanol (20%), flow rate: 1 mL/min).

$^1$H NMR (500 MHz, MeOD) δ 8.16-8.15 (m, 2H), 7.69 (br, 2H), 7.34 (br, 1H), 6.83 (s, 1H), 6.33 (br, 1H), 4.22-4.12 (m, 2H), 4.03-4.00 (m, 1H), 3.93 (s, 3H), 3.71-3.51 (m, 1H), 3.50-3.35 (m, 2H), 3.32-2.96 (m, 2H), 2.73-2.53 (m, 3H), 2.51 (s, 3H), 2.21-2.05 (m, 3H), 1.35-1.22 (m, 3H).

Compound **1-2:**

MS m/z (ESI): 449.1 [M+1].

Chiral HPLC analysis: retention time 12.77 min, chiral purity: 98.7% (column: CHIRALPAKIG, 5 μm, 20 mm × 250 mm (Phenomenex); mobile phase 1: n-hexane (80%); mobile phase 2: containing 0.1% diethylamine, 0.1% trifluoroacetic acid, ethanol (20%), flow rate: 1 mL/min).

$^1$H NMR (500 MHz, MeOD) δ 8.17-8.16 (m, 2H), 7.71 (br, 2H), 7.35 (br, 1H), 6.85 (s, 1H), 6.34 (br, 1H), 4.23-4.19 (m, 2H), 4.08-4.05 (m, 1H), 3.95 (s, 3H), 3.72-3.59 (m, 1H), 3.51-3.34 (m, 2H), 3.32-2.98 (m, 2H), 2.75-2.60 (m, 3H), 2.54 (s, 3H), 2.27-2.20 (m, 3H), 1.25-1.23 (m, 3H).

Example **3**

4-((1*S*,3*S*,5*R*)-8-((5,7-Dimethyl-1*H*-indol-4-yl)methyl)-3-ethoxy-8-azabicyclo[3.2.1]oct an-1-yl)benzoic acid **3**

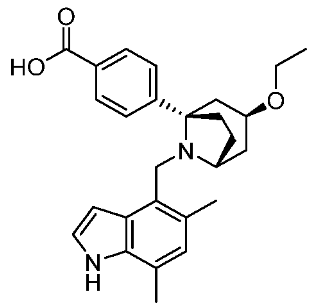

**3**

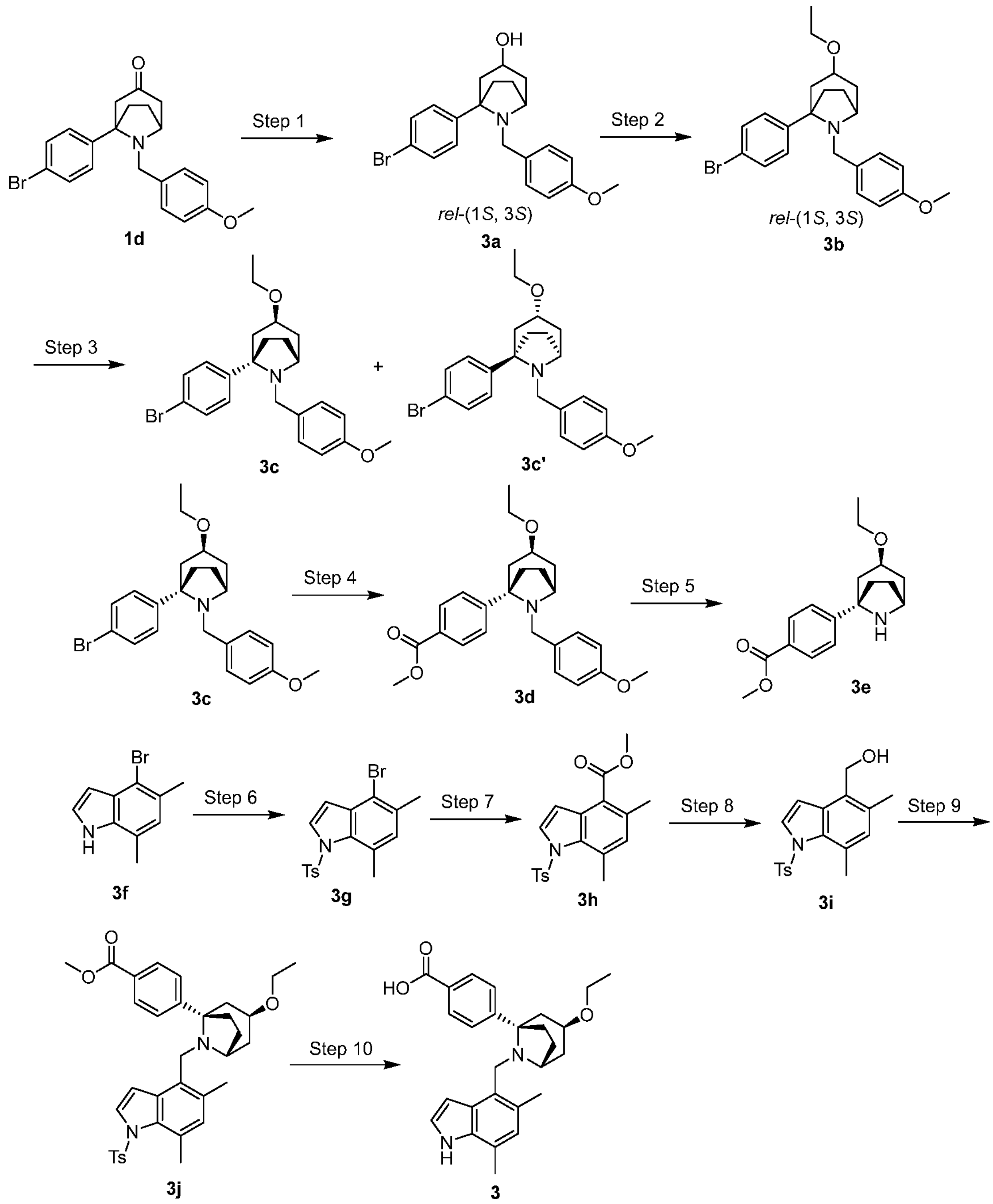


1d, rel-(1S, 3S) 3a, rel-(1S, 3S) 3b, 3c + 3c', 3c, 3d, 3e, 3f, 3g, 3h, 3i, 3j, 3

Step 1

(±)-*rel*-(1*S*,3*S*)-1-(4-Bromophenyl)-8-(4-methoxybenzyl)-8-azabicyclo[3.2.1]octan-3-ol **3a**

Compound **1d** (22.5 g, 56.21 mmol) was dissolved in tetrahydrofuran (220 mL), and a solution of tri-sec-butyllithium borohydride in tetrahydrofuran (168.86 mL, 168.86 mmol) was added dropwise at -78 °C under nitrogen atmosphere. After the addition, the mixture was stirred at -78 °C for 3 h. The reaction mixture was quenched by adding methanol (100 mL) and stirred at room temperature for 1 h. Ethyl acetate was added, and the mixture was washed sequentially with 0.5 M hydrochloric acid and saturated brine. The organic phase was dried over anhydrous sodium sulfate, filtered, and concentrated under reduced pressure. The residue was purified by column chromatography with eluent system C and then washed with saturated aqueous sodium bicarbonate solution and saturated brine. The organic phase was dried over anhydrous sodium sulfate, filtered, and concentrated under reduced pressure to give the title compound **3a** (18.7 g, yield: 82.69%).

MS m/z (ESI): 401.9 [M+1].

Step 2

(±)-*rel*-(1*S*,3*S*)-1-(4-Bromophenyl)-3-ethoxy-8-(4-methoxybenzyl)-8-azabicyclo[3.2.1]o ctane **3b**

**3a** (18.7 g, 46.48 mmol) was dissolved in tetrahydrofuran (200 mL), and sodium hydride (8.91 g, 232.54 mmol, content 60%) was added at 0 °C. The mixture was stirred for another hour, and 18.62 mL of iodoethane (36.3 g, 232.74 mmol) was added. The reaction mixture was stirred at 50 °C overnight, cooled to room temperature, quenched by adding saturated aqueous ammonium chloride solution, extracted with ethyl acetate, washed with saturated brine, dried over anhydrous sodium sulfate, filtered, and concentrated under reduced pressure. The residue was purified by column chromatography with eluent system C to give the title compound **3b** (13.0 g, yield: 65%).
MS m/z (ESI): 429.9 [M+1].

Step 3

(1*S*,3*S*,5*R*)-1-(4-Bromophenyl)-3-ethoxy-8-(4-methoxybenzyl)-8-azabicyclo[3.2.1]octan e **3c**

(1*R*,3*R*,5*S*)-1-(4-Bromophenyl)-3-ethoxy-8-(4-methoxybenzyl)-8-azabicyclo[3.2.1]octa ne **3c'**

Compound **3b** was purified by preparative chiral chromatography (resolution conditions: preparative chiral column Waters SFC 150, DAICEL CHIRALCEL®OJ, mobile phase: supercritical carbon dioxide/methanol = 70/30)), and the corresponding fractions were collected and concentrated under reduced pressure to give the title compounds **3c** (7.2 g, yield: 55.38%) and **3c'** (5.8 g, yield: 44.62%).

Compound **3c:**

MS m/z (ESI): 429.9 [M+1].
Chiral HPLC analysis: retention time 7.946 min, chiral purity: 100% (column: CHIRALPAK IG, 5 μm, 20 mm × 250 mm (Phenomenex); mobile phase 1: n-hexane (80%); mobile phase 2: containing 0.1% diethylamine, 0.1% trifluoroacetic acid, ethanol (20%), flow rate: 1 mL/min).

Compound **3c':**

MS m/z (ESI): 429.9 [M+1].
Chiral HPLC analysis: retention time 12.77 min, chiral purity: 98.7% (column: CHIRALPAKIG, 5 μm, 20 mm × 250 mm (Phenomenex); mobile phase 1: n-hexane (80%); mobile phase 2: containing 0.1% diethylamine, 0.1% trifluoroacetic acid, ethanol (20%), flow rate: 1 mL/min).

Step 4

Methyl 4-((1*S*,3*S*,5*R*)-3-ethoxy-8-(4-methoxybenzyl)-8-azabicyclo[3.2.1]octan-1-yl)benzoate **3d**

Compound **3c** (7.2 g, 16.73 mmol) was dissolved in methanol (40 mL) and *N*,*N*-dimethylformamide (40 mL), and palladium acetate (1.14 g, 5.08 mmol, Innochem reagent), 1,3-bis(diphenylphosphino)propane (2.08 g, 5.04 mmol, Accela ChemBio Inc.), and triethylamine (17 g, 168.00 mmol) were added. The system was purged three times with carbon monoxide, and the mixture was stirred at 80 °C overnight in a carbon monoxide atmosphere. After the reaction was complete, the reaction mixture was poured into water, extracted with ethyl acetate, and washed with saturated brine. The organic phase was dried over anhydrous sodium sulfate, filtered, and concentrated. The residue was purified by column chromatography with eluent system C to give the title compound **3d** (5.9 g, yield: 86.12%).
MS m/z (ESI): 410.0 [M+1].

Step 5

Methyl 4-((1*S*,3*S*,5*R*)-3-ethoxy-8-azabicyclo[3.2.1]octan-1-yl)benzoate **3e**

Compound **3d** (5.9 g, 14.41 mmol) was dissolved in water (30 mL) and acetonitrile (30 mL), the solution was cooled to 0 °C, and ceric ammonium nitrate (23.7 g, 43.23 mmol, Adamas Reagent Co., Ltd.) was added. After 1 h of

reaction at room temperature, ceric ammonium nitrate (16 g, 29.18 mmol) was added again, and the reaction was continued at room temperature for 1 h. A large amount of water was added, and extraction was performed 2 times with dichloromethane. The organic phases were combined, washed with saturated brine, dried over anhydrous sodium sulfate, filtered, and concentrated. The residue was purified by column chromatography with eluent system A to give the title compound **3e** (3.2 g, yield: 76.76%).

MS m/z (ESI): 290.0 [M+1].

Step 6

4-Bromo-5,7-dimethyl-1-tosyl-1*H*-indole **3g**

4-Bromo-5,7-dimethyl-1*H*-indole **3f** (1.4 g, 6.25 mmol, Acon reagent) was dissolved in N,N-dimethylformamide (25 mL), the solution was cooled to 0 °C, and sodium hydride (500 mg, 12.50 mmol, content 60%) was added. After the reaction mixture was stirred for 10 min, p-toluenesulfonyl chloride (1.3 g, 6.82 mmol, SinoPharm reagent) was added. After the addition, the mixture was stirred at room temperature overnight. Saturated aqueous ammonium chloride solution was added to the reaction mixture, and the mixture was extracted with ethyl acetate, washed with saturated brine, dried over anhydrous sodium sulfate, filtered, and concentrated. The residue was purified by column chromatography with eluent system C to give the title compound **3g** (1.2 g, yield: 50.78%).

MS m/z (ESI): 377.9 [M+1].

Step 7

Methyl 5,7-dimethyl-1-tosyl-1*H*-indole-4-carboxylate **3h**

Compound **3g** (600 mg, 1.59 mmol) was dissolved in methanol (5 mL) and *N*,*N*-dimethylformamide (5 mL), and palladium acetate (106.9 mg, 476.15 μmol), 1,3-bis(diphenylphosphino)propane (196.3 mg, 475.94 μmol), and triethylamine (1.61 g, 15.86 mmol) were added. The system was purged three times with carbon monoxide, and the mixture was stirred at 80 °C overnight in a carbon monoxide atmosphere. After the reaction was complete, the reaction mixture was poured into water, extracted with ethyl acetate, and washed with saturated brine. The organic phase was dried over anhydrous sodium sulfate, filtered, and concentrated. The residue was purified by column chromatography with eluent system C to give the title compound **3h** (488 mg, yield: 86.08%).

MS m/z (ESI): 357.9 [M+1].

Step 8

(5,7-Dimethyl-1-tosyl-1*H*-indol-4-yl)methanol **3i**

Compound **3h** (488 mg, 1.37 mmol) was dissolved in tetrahydrofuran (5 mL), and a solution of lithium borohydride in tetrahydrofuran (3.06 mmol, 1.53 mL) was added at 0 °C. After the addition, the mixture was stirred at room temperature overnight. The reaction mixture was cooled to 0 °C and quenched by slowly adding dropwise saturated aqueous ammonium chloride solution until no bubbles were produced. Ethyl acetate was added, and the organic phase was washed with saturated brine, dried over anhydrous sodium sulfate, filtered, and concentrated. The residue was purified by column chromatography with eluent system C to give the title compound **3i** (253 mg, yield: 56.25%).

MS m/z (ESI): 311.9 [M-OH].

Step 9

Methyl 4-((1*S*,3*S*,5*R*)-8-((5,7-dimethyl-1-tosyl-1*H*-indol-4-yl)methyl)-3-ethoxy-8-azabicyclo[3. 2.1]octan-1-yl)benzoate **3j**

Compound **3i** (253 mg, 768.03 μmol) was dissolved in dichloromethane (5.6 mL), the solution was cooled to 0 °C under nitrogen atmosphere, and carbon tetrabromide (255.1 mg, 769.24 μmol, Energy reagent) and triphenylphosphine (202.4 mg, 771.68 μmol, SinoPharm reagent) were added sequentially. The reaction mixture was stirred at 0 °C for 1.5 h. The resulting solution was kept for later use. Compound **3e** (150.7 mg, 0.52 mmol) was dissolved in *N*,*N*-dimethylformamide (4 mL), and sodium hydride (43 mg, 1.08 mmol, content 60%) was added at 0 °C. The mixture was stirred for another hour, and the above solution was added. The mixture was gradually warmed to room temperature and stirred for 1 h. The reaction mixture was quenched by adding saturated aqueous ammonium chloride solution, extracted with ethyl acetate, washed with saturated brine, dried over anhydrous sodium sulfate, filtered, and concentrated. The residue

was purified by column chromatography with eluent system C to give the title compound **3j** (67 mg, yield: 21.45%).
MS m/z (ESI): 600.9 [M+1].

Step 10

4-((1*S*,3*S*,5*R*)-8-((5,7-Dimethyl-1*H*-indol-4-yl)methyl)-3-ethoxy-8-azabicyclo[3.2.1]oct an-1-yl)benzoic acid **3**

Compound **3j** (67 mg, 111.52 μmol) was dissolved in methanol (1.4 mL) and water (0.3 mL), and potassium hydroxide (62.6 mg, 1.12 mmol) was added. The reaction mixture was stirred at 60 °C overnight in a sealed tube. The reaction mixture was cooled to room temperature, the pH of the solution was adjusted to 5 to 6 with 1 M hydrochloric acid, and water and ethyl acetate were added. The organic phase was washed with saturated brine, dried over anhydrous sodium sulfate, filtered, and concentrated. The residue was dissolved with a small amount of methanol and purified by preparative high performance liquid chromatography (instrument model: Waters Autopurification-SQD2; column: Welch XB-C18 30 mm × 150 mm; 5 μm; mobile phase 1: purified water (containing 10 mM ammonium bicarbonate); mobile phase 2: acetonitrile; gradient: 25%-95%; flow rate: 30 mL/min) to give the title compound 3 (6 mg, yield: 12.44%).
MS m/z (ESI): 433.1 [M+1].
$^1$H NMR (500 MHz, $CD_3OD$): δ 8.14 (d, 2H), 7.72-7.74 (m, 2H), 7.33 (s, 1H), 6.86 (s, 1H), 6.51 (br, 1H), 4.60 (s, 1H), 4.08 (br, 3H), 3.73 (br, 1H), 3.32-3.37 (m, 4H), 2.69 (br, 4H), 2.48 (s, 3H), 2.29-2.37 (m, 4H), 1.23 (t, 3H).

Example 4

4-((1*S*,3*S*,5*R*)-8-((5-Cyclopropyl-7-methyl-1*H*-indol-4-yl)methyl)-3-ethoxy-8-azabicycl o[3.2.1]octan-1-yl)benzoic acid **4**

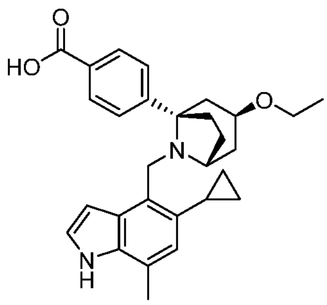

**4**

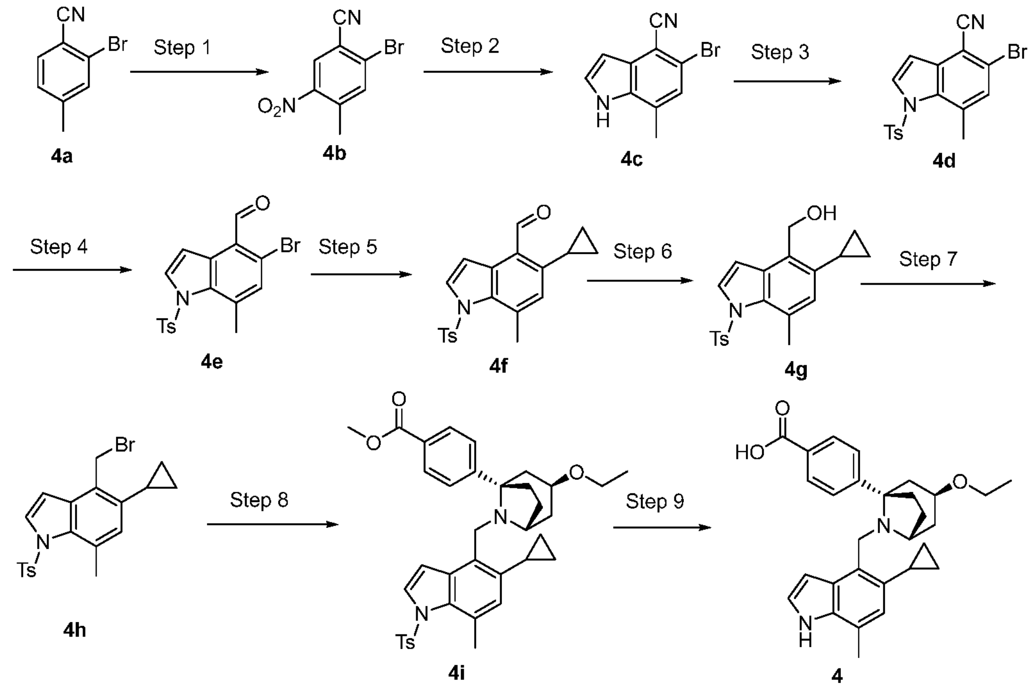


Step 1

2-Bromo-4-methyl-5-nitrobenzonitrile **4b**

2-Bromo-4-methylbenzonitrile **4a** (4 g, 20.40 mmol, Bide Pharmatech Ltd.) was dissolved in sulfuric acid (6 mL), and a solution of potassium nitrate (2.06 g, 20.40 mmol) in sulfuric acid (18 mL) was added dropwise under ice bath. After the dropwise addition, the mixture was reacted at 0 °C for 1.5 h. The reaction mixture was poured into ice water, and the mixture was stirred and filtered. The filter cake was washed with ice water and dried to give the crude title product **4b** (4.9 g). The product was directly used in the next step without purification.
MS m/z (ESI): 240.8 [M-1].

Step 2

5-Bromo-7-methyl-1*H*-indole-4-carbonitrile **4c**

Compound **4b** (3.9 g, 16.18 mmol) was dissolved in anhydrous tetrahydrofuran (30 mL), the solution was cooled to -20 °C under nitrogen atmosphere, and vinylmagnesium bromide (64.76 mL, 64.76 mmol) was added dropwise. After the dropwise addition, the mixture was reacted at room temperature for 1.5 h, cooled to -20 °C, quenched with an aqueous sodium hydroxide solution, and extracted 2 times with ethyl acetate (100 mL). The organic phases were combined, washed with saturated sodium bicarbonate (50 mL), dried over anhydrous sodium sulfate, filtered, concentrated, and purified by silica gel column chromatography with eluent B to give the title compound **4c** (500 mg, yield: 13.1%).
MS m/z (ESI): 234.9 [M-1].

Step 3

5-Bromo-7-methyl-1-tosyl-1*H*-indole-4-carbonitrile **4d**

Compound **4c** (500 mg, 2.13 mmol) was dissolved in dichloromethane (10 mL), 4-toluenesulfonyl chloride (609 mg, 3.19 mmol) and benzyltriethylammonium chloride (48.5 mg, 212.93 μmol) were added, and then sodium hydroxide (128 mg, 3.20 mmol) was added. The mixture was reacted at room temperature overnight and concentrated. The residue was purified by silica gel column chromatography with eluent B to give the title compound **4d** (500 mg, yield: 60.4%).
MS m/z (ESI): 388.9 [M-1].

Step 4

5-Bromo-7-methyl-1-tosyl-1*H*-indole-4-carbaldehyde **4e**

Compound **4d** (500 mg, 1.28 mmol) was dissolved in dichloromethane (5 mL), and 1 M diisobutylaluminum hydride (1.9274 mL) was added dropwise at -78 °C. The mixture was reacted at -78 °C for 1.5 h and quenched with methanol, and 2 M hydrochloric acid (20 mL) was added. The mixture was extracted 2 times with dichloromethane (50 mL). The organic phases were combined, dried over anhydrous sodium sulfate, filtered, and concentrated. The residue was purified by silica gel column chromatography with eluent B to give the title compound **4e** (140 mg, yield: 27.8%).
MS m/z (ESI): 392.0 [M-1].

Step 5

5-Cyclopropyl-7-methyl-1-tosyl-1*H*-indole-4-carbaldehyde **4f**

Compound **4e** (140 mg, 356.9 μmol) was dissolved in toluene (3 mL) and water (1 mL), and potassium cyclopropyltrifluoroborate (106 mg, 716.33 μmol), 2-dicyclohexylphosphino-2',6'-diisopropoxy-1,1'-biphenyl (50 mg, 107.15 μmol), palladium acetate (16.1 mg, 71.71 μmol), and cesium carbonate (698 mg, 2.14 mmol) were added. The mixture was reacted at 100 °C for 2 h in a nitrogen atmosphere, filtered, and concentrated to give the crude title product **4f** (126 mg). The product was directly used in the next step without purification.
MS m/z (ESI): 353.9 [M+1].

Step 6

(5-Cyclopropyl-7-methyl-1-tosyl-1*H*-indol-4-yl)methanol **4g**

Compound **4f** (126 mg, 356.50 μmol) was dissolved in methanol (3 mL), and sodium borohydride (27 mg, 713.67 μmol) was added at 0 °C. The mixture was reacted at room temperature for 2 h, quenched by adding saturated aqueous ammonium chloride solution (10 mL), and extracted 2 times with ethyl acetate (20 mL). The organic phases were combined, dried over anhydrous sodium sulfate, filtered, and concentrated. The residue was purified by silica gel column chromatography with eluent B to give the title compound **4g** (126 mg, yield: 99.4%).
MS m/z (ESI): 354.1 [M-1].

Step 7

4-(Bromomethyl)-5-cyclopropyl-7-methyl-1-tosyl-1*H*-indole **4h**

Compound **4g** (70 mg, 196.93 μmol) was dissolved in dichloromethane (1 mL), carbon tetrabromide (65.4 mg, 197.21 μmol) was added, and triphenylphosphine (51.7 mg, 197.11 μmol) was added at 0 °C. The mixture was reacted at 0 °C for 1.5 h and concentrated to give the crude title product **4h** (40 mg, yield: 48.5%). The product was directly used in the next step without purification.

Step 8

Methyl 4-((1*S*,3*S*,5*R*)-8-((5-cyclopropyl-7-methyl-1-tosyl-1*H*-indol-4-yl)methyl)-3-ethoxy-8-az abicyclo[3.2.1]octan-1-yl)benzoate **4i**

Compound **3e** (40 mg, 138.23 μmol) was dissolved in *N,N*-dimethylformamide (1 mL), and sodium hydride (11 mg, 287.08 μmol, content 60%) was added at 0 °C. The mixture was stirred for another hour, and a solution of compound **4h** (40 mg, 95.61 μmol) in *N,N*-dimethylformamide (1 mL) was added. The mixture was stirred at room temperature for 1 h, quenched by adding saturated aqueous ammonium chloride solution (10 mL), and extracted 2 times with ethyl acetate (20 mL). The organic phases were combined, dried over anhydrous sodium sulfate, filtered, and concentrated to give the crude title product **4i** (59 mg). The product was directly used in the next step without purification.
MS m/z (ESI): 626.9 [M+1].

Step 9

4-((1*S*,3*S*,5*R*)-8-((5-Cyclopropyl-7-methyl-1*H*-indol-4-yl)methyl)-3-ethoxy-8-azabicycl o[3.2.1]octan-1-yl)benzoic acid **4**

Compound **4i** (59 mg, 94.13 μmol) was dissolved in methanol (1 mL) and water (0.25 mL), and potassium hydroxide (27 mg, 481.24 μmol) was added. The mixture was reacted at 60 °C overnight, adjusted to pH 8-9 with a 2 M hydrochloric acid solution, concentrated, diluted with methanol, and purified by preparative high performance liquid chromatography (Waters 2545, column: Sharpsil-T C18, 30 × 150 mm, 5 μm; mobile phase: water (containing 10 mmol/L ammonium bicarbonate); mobile phase: acetonitrile; gradient: 36%-43%, flow rate: 30 mL/min) to give the title compound **4** (20 mg, yield: 46.3%).

MS m/z (ESI): 459.1 [M+1].

$^1H$ NMR(500 MHz, $CD_3OD$): $\delta$ 8.08-8.10 (d, 2H), 7.72-7.74 (d, 2H), 7.33 (s, 1H), 6.72 (s,1H), 6.42-6.55 (m, 1H), 4.03-4.42 (m, 4H), 3.74 (s, 1H), 3.43-3.56 (m, 2H), 3.35 (s, 2H), 2.85 (s, 1H), 2.55-2.75 (m, 3H), 2.47 (s, 3H), 1.99-2.30 (m, 4H), 1.26-1.40 (m, 3H), 1.18-1.25 (m, 2H).

Example 5

(2*S*,3*S*,4*S*,5*R*,6*S*)-6-((4-((1*S*,3*S*,5*R*)-3-Ethoxy-8-((5-methoxy-7-methyl-1H-indol-4-yl)m ethyl)-8-azabicyclo[3.2.1]octan-1-yl)benzoyl)oxy)-3,4,5-trihydroxytetrahydro-2*H*-pyran -2-carboxylic acid **5**

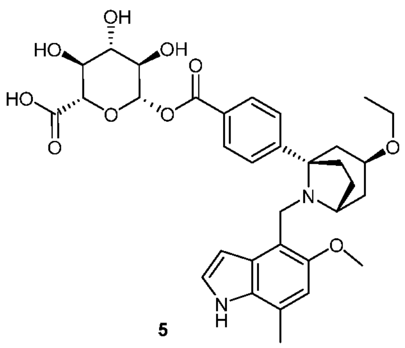

**5**

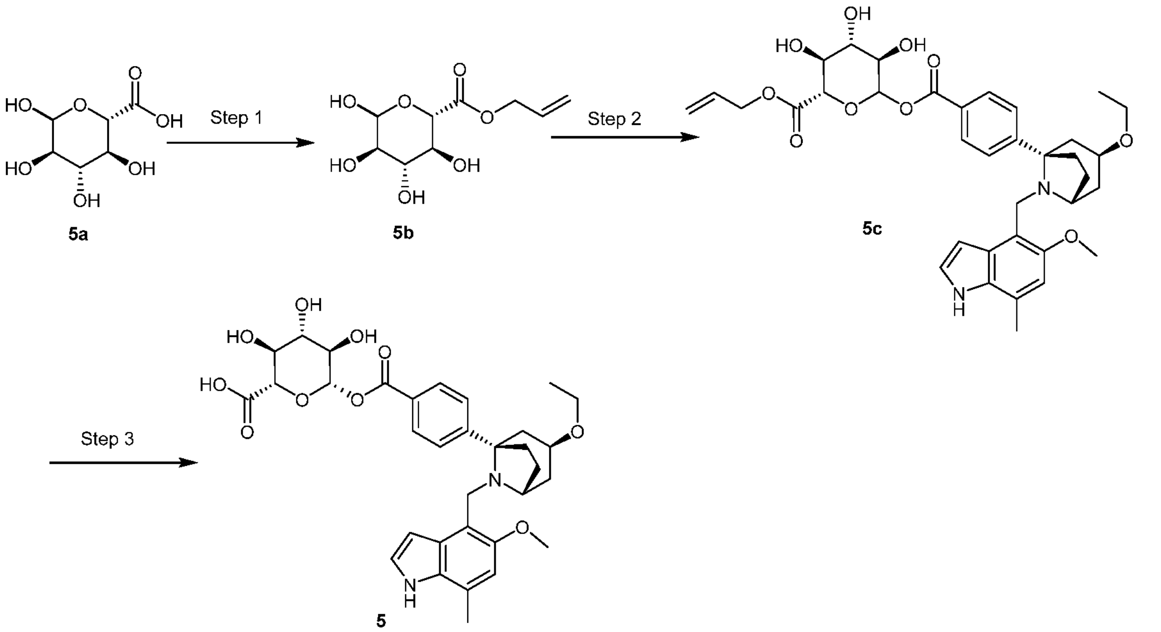


**5a** **5b** **5c** **5**

Step 1

Allyl (2*S*,3*S*,4*S*,5*R*)-3,4,5,6-tetrahydroxytetrahydro-2*H*-pyran-2-carboxylate **5b** D-glucuronic acid **5a** (15 g,

77.26 mmol, Jiangsu Aikon Biopharmaceutical R&D Co., Ltd.) was dissolved in *N,N*-dimethylformamide (150 mL), and 1,8-diazabicyclo[5.4.0]undec-7-ene (13 g, 85.39 mmol) was added dropwise at room temperature. The mixture was well stirred and cooled in an ice bath for 10 min, and 3-bromopropene (11.2 g, 92.58 mmol) was added dropwise. After the addition, the mixture was reacted at room temperature for 16 h, quenched by adding 1.5 mL of water, and stirred for 5 min. The reaction mixture was concentrated, and the residue was purified by column chromatography with eluent system E to give the title compound **5b** (14 g, yield: 77.4%).

MS m/z (ESI): 235.1 [M+1].

Step 2

Allyl (2*S*,3*S*,4*S*,*SR*)-6-((4-((1*S*,3*S*,5*R*)-3-ethoxy-8-((5-methoxy-7-methyl-1H-indol-4-yl)meth yl)-8-azabicyclo[3.2.1]octan-1-yl)benzoyl)oxy)-3,4,5-trihydroxytetrahydro-2H-pyran-2-carboxylate **5c**

Acetonitrile (100 mL) was added to compound **1-1** (5 g, 11.15 mmol) and compound **5b** (4.18 g, 17.85 mmol), and 2-(7-azobenzotriazol)-*N,N*,*N*',*N*'-tetramethyluronium hexafluorophosphate (5.09 g, 13.39 mmol) was added. The mixture was well stirred, and N-methylmorpholine (3.95 g, 39.05 mmol) was added dropwise to the reaction mixture. After the addition, the mixture was reacted at room temperature for 16 h. The reaction mixture was quenched by adding acetic acid (2.68 g, 44.63 mmol) and concentrated under reduced pressure. The residue was purified by silica gel column chromatography with eluent A to give the crude title compound **5c** (12.3 g). The product was directly used in the next step without purification.

MS m/z (ESI): 665.3 [M+1].

Step 3

(2*S*,3*S*,4*S*,5*R*,6*S*)-6-((4-((1*S*,3*S*,5*R*)-3-Ethoxy-8-((5-methoxy-7-methyl-1H-indol-4-yl)m ethyl)-8-azabicyclo[3.2.1]octan-1-yl)benzoyl)oxy)-3,4,5-trihydroxytetrahydro-2*H*-pyran -2-carboxylic acid **5**

Compound **5c** (10.5 g, 15.80 mmol) was dissolved in acetonitrile (85 mL), the solution was cooled in an ice bath for 10 min, and tetrakis(triphenylphosphine)palladium(0) (3.65 g, 3.16 mmol) and tetrahydropyrrole (1.25 g, 17.58 mmol) were added. The mixture was reacted in an ice bath for 3 h. Acetic acid (2.85 g, 47.46 mmol) was added to quench the reaction, and 15 mL of *N,N*-dimethylformamide was added. The mixture was stirred for 5 min and filtered. The filtrate was concentrated under reduced pressure, and the residue was purified by preparative high performance liquid chromatography (Waters 2545, column: Welch Xtimate C18, 5 $\mu$m, 30 mm $\times$ 150 mm; mobile phase: water (containing 0.1% formic acid, adjusted to pH 4.5 with ammonia water); mobile phase: acetonitrile; gradient: 15%-35%, flow rate: 30 mL/min) to give the title compound 5 (2.47 g, yield: 25.0%).

MS m/z (ESI): 625.2 [M+1].

$^1$H NMR(500 MHz, DMSO-$d_6$): $\delta$10.84 (s,1H), 8.26-8.27(d, 1H), 8.05-8.07(d, 2H), 7.81-7.83(d, 2H), 7.28-7.29(t, 1H), 6.67(s,1H), 6.53(s,1H), 5.57-5.59 (m, 1H), 3.73-3.75 (m, 3H), 3.69-3.71(d, 1H), 3.50-3.52 (d, 1H), 3.39-3.43 (m, 2H), 3.21-3.32 (m, 4H), 3.10-3.11(d, 1H), 2.51 (s, 3H), 2.45 (s, 1H), 2.43 (s, 3H), 2.20-2.24 (dd, 1H), 1.97-2.10 (m, 5H), 1.52-1.53 (d, 1H), 1.24 (s,1H), 1.14-1.14 (m, 3H).

Biological Evaluation

The present disclosure is further described and explained below with reference to test examples, but these examples are not intended to limit the scope of the present disclosure.

Test Example 1: Inhibitory Effects of Compounds Disclosed Herein on Factor B Enzyme Activity

I. Experimental materials and instruments

1. Recombinant human complement factor B protein (expressed by Nanjing GenScript Biotech Co., Ltd.)
2. Recombinant human complement factor D protein (1824-SE-010, R&D system)
3. Human complement factor C3 (204885-250UGCN, EMDmillipore)
4. Cobra venom factor (CVF) (A600, Quidel)
5. StartingBlock™ T20 (TBS) blocking buffer (37543, Thermo Fisher)
6. Goat anti-mouse IgG heavy chain + light chain (horseradish peroxidase-labeled) (ab205719, Abcam)

7. Anti-C3a/C3a des Arg antibody, clone number [2991] (ab11873, Abcam)
8. QuantaBlu™ fluorogenic peroxidase substrate kit (15169, Thermo Fisher)
9. Amphoteric surfactant (CHAPS) (C3023, Sigma)
10. Magnesium chloride solution (M1028-100ML, Sigma)
11. Sodium carbonate $Na_2CO_3$ (10019260, Hushi)
12. Sodium bicarbonate $NaHCO_3$ (10018960, Hushi)
13. Tween 20 (P7949-500ML, Sigma)
14. 20× PBS buffer (B548117-0500, Sangon)
15. 96-well low-volume white plate (66PL96025, Cisbio)
16. 96-well adsorption black plate (437111, Thermo Fisher)
17. Phosphate-buffered saline (B320, Shanghai BasalMedia Technologies Co., Ltd.)
18. Sterile purified water (made in-house by Shanghai Hengrui)
19. 96-well formulating plate (3795, Corning)
20. Constant-temperature incubator (Shanghai Yiheng Scientific Instruments Co., Ltd.)
21. Flexstation3 microplate reader (Molecular Device)

II. Experimental procedures

The functioning of the human complement factor B protein as a protease requires binding to human complement factor C3 to form a complex. Human complement factor B is hydrolyzed into the Ba and Bb fragments by the human complement factor D protein. Bb and the C3b fragment of human complement factor C3 form a complex C3bBb, i.e., C3 convertase. Only the formation of the complex can enable human complement factor B to function as a protease. C3bBb continues to hydrolyze C3 into the C3a and C3b fragments. C3b and C3bBb form a complex C3bBbC3b, i.e., C5 convertase, and the C3a fragment is released. Assays for the C3a des Arg epitope generated after C3 is cleaved can be used to evaluate the efficiency of C3 being hydrolyzed, i.e., the C3bBb enzyme activity, and thereby to evaluate the effects of the compounds on the C3bBb enzyme. Since C3b is unstable *in vitro,* cobra venom factor (hereinafter referred to as CVF) was used in place of C3b to form a complex with human complement factor B. It functions in the same way as C3b.

The coding gene for the AA128-2422 amino acid segment of the human complement factor B protein (NM_001710.6) was subjected to codon optimization, gene synthesis, and cloning into the pcDNA3.4 vector by Nanjing GenScript Biotech Co., Ltd. and was expressed in HD CHO-S cells, and the product was purified. The recombinant human complement factor B protein obtained from the purification was aliquoted and then stored in a -80 °C freezer.

Cleavage reactions of the human complement factor B protein: The recombinant human complement factor D protein was diluted 10-fold with PBS (pH 7.4) and stored on ice for later use. The recombinant human complement factor D protein, the recombinant human complement factor B protein, and CVF were added to a reaction buffer (PBS pH 7.4, 10 mM $MgCl_2$, 0.05% CHAPS) to final concentrations of 300 nM, 1 μM, and 1 μM, respectively. After they were well mixed, the mixture was reacted in a constant-temperature incubator at 37 °C for 3 h to give a complex of CVF and the post-cleavage fragment Bb of the recombinant human complement factor B protein (hereinafter referred to as CVF:Bb).

A 100 mM $Na_2CO_3$ solution and a 100 mM $NaHCO_3$ solution were prepared and mixed in a ratio of $Na_2CO_3$ to $NaHCO_3$ of 3:7 (v/v) to adjust the pH to 9.5. The mixture was stored at room temperature for later use.

20 mM test compounds dissolved in 100% DMSO were serially diluted with 100% DMSO to 2000, 500, 125, 31.25, 7.8125, 0.488281, 0.12207, 0.030518, and 0.007629 μM, with the blank well containing 100% DMSO. The compounds and 100% DMSO were then 20-fold diluted in C3 reaction buffer (PBS pH 7.4, 1 mM $MgCl_2$, 0.05% CHAPS).

Cleavage reactions of the C3 protein: In a 96-well low-volume white plate, 10-μL reaction systems were prepared by adding CVF:Bb (to a final concentration of 2 nM) and 1 μL of the above test compounds and DMSO diluted in C3 reaction buffer to C3 reaction buffer (PBS pH 7.4, 1 mM $MgCl_2$, 0.05% CHAPS). The plate was incubated at room temperature for 1 h. The final concentrations of the test compounds were 10,000, 2500, 625, 156.25, 39.0625, 9.765625, 2.441406, 0.6103515, and 0.152588 nM, respectively. Human complement factor C3 was added to the reaction systems to a final concentration of 500 nM. After they were well mixed, the mixtures were reacted in a constant-temperature incubator at 37 °C for 2 h. Among the reaction mixtures, the reaction well that contained only 500 nM human complement factor C3 was used as a negative control. To a 96-well adsorption black plate, 97 μL of the carbonic acid buffer (pH 9.5) was added, and the C3 protein cleavage reaction mixtures were added at 3 μL/well. After they were well mixed, the plate was sealed and incubated at 4 °C overnight.

C3a des Arg assays: The plate was washed 3 times with 300 μL/well TBST (0.05% tween 20) solution, the StartingBlock™ T20 (TBS) blocking buffer was added at 300 μL/well, and the plate was incubated at 37 °C for 5 min. The plate was washed 3 times with 300 μL/well PBST solution, the anti-C3a/C3a des Arg antibody [2991] was 1:1000 diluted in PBST solution and added at 100 μL/well, and the plate was incubated at 37 °C for 1 h. The plate was washed 3 times with 300 μL/well PBST solution, the goat anti-mouse IgG H&L (HRP) antibody was 1:5000 diluted in PBST

solution and added at 100 μL/well, and the plate was incubated at 37 °C for 30 min. A QuantaBlu™ fluorogenic peroxidase substrate kit substrate was prepared by diluting 1 part of the QuantaBlu™ stable peroxide solution with 9 parts of the QuantaBlu™ substrate solution. The plate was washed 3 times with 300 μL/well PBST solution, and dried after the last wash. The substrate was added at 100 μL/well, and the plate was incubated at room temperature for 20 min. After the QuantaBlu™ stop solution was added at 100 μL/well, the fluorescence readings were taken on Flexstation, with the excitation light wavelength Ex set to 320 nM and the emission light wavelength Em set to 460 nM, cutoff 455.

The inhibition rate was calculated using the following formula:

$$\text{Inhibition rate} = \{1- (RFU_{\text{test compound}} - RFU_{\text{negative control well}}) / (RFU_{\text{blank well}} - RFU_{\text{negative control well}})\} \times 100\%$$

Inhibition curves were plotted using Graphpad Prism software according to the concentrations of the compounds and the corresponding inhibition rates, and the concentrations of the compounds at which the inhibition rate reached 50%, i.e., $IC_{50}$ values, were calculated.

Table 1. The $IC_{50}$ values of the compounds of the present disclosure as measured by their inhibitory effects on the factor B enzyme activity

| Example No. | $IC_{50}$(nM) |
|---|---|
| **1** | 4.2 |
| **1-1** | 1.3 |
| **1-2** | 72.2 |
| **3** | 4.9 |
| **4** | 1.6 |
| **5** | 30.0 |

Conclusion: the compounds of the present disclosure have good inhibitory effects on the factor B enzyme activity.

Test Example 2: Inhibitory Effects of Compounds Disclosed Herein on Human Complement Alternative Pathway

I. Experimental materials and instruments

1. Zymosan A (Z4250, Sigma)
2. Human serum
3. StartingBlock™ T20 (TBS) blocking buffer (37543, Thermo Fisher)
4. Goat anti-mouse IgG heavy chain + light chain (horseradish peroxidase-labeled) (ab205719, Abcam)
5. Anti-C5b-9 + C5b-8 antibody, clone number [aE11] (ab66768, Abcam)
6. QuantaBlu™ fluorogenic peroxidase substrate kit (15169, Thermo Fisher)
7. Gelatin, derived from the skin of cold-water fish (G7765, Sigma)
8. EDTA (AM9260G, Invitrogen)
9. EGTA (E3889-100G, Sigma)
10. Magnesium chloride solution (M1028-100ML, Sigma)
11. Buffer (HEPES) (H3375-250G, Sigma)
12. Calcium chloride (C5670-500G, Sigma)
13. Sodium carbonate $Na_2CO_3$ (10019260, Hushi)
14. Sodium bicarbonate $NaHCO_3$ (10018960, Hushi)
15. 20× TBS (B548105-0500, Sangong)
16. Tween 20 (P7949-500ML, Sigma)
17. 96-well adsorption black plate (437111, Thermo Fisher)
18. Phosphate-buffered saline (PBS, pH 7.4) (B320, Shanghai BasalMedia Technologies Co., Ltd.)
19. Sterile purified water (made in-house by Shanghai Hengrui)
20. 96-well formulating plate (3795, Corning)
21. Constant-temperature incubator (Shanghai Yiheng Scientific Instruments Co., Ltd.)

22. Flexstation3 microplate reader (Molecular Device)

II. Experimental procedures

There are 3 major pathways for complement activation: the classical pathway, the lectin pathway, and the alternative pathway. The alternative pathway can be activated by exogenous pathogenic bacterial molecules such as bacterial cell wall proteins and lipopolysaccharides. A range of complement factors amplify the activation signal in a cascaded way via hydrolysis and enzymolysis reactions, and finally, C5b, C6, C7, C8, and several C9 proteins form a membrane attack complex. The alternative pathway can be activated by zymosan. The extent of activation of the alternative pathway can be evaluated by assaying the formation of the C5b-9 complex. The zymosan can also activate the classical pathway and the lectin pathway. Since the activation of the two pathways requires both magnesium and calcium ions, and the alternative pathway requires magnesium ions only, after chelation with the calcium ion chelator EGTA, the alternative pathway is not inhibited, while the classical pathway and the lectin pathway are. After chelation with EDTA, the three pathways are all inhibited. Therefore, the inhibitory effects of the small-molecule compounds on the alternative pathway can be evaluated by activating the complement alternative pathway in human serum with zymosan in the presence of the EGTA chelator and assaying the formation of the C5b-9 complex.

Zymosan A powder was resuspended at 10 mg/mL in TBS solution and activated by heating at 100 °C for 2 h. After the activation, the suspension was centrifuged at 4000 rpm for 30 min, and the supernatant was discarded. The residue was resuspended in TBS to form a 50 mg/mL suspension. The suspension was aliquoted and stored in a -20 °C freezer. The day before the experiment, the activated zymosan A solution was diluted to 1 mg/mL with the carbonic acid buffer (pH 9.5) and added to a 96-well adsorption black plate at 100 μL/well, and the plate was coated at 4 °C overnight.

A 100 mM $Na_2CO_3$ solution and a 100 mM $NaHCO_3$ solution were prepared and mixed in a ratio of $Na_2CO_3$ to $NaHCO_3$ of 3:7 (v/v) to adjust the pH to 9.5. The mixture was stored at room temperature for later use.

Activation reactions of the complement alternative pathway: Human serum was 5-fold diluted with PBS solution to form a 20% serum solution, and the solution was placed on ice for later use. 20 mM test compounds dissolved in 100% DMSO were serially diluted with 100% DMSO to 2000, 500, 125, 31.25, 7.8125, 0.488281, 0.12207, 0.030518, and 0.007629 μM, with the blank well containing 100% DMSO. The compounds and 100% DMSO were then 20-fold diluted in 2× EGTA reaction buffer (4.2 mM HEPES, 0.15 mM $CaCl_2$, 140 mM NaCl, 4.5 mM MgClz, 0.1% gelatin, 20 mM EGTA). Meanwhile, 100% DMSO was 20-fold diluted in 2× EDTA reaction buffer (4.2 mM HEPES, 0.15 mM $CaCl_2$, 140 mM NaCl, 4.5 mM MgClz, 0.1% gelatin, 20 mM EDTA) as a negative control well. 40-μL serum-EGTA reaction mixtures were prepared by mixing well 20 μL of 20% human serum, 4 μL of the above test compounds or DMSO diluted in 2× EGTA reaction buffer, and 16 μL of 2× EGTA reaction buffer. Serum-EGTA-DMSO was used as a blank control. The mixtures were incubated at room temperature for 30 min. In the negative control well, a 40-μL serum-EDTA reaction mixture was prepared by mixing well 20 μL of 20% human serum, 4 μL of the above DMSO diluted in 2× EDTA reaction buffer, and 16 μL of 2× EDTA reaction buffer. The mixture was incubated at room temperature for 30 min. The 96-well adsorption black plate coated with zymosan A one day ahead of time was washed 3 times with 300 μL/well TBST (0.05% tween 20) solution, and the serum-EGTA-test compound reaction mixtures, the blank control well, and the serum-EDTA-DMSO reaction mixture (negative control well) were added at 25 μL/well. The mixtures were reacted at 37 °C for 40 min. The plate was washed 3 times with 300 μL/well TBST solution, the StartingBlock™ T20 (TBS) blocking buffer was added at 300 μL/well, and the plate was incubated at 37 °C for 15 min. The plate was washed 3 times with 300 μL/well TBST solution, the anti-C5b-9 + C5b-8 antibody [aE11] was 1:5000 diluted in TBST solution and added at 100 μL/well, and the plate was incubated at 37 °C for 1 h. The plate was washed 3 times with 300 μL/well TBST solution, the goat anti-mouse IgG H&L (HRP) antibody was 1:5000 diluted in TBST solution and added at 100 μL/well, and the plate was incubated at 37 °C for 30 min. A QuantaBlu™ fluorogenic peroxidase substrate kit substrate was prepared by diluting 1 part of the QuantaBlu™ stable peroxide solution with 9 parts of the QuantaBlu™ substrate solution. The plate was washed 3 times with 300 μL/well TBST solution, and dried after the last wash. The substrate was added at 100 μL/well, and the plate was incubated at room temperature for 20 min. After the QuantaBlu™ stop solution was added at 100 μL/well, the fluorescence readings were taken on Flexstation 3, with the excitation light wavelength Ex set to 320 nM and the emission light wavelength Em set to 460 nM, cutoff 455.

The inhibition rate was calculated using the following formula:

$$\text{Inhibition rate} = \{1 - (RFU_{\text{test compound}} - RFU_{\text{negative control well}}) / (RFU_{\text{blank well}} - RFU_{\text{negative control well}})\} \times 100\%$$

Inhibition curves were plotted using Graphpad Prism software according to the concentrations of the compounds and the corresponding inhibition rates, and the concentrations of the compounds at which the inhibition rate reached 50%, i.e., $IC_{50}$ values, were calculated.

Table 2. The measured $IC_{50}$ values of the compounds of the present disclosure for the human complement alternative pathway

| Example No. | $IC_{50}$(nM) |
|---|---|
| **1** | 43.2 |
| **1-1** | 27.0 |
| **3** | 49.8 |
| **4** | 50.6 |
| **5** | 74.6 |
| Conclusion: the compounds of the present disclosure have good inhibitory effects on the human complement alternative pathway. | |

Test Example 3: Therapeutic Effects of Compounds Disclosed Herein on Heymann Nephritis in Rats

1. Abstract

In this experiment, male SD rats were selected to build Heymann nephritis models induced by sheep anti-rat FxIA serum, and the therapeutic effects of the positive drug LNP023 (synthesized by reference to Example 26 of WO2015009616A1) and the compound of Example 1-1 on nephritis in the male SD rats were evaluated.

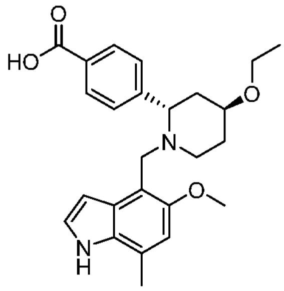

**LNP023**

2. Experimental method and materials

2.1. Experimental animals and housing conditions

Experimental animals: laboratory male SD rats, provided by Vital River Laboratory Animal Technology Co., Ltd. (production license number: SCXK (Zhejiang) 2019-0001; animal certificate number: 20210401Abzz0619000322; weighing 180-200 g at purchase).

Housing conditions: 5 rats/cage, 12/12-hour light/dark cycles, at a constant temperature of 23 ± 1 °C with humidity at 50-60%, given *ad libitum* access to food and water. After purchase, the animals were acclimatized for 1 week, and then the experiment began.

2.2. Experimental reagents and instruments

Positive drug LNP023: molecular weight 422.5, purity 99.9%.

Compound of Example 1-1: molecular weight 448.55, purity 99.9%.

Tween 80: Sinopharm Chemical Reagent Co., Ltd., catalog No. 30189828, lot No. 20180104.

Methylcellulose M450: Sinopharm Chemical Reagent Co., Ltd., catalog No. 69016460, lot No. 20170308.

Normal goat serum: Sangon biotech, lot No. GB02FC0260.

Sheep anti-rat Fx1A serum: PROBETEX, catalog No. PTX-002S, lot No. 376-4T.

Rat iC3b-ELISA kit: MYBioSource, catalog No. MBS 7255121, lot No. 20210301C.

Rat C3d-ELISAkit: MYBioSource, catalog No. MBS 7244846, lot No. 20210301C.

Urine/cerebrospinal fluid total protein (TPUC) kit: Shenzhen Mindray Bio-Medical, catalog No. 105-008805-00,

lot No. 147920004.

Creatinine kit: Shenzhen Mindray Bio-Medical, catalog No. 105-000457-00, lot No. 141120024.

Microalbuminuria (MALB) (with calibrator): Shenzhen Mindray Bio-Medical, catalog No. 105-009325-00, lot No. 046020011.

Microalbuminuria control material: Shenzhen Mindray Bio-Medical, catalog No. 105-002957-00, lot No. 053120008.

Urine/cerebrospinal fluid total protein (TPUC) control material: Shenzhen Mindray Bio-Medical, catalog No. 105-008841-00, lot No. 060520005.

Biochemical composite control material (control 1): Shenzhen Mindray Bio-Medical, catalog No. 105-007316-00, lot No. 059320002.

Biochemical composite control material (control 2): Shenzhen Mindray Bio-Medical, catalog No. 105-007317-00, lot No. 059419004.

Fully automatic blood biochemical analyzer: manufacturer Mindray Bio-Medical, model SB380.

Microplate reader: manufacturer BMGlabtech, model PHERAstar Fs.

Benchtop low-speed centrifuge: manufacturer Eppendorf, model 5417R.

Electronic balance: Mettler-Toledo Instruments Co., Ltd., model AL204.

2.3. Experimental design and method

2.3.1. Grouping of animals

After the acclimatization, the male SD rats were grouped as follows according to total protein in urine and body weight in the 2 days before the experiment:

| Group | Number of male SD rats | Route of administration for modeling | Drug and dose | Route of drug administration | Volume of drug administration |
|---|---|---|---|---|---|
| Normal group | 10 | iv normal goat serum | Solvent group | Orally administered twice daily | 10mL/kg |
| Model group | 10 | iv Fx1A serum | Solvent group | | |
| LNP023 group | 10 | iv FxlA serum | LNP023 20 mg/kg | | |
| LNP023 group | 10 | iv FxlA serum | LNP023 60 mg/kg | | |
| Example 1-1 group | 10 | iv Fx1A serum | Compound of Example 1-1 10 mg/kg | | |
| Example 1-1 group | 10 | iv Fx1A serum | Compound of Example 1-1 30 mg/kg | | |
| Example 1-1 group | 10 | iv Fx1A serum | Compound of Example 1-1 90 mg/kg | | |
| Note: solvent group: 0.5% methylcellulose M450 suspension (containing 0.5% tween 80); iv: intravenous. | | | | | |

2.3.2. Methods of drug preparation

Preparation of positive drug LNP023 (20 mg/kg): measuring out 600 mg + 1.5 mL of tween 80 + 298.5 mL of methylcellulose M450.

Preparation of positive drug LNP023 (60 mg/kg): measuring out 1800 mg + 1.5 mL of tween 80 + 298.5 mL of methylcellulose M450; prepared 3 times in total.

Preparation of the compound of Example 1-1 (10 mg/kg): measuring out 300 mg + 1.5 mL of tween 80 + 298.5 mL of methylcellulose M450.

Preparation of the compound of Example 1-1 (30 mg/kg): measuring out 900 mg + 1.5 mL of tween 80 + 298.5 mL of methylcellulose M450.
Preparation of the compound of Example 1-1 (90 mg/kg): measuring out 2700 mg + 1.5 mL of tween 80 + 298.5 mL of methylcellulose M450.
Preparation of 600-mL solvents: mixing well 3 mL of tween 80 + 597 mL of methylcellulose M450; prepared 3 times in total.

2.3.3. Experimental method

The male SD rats were randomly divided into 7 groups according to protein in urine and body weight: a normal group, a model group, LNP023 groups (20 mg/kg, 60 mg/kg, orally administered twice daily), and Example 1-1 groups (10 mg/kg, 30 mg/kg, 90 mg/kg, orally administered twice daily). On day 0 of the experiment, the model group and administration groups were given a single tail vein injection of FxIA serum (5 mL/kg), and the normal group was given a tail vein injection of normal goat serum. The administration groups were intragastrically administered positive drug LNP023 and the compound of Example 1-1 (10 mL/kg) from day 6 of the experiment, and the normal group and the model group were intragastrically administered the corresponding solvents. The experiment ended on day 15. Two-hour urine was collected on days 2, 4, 6, 8, 11, and 14 and assayed for creatinine and total protein. Serum was collected on day 15 for complement activation fragment (C3d+iC3b) analysis.

$$\text{Inhibition rate} = 100\% \times (\text{model group - administration group}) / (\text{model group - normal group})$$

2.4. Data expression and statistical processing

The experimental data are expressed as mean ± standard error (SEM). Statistical comparison was performed using Excel software t-test. The data of the model group and the data of the blank control group were analyzed and compared to judge whether a significant mathematical statistical significance exists. *P < 0.05 indicates a significant difference between the model group and the blank control group, **P < 0.01 indicates a highly significant difference between the model group and the blank control group, and ***P < 0.001 indicates a very highly significant difference between the model group and the blank control group. #P < 0.05 indicates a significant difference between an administration group and the model group, ##P < 0.01 indicates a highly significant difference between an administration group and the model group, and ###P < 0.001 indicates a very highly significant difference between an administration group and the model group.

3. Experimental results

The effects of the compound of Example 1-1 and the positive drug LNP023 on serum iC3b+C3d in passive heymann nephritis rats are shown in FIG. 1. The serum iC3b+C3d results in FIG. 1 show that: compared to the normal group, the model group showed a statistical increase (P < 0.001) in rat serum iC3b+C3d; compared to the model group, the 20 mg/kg and 60 mg/kg LNP023 groups showed significant decreases in rat serum iC3b+C3d, which are 62.2% (P < 0.01) and 68.8% (P < 0.001), respectively; compared to the model group, the 10 mg/kg, 30 mg/kg, and 90 mg/kg Example 1-1 groups showed significant decreases in rat serum iC3b+C3d, which are 76.2% (P < 0.001), 105.5% (P < 0.001), and 108.7% (P < 0.001), respectively.
The effects of the compound of Example 1-1 and the positive drug LNP023 on protein/creatinine in urine in passive heymann nephritis rats are shown in FIG. 2 and FIG. 3. FIG. 2 and FIG. 3 show that: the ratio of total protein/creatinine in rat urine of the model group constantly increased over time, and began to statistically increase from day 6 and constantly increased, compared to that of the normal group; compared to the model group, the 20 mg/kg and 60 mg/kg LNP023 groups began to show statistical decreases in the ratio of total protein/creatinine in rat urine from day 11, and showed a decrease of 56.4% (P < 0.01) and a decrease of 47.1% (P < 0.05), respectively on day 14, which are not significantly dose-dependent; the 10 mg/kg, 30 mg/kg, and 90 mg/kg Example 1-1 groups showed significant decreases in the ratio of total protein/creatinine in rat urine: the high-dose group began to show statistical decreases from day 8, the low- and medium-dose groups began to show statistical decreases from day 11, and the three dose groups showed a decrease of 48.1% (P < 0.05), a decrease of 70.9% (P < 0.001), and a decrease of 77.2% (P < 0.001), respectively on day 14, which are significantly dose-dependent.

## Claims

1. A compound of general formula (I) or a pharmaceutically acceptable salt thereof:

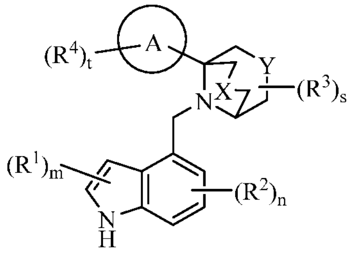

(I)

wherein:

X is selected from the group consisting of a chemical bond, $-CR^aR^b-$, -NR-, and -O-;
Y is selected from the group consisting of $-CR^{3a}R^{3b}-$, $-NR^{3c}-$, and -O-;
ring A is aryl or heteroaryl;
R, $R^a$, and $R^b$ are identical or different and are each independently selected from the group consisting of a hydrogen atom, alkyl, and haloalkyl;
each $R^1$ is identical or different and is independently selected from the group consisting of a hydrogen atom, alkyl, haloalkyl, hydroxyalkyl, alkenyl, alkynyl, alkoxy, haloalkoxy, halogen, cyano, $-NR^6R^7$, hydroxy, $-C(O)R^5$, $-CH_2C(O)R^5$, $-OCH_2C(O)R^5$, $-CH_2NHC(O)R^5$, $-C(O)NR^6R^7$, $-S(O)_pR^5$, $-S(O)_pNR^6R^7$, cycloalkyl, heterocyclyl, aryl, and heteroaryl, wherein the alkyl, alkoxy, cycloalkyl, heterocyclyl, aryl, and heteroaryl are each independently and optionally substituted with one or more substituents selected from the group consisting of halogen, alkyl, alkoxy, haloalkyl, haloalkoxy, cyano, amino, hydroxy, hydroxyalkyl, cycloalkyl, heterocyclyl, aryl, and heteroaryl;
each $R^2$ is identical or different and is independently selected from the group consisting of a hydrogen atom, alkyl, haloalkyl, hydroxyalkyl, alkenyl, alkynyl, alkoxy, haloalkoxy, halogen, cyano, $-NR^6R^7$, hydroxy, $-C(O)R^5$, $-CH_2C(O)R^5$, $-OCH_2C(O)R^5$, $-CH_2NHC(O)R^5$, $-C(O)NR^6R^7$, $-S(O)_pR^5$, $-S(O)_pNR^6R^7$, cycloalkyl, heterocyclyl, aryl, and heteroaryl, wherein the alkyl, alkoxy, cycloalkyl, heterocyclyl, aryl, and heteroaryl are each independently and optionally substituted with one or more substituents selected from the group consisting of halogen, alkyl, alkoxy, haloalkyl, haloalkoxy, cyano, amino, hydroxy, hydroxyalkyl, cycloalkyl, heterocyclyl, aryl, and heteroaryl;
each $R^3$, $R^{3a}$, $R^{3b}$, and $R^{3c}$ is identical or different and is independently selected from the group consisting of a hydrogen atom, alkyl, haloalkyl, hydroxyalkyl, alkoxy, haloalkoxy, oxo, halogen, cyano, $-NR^xR^y$, and hydroxy, wherein the alkyl is optionally substituted with one or more cyano or amino groups;
each $R^4$ is identical or different and is independently selected from the group consisting of a hydrogen atom, alkyl, haloalkyl, hydroxyalkyl, alkoxy, haloalkoxy, halogen, cyano, $-NR^8R^9$, hydroxy, $-C(O)R^{10}$, $-CH_2C(O)R^{10}$, $-C(O)NR^8R^9$, $-C(O)NHS(O)_pR^{10}$, $-S(O)_pNHC(O)R^{10}$, $-S(O)_pR^{10}$, $-S(O)_pNR^8R^9$, cycloalkyl, heterocyclyl, aryl, and heteroaryl, wherein the alkyl, cycloalkyl, heterocyclyl, aryl, and heteroaryl are each independently and optionally substituted with one or more substituents selected from the group consisting of halogen, alkyl, alkoxy, haloalkyl, haloalkoxy, cyano, amino, hydroxy, hydroxyalkyl, cycloalkyl, heterocyclyl, aryl, and heteroaryl;
$R^5$ are identical or different at each occurrence and are each independently selected from the group consisting of a hydrogen atom, hydroxy, alkyl, haloalkyl, alkoxy, cycloalkyl, heterocyclyl, aryl, and heteroaryl, wherein the alkyl, cycloalkyl, heterocyclyl, aryl, and heteroaryl are each independently and optionally substituted with one or more substituents selected from the group consisting of halogen, alkyl, alkoxy, haloalkyl, haloalkoxy, cyano, and hydroxy;
$R^{10}$ are identical or different at each occurrence and are each independently selected from the group consisting of a hydrogen atom, $-OR^{11}$, alkyl, haloalkyl, cycloalkyl, heterocyclyl, aryl, and heteroaryl, wherein the alkyl, cycloalkyl, heterocyclyl, aryl, and heteroaryl are each independently and optionally substituted with one or more substituents selected from the group consisting of halogen, alkyl, alkoxy, haloalkyl, haloalkoxy, cyano, and hydroxy;
$R^{11}$ is selected from the group consisting of a hydrogen atom, alkyl, haloalkyl, cycloalkyl, heterocyclyl, aryl, and heteroaryl, wherein the alkyl, cycloalkyl, heterocyclyl, aryl, and heteroaryl are each independently and optionally

substituted with one or more substituents selected from the group consisting of halogen, alkyl, haloalkyl, cyano, $-OR^{12}$, and $-C(O)OR^{12}$,

$R^{12}$ is selected from the group consisting of a hydrogen atom, alkyl, haloalkyl, cycloalkyl, heterocyclyl, aryl, and heteroaryl, wherein the alkyl, cycloalkyl, heterocyclyl, aryl, and heteroaryl are each independently and optionally substituted with one or more substituents selected from the group consisting of halogen, alkyl, alkoxy, haloalkyl, haloalkoxy, cyano, and hydroxy;

$R^6$, $R^7$, $R^x$, $R^y$, $R^8$, and $R^9$ are identical or different at each occurrence and are each independently selected from the group consisting of a hydrogen atom, alkyl, haloalkyl, hydroxyalkyl, cycloalkyl, heterocyclyl, aryl, and heteroaryl, wherein the alkyl, cycloalkyl, heterocyclyl, aryl, and heteroaryl are each independently and optionally substituted with one or more substituents selected from the group consisting of halogen, alkyl, alkoxy, haloalkyl, haloalkoxy, cyano, hydroxy, and hydroxyalkyl;

or $R^6$ and $R^7$, together with the nitrogen atom to which they are attached, form heterocyclyl, or $R^x$ and $R^y$, together with the nitrogen atom to which they are attached, form heterocyclyl, or $R^8$ and $R^9$, together with the nitrogen atom to which they are attached, form heterocyclyl, and the heterocyclyl is identical or different at each occurrence and is independently and optionally substituted with one or more substituents selected from the group consisting of halogen, alkyl, oxo, alkoxy, haloalkyl, haloalkoxy, cyano, amino, hydroxy, and hydroxyalkyl;

m is 0, 1, or 2;

n is 0, 1, 2, or 3;

s is 0, 1, 2, 3, or 4;

t is 0, 1, 2, 3, or 4; and

p is 0, 1, or 2.

2. The compound of general formula (I) or the pharmaceutically acceptable salt thereof according to claim 1, wherein $R^{10}$ is selected from the group consisting of a hydrogen atom, hydroxy, alkyl, haloalkyl, alkoxy, cycloalkyl, heterocyclyl, aryl, and heteroaryl, wherein the alkyl, cycloalkyl, heterocyclyl, aryl, and heteroaryl are each independently and optionally substituted with one or more substituents selected from the group consisting of halogen, alkyl, alkoxy, haloalkyl, haloalkoxy, cyano, and hydroxy.

3. The compound of general formula (I) or the pharmaceutically acceptable salt thereof according to claim 1 or 2, wherein Y is $-CR^{3a}R^{3b}-$; $R^{3a}$ and $R^{3b}$ are as defined in claim 1.

4. The compound of general formula (I) or the pharmaceutically acceptable salt thereof according to any one of claims 1 to 3, being a compound of general formula (II) or a pharmaceutically acceptable salt thereof:

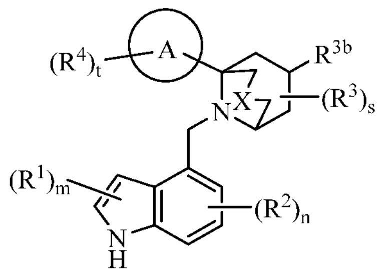

(II)

wherein:

ring A, X, $R^1$ to $R^3$, $R^{3b}$, $R^4$, m, n, s, and t are as defined in claim 1.

5. The compound of general formula (I) or the pharmaceutically acceptable salt thereof according to any one of claims 1 to 4, wherein $R^3$ is a hydrogen atom.

6. The compound of general formula (I) or the pharmaceutically acceptable salt thereof according to any one of claims 1 to 5, wherein m is 1, and n is 2.

7. The compound of general formula (I) or the pharmaceutically acceptable salt thereof according to any one of claims 1 to 6, being a compound of general formula (III) or a pharmaceutically acceptable salt thereof:

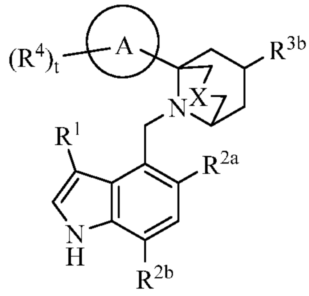

(III)

wherein:

$R^{2a}$ and $R^{2b}$ are identical or different and are each independently selected from the group consisting of a hydrogen atom, alkyl, haloalkyl, hydroxyalkyl, alkenyl, alkynyl, alkoxy, haloalkoxy, halogen, cyano, $-NR^6R^7$, hydroxy, $-C(O)R^5$, $-CH_2C(O)R^5$, $-OCH_2C(O)R^5$, $-CH_2NHC(O)R^5$, $-C(O)NR^6R^7$, $-S(O)_pR^5$, $-S(O)_pNR^6R^7$, cycloalkyl, heterocyclyl, aryl, and heteroaryl, wherein the alkyl, alkoxy, cycloalkyl, heterocyclyl, aryl, and heteroaryl are each independently and optionally substituted with one or more substituents selected from the group consisting of halogen, alkyl, alkoxy, haloalkyl, haloalkoxy, cyano, amino, hydroxy, hydroxyalkyl, cycloalkyl, heterocyclyl, aryl, and heteroaryl;
ring A, X, $R^1$, $R^{3b}$, $R^4$ to $R^7$, p, and t are as defined in claim 1.

**8.** The compound of general formula (I) or the pharmaceutically acceptable salt thereof according to any one of claims 1 to 7, wherein each $R^4$ is identical or different and is independently selected from the group consisting of a hydrogen atom, $C_{1-6}$ alkyl, $C_{1-6}$ haloalkyl, $C_{1-6}$ hydroxyalkyl, $C_{1-6}$ alkoxy, $C_{1-6}$ haloalkoxy, halogen, cyano, $-NR^8R^9$, hydroxy, and $-C(O)R^{10}$, and $R^8$, $R^9$, and $R^{10}$ are as defined in claim 1.

**9.** The compound of general formula (I) or the pharmaceutically acceptable salt thereof according to any one of claims 1 to 8, being a compound of general formula (IV) or a pharmaceutically acceptable salt thereof:

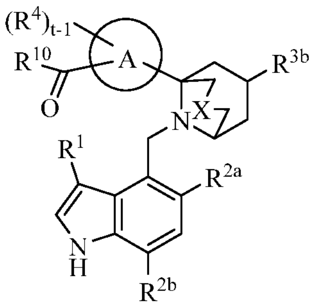

(IV)

wherein: $R^{10}$ is selected from the group consisting of hydroxy,

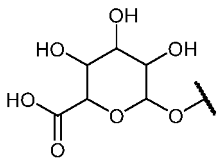

,

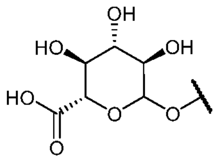, 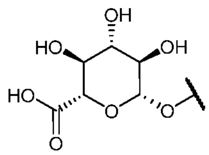, and 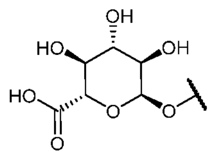;

preferably, $R^{10}$ is hydroxy;

t is 1 or 2;
$R^{2a}$ and $R^{2b}$ are identical or different and are each independently selected from the group consisting of a hydrogen atom, alkyl, haloalkyl, hydroxyalkyl, alkenyl, alkynyl, alkoxy, haloalkoxy, halogen, cyano, $-NR^6R^7$, hydroxy, $-C(O)R^5$, $-CH_2C(O)R^5$, $-OCH_2C(O)R^5$, $-CH_2NHC(O)R^5$, $-C(O)NR^6R^7$, $-S(O)_pR^5$, $-S(O)_pNR^6R^7$, cycloalkyl, heterocyclyl, aryl, and heteroaryl, wherein the alkyl, alkoxy, cycloalkyl, heterocyclyl, aryl, and heteroaryl are each independently and optionally substituted with one or more substituents selected from the group consisting of halogen, alkyl, alkoxy, haloalkyl, haloalkoxy, cyano, amino, hydroxy, hydroxyalkyl, cycloalkyl, heterocyclyl, aryl, and heteroaryl;
ring A, X, $R^1$, $R^{3b}$, $R^4$ to $R^7$, and p are as defined in claim 1.

**10.** The compound of general formula (I) or the pharmaceutically acceptable salt thereof according to any one of claims 1 to 9, wherein X is a chemical bond.

**11.** The compound of general formula (I) or the pharmaceutically acceptable salt thereof according to any one of claims 1 to 10, wherein ring A is 6- to 10-membered aryl or 5-to 10-membered heteroaryl.

**12.** The compound of general formula (I) or the pharmaceutically acceptable salt thereof according to any one of claims 1 to 11, wherein each $R^1$ is identical or different and is independently selected from the group consisting of a hydrogen atom, $C_{1-6}$ alkyl, $C_{1-6}$ haloalkyl, halogen, and cyano.

**13.** The compound of general formula (I) or the pharmaceutically acceptable salt thereof according to any one of claims 1 to 6, wherein each $R^2$ is identical or different and is independently selected from the group consisting of a hydrogen atom, $C_{1-6}$ alkyl, $C_{1-6}$ haloalkyl, $C_{1-6}$ hydroxyalkyl, $C_{1-6}$ alkoxy, $C_{1-6}$ haloalkoxy, halogen, cyano, amino, hydroxy, 3- to 8-membered cycloalkyl, and 3- to 8-membered heterocyclyl.

**14.** The compound of general formula (I) or the pharmaceutically acceptable salt thereof according to any one of claims 1 to 13, wherein $R^{3b}$ is selected from the group consisting of a hydrogen atom, $C_{1-6}$ alkyl, $C_{1-6}$ haloalkyl, $C_{1-6}$ hydroxyalkyl, $C_{1-6}$ alkoxy, and $C_{1-6}$ haloalkoxy.

**15.** The compound of general formula (I) or the pharmaceutically acceptable salt thereof according to any one of claims 1 to 14, being selected from the group consisting of the following compounds:

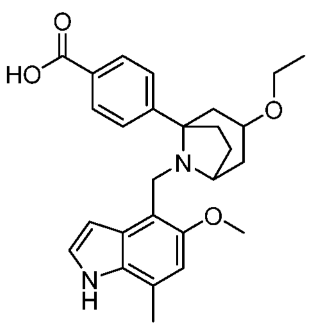,

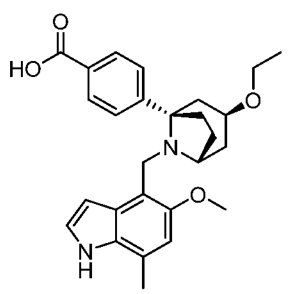
(±)-*rel*-(1*S*,3*S*,5*R*) **1**,

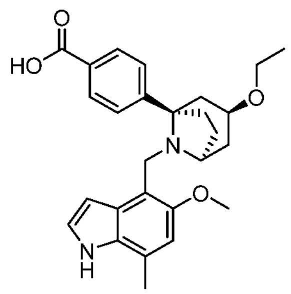
(±)-*rel*-(1*R*,3*S*,5*S*) **2**,

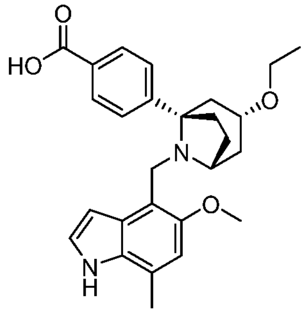

,

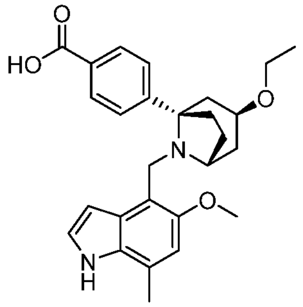

1-1

,

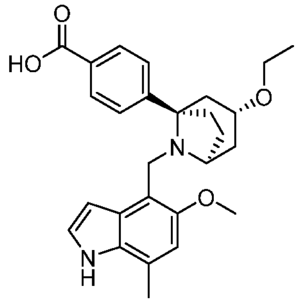

1-2

,

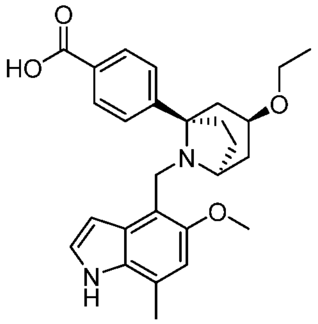

,

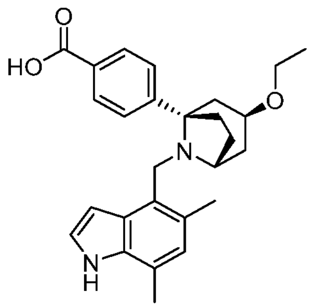

3

,

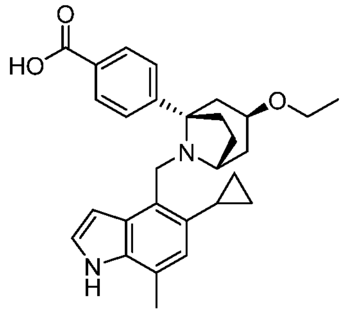

4

,

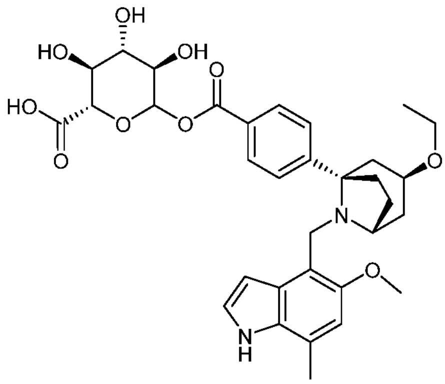

,

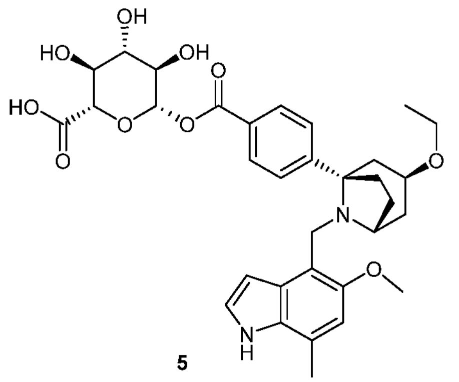

5

,

and

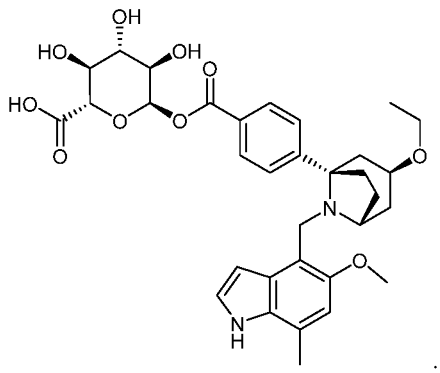

.

**16.** A compound of general formula (IVA) or a pharmaceutically acceptable salt thereof:

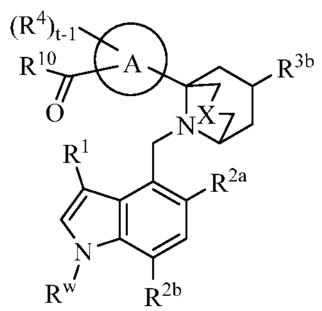

(IVA)

wherein:

$R^w$ is an amino protecting group; preferably, $R^w$ is tert-butoxycarbonyl or p-toluenesulfonyl; more preferably, $R^w$ is tert-butoxycarbonyl;
$R^{10}$ is alkoxy; preferably, $R^{10}$ is $C_{1-6}$ alkoxy; more preferably, $R^{10}$ is methoxy;
t is 1 or 2;
ring A, X, $R^1$, $R^{2a}$, $R^{2b}$, $R^{3b}$, and $R^4$ are as defined in claim 9.

**17.** A compound or a pharmaceutically acceptable salt thereof, being selected from the group consisting of the following compounds:

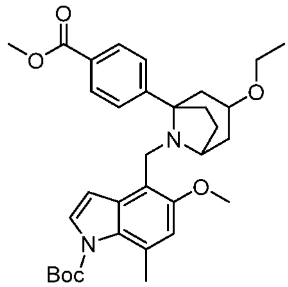

**1k**

,

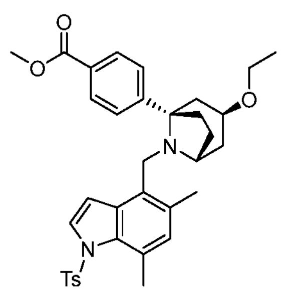

**3j**

,

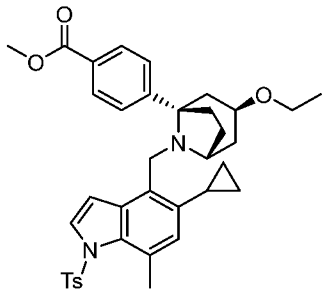

**4i**

,

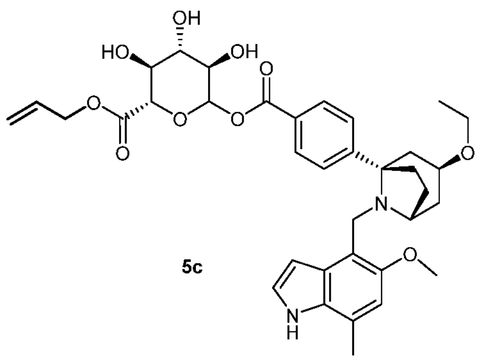

**5c**

,

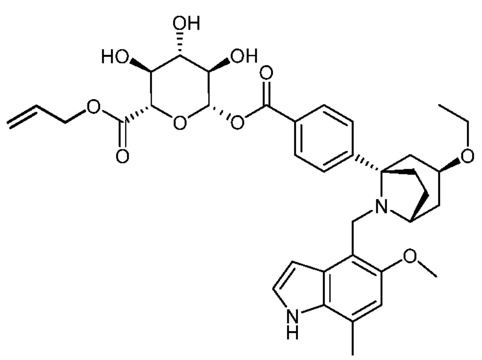

, and

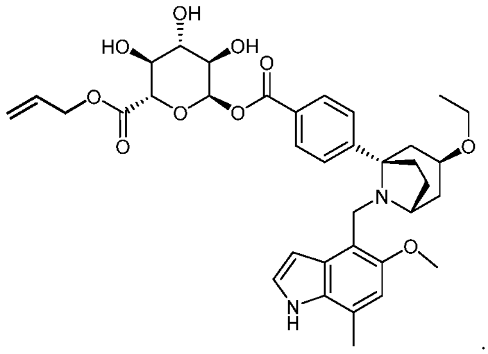

18. A method for preparing a compound of general formula (IV) or a pharmaceutically acceptable salt thereof, wherein the method comprises:

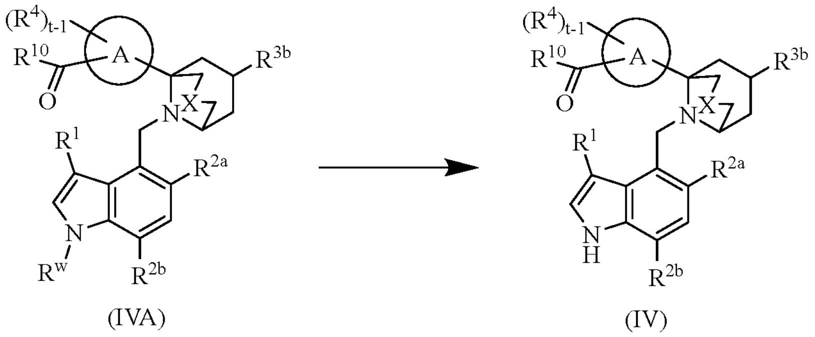

removing protecting group $R^w$ from a compound of general formula (IVA) in which $R^{10}$ is alkoxy (preferably $C_{1-6}$ alkoxy, and more preferably methoxy) or a pharmaceutically acceptable salt thereof and conducting a hydrolysis reaction to give a compound of general formula (IV) in which $R^{10}$ is hydroxy or a pharmaceutically acceptable salt thereof,
wherein:

$R^w$ is an amino protecting group; preferably, $R^w$ is tert-butoxycarbonyl or p-toluenesulfonyl; more preferably, $R^w$ is tert-butoxycarbonyl;
t is 1 or 2;
ring A, X, $R^1$, $R^{2a}$, $R^{2b}$, $R^{3b}$, and $R^4$ are as defined in claim 9.

19. A pharmaceutical composition, wherein the pharmaceutical composition comprises the compound of general formula (I) or the pharmaceutically acceptable salt thereof according to any one of claims 1 to 15, and one or more pharmaceutically acceptable carriers, diluents, or excipients.

20. Use of the compound of general formula (I) or the pharmaceutically acceptable salt thereof according to any one of claims 1 to 15 or the pharmaceutical composition according to claim 19 in the preparation of a medicament for inhibiting activation of the complement alternative pathway, preferably in the preparation of a medicament for inhibiting complement factor B.

21. Use of the compound of general formula (I) or the pharmaceutically acceptable salt thereof according to any one of claims 1 to 15 or the pharmaceutical composition according to claim 19 in the preparation of a medicament for treating and/or preventing a disease or disorder mediated by activation of the complement alternative pathway; preferably in the preparation of a medicament for treating and/or preventing a disease or disorder mediated by complement factor B, wherein the disease or disorder is selected from the group consisting of glomerulopathy, hemolytic uremic syndrome, atypical haemolytic uraemic syndrome, paroxysmal nocturnal hemoglobinuria, age-related macular degeneration, geographic atrophy, diabetic retinopathy, uveitis, retinitis pigmentosa, macular edema, Behcet's uveitis, multifocal choroiditis, Vogt-Koyanagi-Harada syndrome, birdshot retino-chorioditis, sympathetic ophthalmia, ocular dicatricial pemphigoid, ocular pemphigus, nonartertic ischemic optic neuropathy, post-operative inflammation, retinal vein occlusion, neurological disorders, multiple sclerosis, stroke, Guillain-Barré syndrome,

traumatic brain injury, Parkinson's disease, disorders of inappropriate or undesirable complement activation, hemodialysis complications, hyperacute allograft rejection, xenograft rejection, interleukin-2 induced toxicity during IL-2 therapy, Crohn's disease, adult respiratory distress syndrome, myocarditis, post-ischemic reperfusion conditions, myocardial infarction, balloon angioplasty, post-pump syndrome in cardiopulmonary bypass or renal bypass, atherosclerosis, hemodialysis, renal ischemia, mesenteric artery reperfusion after aortic reconstruction, infectious disease or sepsis, systemic lupus erythematosus, systemic lupus erythematosus nephritis, proliferative nephritis, liver fibrosis, hemolytic anemia, myasthenia gravis, tissue regeneration, neural regeneration, dyspnea, hemoptysis, acute respiratory distress syndrome, asthma, chronic obstructive pulmonary disease, emphysema, pulmonary embolisms and infarcts, pneumonia, fibrogenic dust diseases, pulmonary fibrosis, asthma, allergy, bronchoconstriction, parasitic diseases, Goodpasture's syndrome, pulmonary vasculitis, pauci-immune vasculitis, immune complex-associated inflammation, antiphospholipid syndrome, and obesity; and more preferably in the preparation of a medicament for treating and/or preventing C3 glomerulopathy, IgA nephropathy, membranous glomerulonephritis, atypical haemolytic uraemic syndrome, and paroxysmal nocturnal hemoglobinuria.

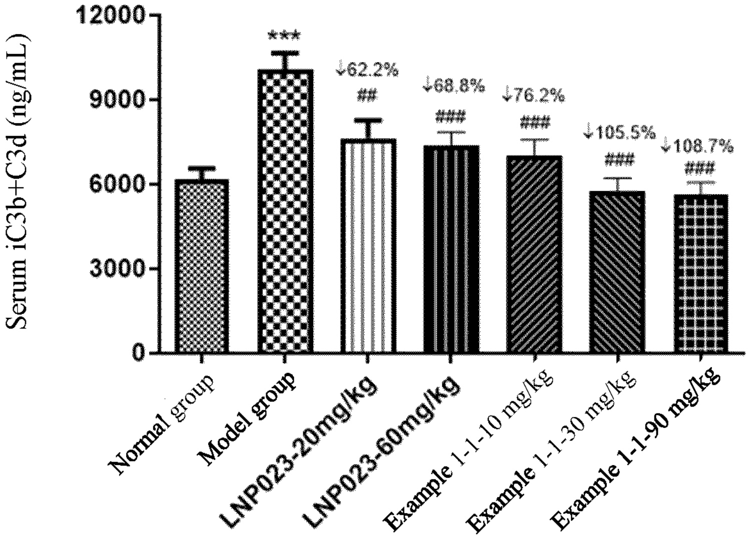
Serum iC3b+C3d (ng/mL)
12000
9000
6000
3000
0
***
↓62.2%
##
↓68.8%
###
↓76.2%
###
↓105.5%
###
↓108.7%
###
Normal group
Model group
LNP023-20mg/kg
LNP023-60mg/kg
Example 1-1-10 mg/kg
Example 1-1-30 mg/kg
Example 1-1-90 mg/kg

FIG. 1

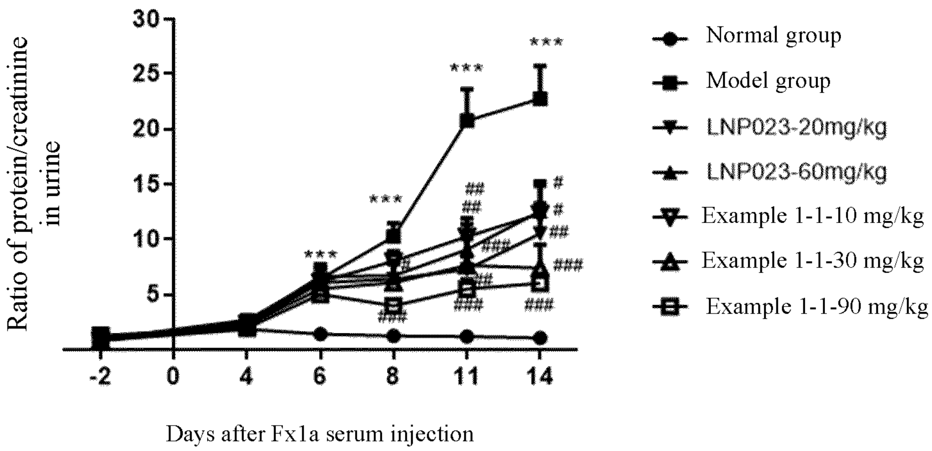
Ratio of protein/creatinine in urine
30
25
20
15
10
5
-2
0
4
6
8
11
14
Days after Fx1a serum injection
Normal group
Model group
LNP023-20mg/kg
LNP023-60mg/kg
Example 1-1-10 mg/kg
Example 1-1-30 mg/kg
Example 1-1-90 mg/kg

FIG. 2

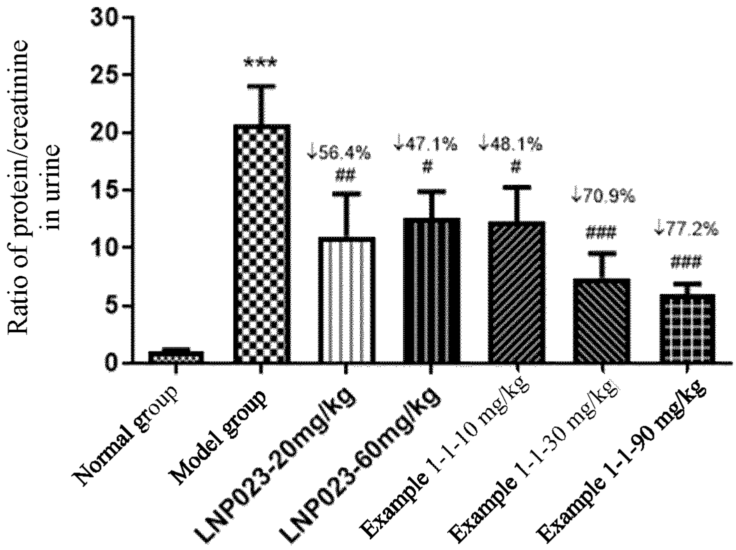
Ratio of protein/creatinine in urine
30
25
20
15
10
5
0
***
↓56.4%
##
↓47.1%
#
↓48.1%
#
↓70.9%
###
↓77.2%
###
Normal group
Model group
LNP023-20mg/kg
LNP023-60mg/kg
Example 1-1-10 mg/kg
Example 1-1-30 mg/kg
Example 1-1-90 mg/kg

FIG. 3

## INTERNATIONAL SEARCH REPORT

International application No.
**PCT/CN2021/142760**

**A. CLASSIFICATION OF SUBJECT MATTER**

C07D 401/04(2006.01)i; C07D 401/02(2006.01)i; A61K 31/454(2006.01)i; A61P 37/00(2006.01)i; A61P 13/12(2006.01)i

According to International Patent Classification (IPC) or to both national classification and IPC

**B. FIELDS SEARCHED**

Minimum documentation searched (classification system followed by classification symbols)

C07D; A61K; A61P

Documentation searched other than minimum documentation to the extent that such documents are included in the fields searched

Electronic data base consulted during the international search (name of data base and, where practicable, search terms used)

CNPAT, CNKI, EPODOC, WPI, REGISTRY（STN）, CAPLUS(STN), MARPAT(STN), structural formula search, 吲哚, 补体因子B, 肾病, indole, factor B, C3G, IgAN

**C. DOCUMENTS CONSIDERED TO BE RELEVANT**

| Category* | Citation of document, with indication, where appropriate, of the relevant passages | Relevant to claim No. |
|---|---|---|
| E | WO 2022028527 A1 (SHANGHAI MEIYUE BIOTECH DEV CO.LTD.) 10 February 2022 (2022-02-10)<br>claim 5 compound 17 | 1-4, 7-8, 10-15, 19-21 |
| A | CN 105579444 A (NOVARTIS AG) 11 May 2016 (2016-05-11)<br>claim 1, description, solution 14, embodiments 5-1, 11, 15 | 1-21 |
| A | CN 111032042 A (NOVARTIS AG) 17 April 2020 (2020-04-17)<br>claims 1 and 4 | 1-21 |
| A | WO 2020016749 A2 (NOVARTIS AG.) 23 January 2020 (2020-01-23)<br>claims 1-13 | 1-21 |

☐ Further documents are listed in the continuation of Box C.　　☑ See patent family annex.

* Special categories of cited documents:
- "A" document defining the general state of the art which is not considered to be of particular relevance
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **04 March 2022** | **07 April 2022** |

Name and mailing address of the ISA/CN

**China National Intellectual Property Administration (ISA/CN)**
**No. 6, Xitucheng Road, Jimenqiao, Haidian District, Beijing 100088, China**

Facsimile No. **(86-10)62019451**

Authorized officer

Telephone No.

Form PCT/ISA/210 (second sheet) (January 2015)

**INTERNATIONAL SEARCH REPORT**
**Information on patent family members**

International application No.
**PCT/CN2021/142760**

| Patent document cited in search report | Publication date (day/month/year) | Patent family member(s) | Publication date (day/month/year) |
|---|---|---|---|
| WO 2022028527 A1 | 10 February 2022 | None | |
| CN 105579444 A | 11 May 2016 | IL 243540 D0 | 29 February 2016 |
| | | IL 243540 A | 26 September 2019 |
| | | DK 3022192 T3 | 22 January 2018 |
| | | UA 117371 C2 | 25 July 2018 |
| | | PL 3022192 T3 | 30 March 2018 |
| | | JP 2016526576 A | 05 September 2016 |
| | | JP 6378761 B2 | 22 August 2018 |
| | | NZ 715780 A | 26 March 2021 |
| | | EA 201690223 A1 | 30 June 2016 |
| | | EA 031030 B1 | 30 November 2018 |
| | | AP 2016008992 A0 | 31 January 2016 |
| | | AP 201608992 A0 | 31 January 2016 |
| | | JO 3425 B1 | 20 October 2019 |
| | | PH 12016500072 A1 | 18 April 2016 |
| | | PH 12016500072 B1 | 18 April 2016 |
| | | CU 20160006 A7 | 31 August 2016 |
| | | CU 24397 B1 | 04 April 2019 |
| | | AU 2014290298 A1 | 11 February 2016 |
| | | AU 2014290298 B2 | 15 June 2017 |
| | | MX 351291 B | 09 October 2017 |
| | | US 2018009795 A1 | 11 January 2018 |
| | | US 10093663 B2 | 09 October 2018 |
| | | PE 20161066 A1 | 30 October 2016 |
| | | CA 2917839 A1 | 22 January 2015 |
| | | SG 11201600086 P A | 26 February 2016 |
| | | HR P20172000 T1 | 09 February 2018 |
| | | PT 3022192 T | 15 January 2018 |
| | | CY 1119767 T1 | 27 June 2018 |
| | | TW 201546058 A | 16 December 2015 |
| | | TW I641600 B | 21 November 2018 |
| | | LT 3022192 T | 27 December 2017 |
| | | HK 1221217 A1 | 26 May 2017 |
| | | SI 3022192 T1 | 31 January 2018 |
| | | IL 268623 D0 | 31 October 2019 |
| | | IL 268623 A | 30 January 2020 |
| | | UY 35663 A | 27 February 2015 |
| | | GT 201600007 A | 18 December 2018 |
| | | HU E037510 T2 | 28 September 2018 |
| | | EP 3299365 A1 | 28 March 2018 |
| | | CL 2016000060 A1 | 10 June 2016 |
| | | WO 2015009616 A1 | 22 January 2015 |
| | | TN 2016000017 A1 | 05 July 2017 |
| | | SV 2016005137 A | 12 June 2018 |
| | | BR 112016000909 A2 | 25 July 2017 |
| | | BR 112016000909 A8 | 07 January 2020 |
| | | US 2016152605 A1 | 02 June 2016 |
| | | US 9682968 B2 | 20 June 2017 |
| | | ES 2655855 T3 | 21 February 2018 |
| | | EP 3022192 A1 | 25 May 2016 |

Form PCT/ISA/210 (patent family annex) (January 2015)

**INTERNATIONAL SEARCH REPORT**
**Information on patent family members**

International application No.
**PCT/CN2021/142760**

| Patent document cited in search report | Publication date (day/month/year) | Patent family member(s) | Publication date (day/month/year) |
|---|---|---|---|
| CN 111032042 A | 17 April 2020 | RU 2020111788 A | 30 September 2021 |
| | | RU 2020111788 A3 | 28 December 2021 |
| | | WO 2019043609 A1 | 07 March 2019 |
| | | JP 2021181471 A | 25 November 2021 |
| | | JP 6754919 B1 | 16 September 2020 |
| | | JP 2020529466 A | 08 October 2020 |
| | | US 2020338059 A1 | 29 October 2020 |
| | | EP 3675854 A1 | 08 July 2020 |
| | | CA 3073346 A1 | 07 March 2019 |
| | | CL 2020000483 A1 | 04 September 2020 |
| | | JP 2020193222 A | 03 December 2020 |
| | | JP 6929425 B2 | 01 September 2021 |
| | | AU 2018326768 A1 | 06 February 2020 |
| | | AU 2018326768 B2 | 29 July 2021 |
| | | IL 272888 D0 | 30 April 2020 |
| | | KR 20200047540 A | 07 May 2020 |
| | | BR 112020003737 A2 | 01 September 2020 |
| WO 2020016749 A2 | 23 January 2020 | IL 279992 D0 | 01 March 2021 |
| | | CN 112513025 A | 16 March 2021 |
| | | JP 2021530505 A | 11 November 2021 |
| | | AU 2019304216 A1 | 21 January 2021 |
| | | KR 20210032950 A | 25 March 2021 |
| | | US 2021269416 A1 | 02 September 2021 |
| | | US 11208398 B2 | 28 December 2021 |
| | | EP 3823963 A2 | 26 May 2021 |
| | | WO 2020016749 A3 | 05 March 2020 |
| | | CA 3106124 A1 | 23 January 2020 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2015009616 A1
- WO 2019043609 A1
- WO 2020016749 A2